# EUROPEAN PATENT APPLICATION

(11) **EP 4 467 141 A1**
(43) Date of publication of application: **27.11.2024**
(21) Application number: 23175081.1
(22) Date of filing: 24.05.2023
(51) Int. Cl.: A61K 31/519, A61P 35/00

(54) **PYRAZOLO-QUINOLINES AS SELECTIVE INHIBITORS OF GENOTOXIC STRESS-INDUCED IKK/NF- KB PATHWAYS FOR CANCER THERAPY**

(71) Applicant: Max-Delbrück-Centrum für Molekulare Medizin in der Helmholtz-Gemeinschaft, 13125 Berlin (DE); Forschungsverbund Berlin E.V., 12489 Berlin (DE)
(72) Inventor: Scheidereit, Claus, 10629 Berlin (DE); Mucka, Patrick, 13187 Berlin (DE); Willenbrock, Michael, 22305 Hamburg (DE); Bosco, Bartolomeo, 10405 Berlin (DE); Lindemann, Peter, 13125 Berlin (DE); Radetzki, Silke, 12683 Berlin (DE); von Kries, Jens Peter, 16341 Panketal (DE); Nazaré, Marc, 12209 Berlin (DE)
(74) Representative: Hertin und Partner Rechts- und Patentanwälte PartG mbB

(57) **Abstract**

The invention relates to chemical compounds and their use in the treatment of a cancer exhibiting genotoxic stress-induced IKK/NF-κB activation. The invention relates to chemical compounds useful for inhibiting genotoxic stress-induced IKK/NF-κB activation. The invention further relates to a pharmaceutical composition comprising a compound of the invention for the treatment of a subject afflicted by a cancer exhibiting genotoxic stress-induced IKK/NF-κB activation. The invention further relates to an *in vitro* method for the inhibition of genotoxic stress-induced NF-kB signaling or inhibition of DNA repair mechanisms.

## Description

The invention is in the field of biochemistry and medicine and relates to chemical compounds and their use in the treatment of a cancer exhibiting genotoxic stress-induced IKK/NF-κB activation.

The invention relates to chemical compounds and their use in the treatment of a cancer exhibiting genotoxic stress-induced IKK/NF-κB activation. The invention relates to chemical compounds useful for inhibiting genotoxic stress-induced IKK/NF-κB activation. The invention further relates to a pharmaceutical composition comprising a compound of the invention for the treatment of a subject suffering from a cancer exhibiting genotoxic stress-induced IKK/NF-κB activation. The invention further relates to an in vitro method for the inhibition of genotoxic stress-induced NF-kB signaling or inhibition of DNA repair mechanisms.

### BACKGROUND OF THE INVENTION

Therapeutic resistance is a major hurdle to the successful clinical treatment of cancer and remains the main cause of treatment failure in cancer patients. However, drugs intervening in the molecular pathways regulating this resistance remain elusive (Ellis and Hicklin, 2009; Labrie et al., 2022).

Traditional cancer treatments, such as chemo- and radiotherapies, induce genotoxic stress such as DNA damage leading to the activation of anti-apoptotic and pro-survival genetic programs, a major regulator of which is the inducible transcription factor NF-κB (nuclear factor kappa-light-chain-enhancer of activated B-cells) (Baud and Karin, 2009).

NF-κB is a widespread and rapidly inducible transcription factor (TF). Several hundred target genes regulated by NF-κB have been identified. Most of the target genes are involved in the regulation of the immune system and inflammation, cell cycle, proliferation and cell death. Activated NF-κB pathways regulate different cellular outcomes by transcriptional regulation of target genes that encode for non-coding RNA (ribonucleic acid) or proteins controlling cell survival and proliferation, adhesion and matrix remodeling, lymphocyte activation, host defense or immunity and inflammation. Besides its prominent functions in development and response to stress, dysregulated NF-κB contributes to a multitude of diseases including, most importantly, chronic inflammation, autoimmune diseases and cancer.

Genotoxic stress, e.g., DNA double-strand breaks (DSBs) caused by chemo- and radiotherapies, induces a complex cellular process called DNA damage response (DDR). The DDR regulates cell fate decisions like cell cycle arrest and DNA repair, senescence, quiescence apoptosis or other kinds of cell death, depending on the extent of genotoxic stress. DNA double strand breaks induce a nuclear-to-cytoplasmic signaling cascade, which finally cause IKK activation analogously to cytokine-induced NF-κB activation (Stilmann et al.; 2009).

DSBs are sensed by two sensors, ATM and PARP1, whose activation is required for the downstream triggering of the IκB kinase (IKK)/NF-κB pathway. PARP1 is recruited to DNA lesions within seconds and synthesizes poly(ADP)-ribose (PAR), attached to itself and other proteins, dissociates, and assembles a nucleoplasmic complex with !KKγ, ATM and PIASy, in which !KKγ is SUMOylated and phosphorylated. Subsequently, p-ATM and modified !KKγ are exported to the cytoplasm (Huang et al., 2003; Mabb et al., 2006; Stilmann et al., 2009). Within the cytoplasm, an ATM-TRAF6-clAP1 axis and PTM-modified !KKγ, lead to activation of the IKK kinase complex and to activation and nuclear translocation of NF-κB (Hinz et al., 2010). Once in the nucleus, NF-κB potently upregulates the expression of numerous anti-apoptotic and pro-survival genes, leading to tumor survival and thus therapeutic resistance to standard cancer therapies (Baud and Karin, 2009; Nakanishi and Toi, 2005; Tergaonkar et al., 2002).

IKK and NF-κB are deregulated in numerous pathological conditions including cancer and chronic inflammation (Karin and Greten, 2005; Rayet and Gélinas, 1999; Taniguchi and Karin, 2018). Aberrant NF-κB activation is associated with tumor-promoting inflammation, which is a driving force in tumorigenesis by sustaining a proliferative environment as a consequence of inflammatory cytokine secretion (Hanahan and Weinberg; 2011). Moreover, NF-κB can affect cellular proliferation, angiogenesis, and metastasis through transcriptional regulation of target genes (Baud and Karin; 2009). Constitutively activated NF-κB was found in several human cancers and tumor cell lines derived from hematopoietic and lymphoid malignancies, such as multiple myeloma, acute myeloid leukemia, T cell lymphoma and Hodgkin lymphoma. Similarly, elevated NF-κB activation was found in melanoma cells, in lung carcinoma cells, in bladder cancer cells, in breast cancer cells, and in pancreatic adenocarcinoma cells.

The wide-reaching physiological roles and their deregulation in numerous disease states suggest the IκB kinase (IKK)/NF-κB pathways as attractive therapeutic targets, a view discussed repeatedly in the literature (Baud and Karin, 2009; Gilmore and Herscovitch, 2006). However, relatively few therapeutics targeting components of IKK/NF-κB signalling cascades have reached the clinic.

Proteasome inhibitors were the first used NF-κB pathway inhibitors. However, proteasome inhibitors have undefined molecular specificity and target the canonical and the non-canonical NF-κB pathway, because both signaling cascades rely on degradation or processing functions. The dose-limiting toxic effects for patient treatment with proteasome inhibitors include peripheral neuropathy, thrombocytopenia, neutropenia, anaemia, fatigue, and diarrhea. General IKK/NF-κB pathway inhibition causes systemic toxicity and severe adverse effects due to its pleiotropic functions and therefore is not applicable as a therapeutic strategy in patients (Baud and Karin; 2009).

Considering IKK's critical role as the master regulator of NF-κB activity, it would seem an attractive therapeutic target (Scheidereit, 2006). However, outcomes of previous attempts to develop clinically applicable IKK inhibitors were unsuccessful. IKK has multiple physiological functions and pharmacological targeting is highly problematic due to widespread toxicity (Baud and Karin, 2009; Gilmore and Herscovitch, 2006). This is explained by the critical physiological role of IKK-controlled NF-κB in integrating numerous endogenous and exogenous stress stimuli, in addition to NF-κB-independent functions of IKK, including the regulation of mRNA stability (Antonia et al., 2021; Hinz and Scheidereit, 2014; Mikuda et al., 2018).

Targeting the ligands, cell surface receptors, or receptor adaptor proteins for various other NF-κB activators, where the signaling cascade is initiated, is an effective therapeutic strategy in numerous contexts, including cancer (Wang et al., 2013). However, targeting NF-κB activity from the cell surface receptor-mediated origin is complicated by factors such as the diverse downstream effectors and pathway crosstalk of the various IKK pathway/NF-κB stimuli (Oeckinghaus et al., 2011).

Considering the nuclear origin of genotoxic stress-induced NF-κB activity, a deeper understanding of the DNA damage-induced NF-κB pathway is required to identify pathway-specific druggable regulators of this pathway. It has been established that PARP1 and poly(ADP-ribose) are critical components of the DNA damage-induced IKK/NF-κB pathway (Stilmann et al., 2009; Tufan et al., 2022). Considering the essential role of PARP1, PARP inhibition would appear a promising avenue to achieve DNA damage-specific NF-κB inhibition. Despite this, PARP inhibitors do not reliably inhibit NF-κB following DNA damage. Olaparib, the first clinically approved PARP inhibitor, does not inhibit IKK-dependent p65 phosphorylation following DNA damage while it has been proposed that another clinically approved compound, Niraparib, radiosensitizes tumors by inhibiting NF-κB (Hunter et al., 2012). The causes of this discrepancy may be due to the unclear interregulation of the larger group of PARP isoforms and the diverse isoform specificities of investigational and clinical PARP inhibitors, and off-targets including several kinases (Antolin et al., 2020). Thus, despite their noted clinical efficacy, PARP inhibitors cannot be used to inhibit NF-κB following standard chemo- and radiotherapies and therefore may lack the tumor-killing effect resulting from abrogated NF-κB-mediated anti-apoptotic and pro-survival target gene expression.

Due to its crucial role in in numerous pathological conditions including cancer, NF-κB inhibition remains conceptually attractive, but inhibitors have to be developed that block IKK and NF-κB in a pathway selective manner upstream of IKK. This has been achieved with Bruton's Tyrosine Kinase (BTK) inhibitor Ibrutinib, which specifically interferes with the BTK-dependent IKK and NF-κB activation downstream of the B cell receptor (BCR), thus blocking BCR-induced NF-κB activation, while not affecting BCR-independent NF-κB pathways. Ibrutinib is used to treat Mantle Cell Lymphoma (MCL) and Chronic Lymphocytic Leukemia (CLL) (De Rooij et al., 2012; Wang et al., 2013). Further, WO 2018/087389 discloses chemical compounds (MW01 and derivatives thereof) that specifically inhibit IKK/NF-κB activation induced by genotoxic stress, and can thus be used to inhibit NF-κB following standard chemo- and radiotherapies.

In light of the prior art there remains a significant need in the art to provide additional means for the treatment of diseases associated with genotoxic stress-induced IKK/NF-κB activation, e.g., as observed for chemo- and radiotherapies. In light of the prior art and considering the relevance of IKK/NF-κB inhibition in multiple cancers and tumorous diseases, there remains a significant need to provide compounds for the inhibition of IKK/NF-κB. Although the compounds disclosed in the prior art selectively interfere with IKK/NF-kB activation, there remains a need to provide further classes of selective pathway-specific inhibitors that exhibit higher selectivity and efficacy, while avoiding serious side effects.

### SUMMARY OF THE INVENTION

In light of the prior art the technical problem underlying the present invention is to provide improved or alternative means for the treatment of a cancer associated with genotoxic stress-induced IKK/NF-κB activation. Another problem underlying the present invention is to provide chemical compounds for selective inhibition of IKK/NF-κB activation induced by genotoxic stress, such as caused by cancer therapy, which show high selectivity, efficacy and/or exhibit low side effects.

This problem is solved by the features of the independent claims. Preferred embodiments of the present invention are provided by the dependent claims.

The invention relates to a compound according to **Formula I** for use in the treatment of a cancer exhibiting genotoxic stress-induced IKK/NF-κB activation, 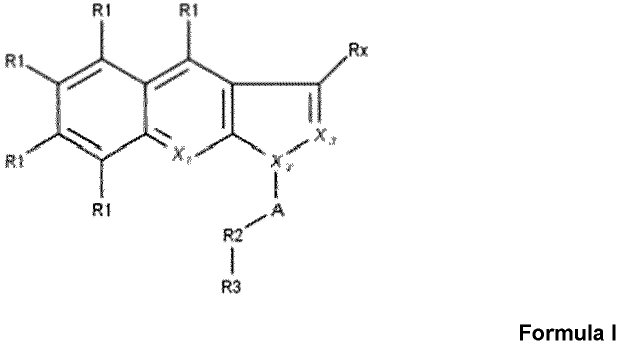 wherein
**R1** = can be the same or different, H, OH, halogen (preferably Br, Cl or F), alkyl (preferably C1 to C6 alkyl), alkenyl, alkynyl, alkoxy (preferably OCH₃, OCH₂CH₃), alkyl carbonyl, carboxyl, alkoxy carbonyl, amine (preferably primary, secondary or tertiary amine),
**Rx** = amine (preferably primary, secondary or tertiary amine), OH, H, alkyl (preferably C1 to C6 alkyl),
**X1**, **X2**, **X3** = can be the same or different, N or C,
**A=** alkyl (preferably C1 to C6 alkyl), alkenyl, alkynyl, alkoxy, carbonyl, carboxyl, cycloalkyl (preferably C3-C6 cycloalkyl), aryl (preferably phenyl), sulfonyl
**R2** = alkyl (preferably C1 to C6-alkyl), alkenyl, alkynyl, alkoxy, carbonyl, carboxyl, alkyl carbonyl, alkoxy carbonyl, amine (preferably primary, secondary or tertiary amine), aryl (preferably phenyl or naphtyl), C3 to C8 cycloalkyl, a 5 or 6-membered heterocyclyl or heteroaryl comprising one or more of N, O and/or S,
**R3** = is H, halogen (preferably Cl, Br, F), alkyl (preferably C1 to C6 alkyl), haloalkyl, OH, alkoxy (preferably OCH₃, OCH₂CH₃), alkoxy carbonyl (preferably - COOCH₃, -CH₂COOCH₃, - CH₂CH₂COOCH₃), cycloalkyl (preferably C3 to C6 cycloalkyl), aryl (preferably phenyl), alkyl-cycloalkyl, C1 to C6 alkyl-aryl, heterocyclyl, heteroaryl, carboxamide, CO-Cx alkyl carboxamide (preferably CON(CH₂CH₂)₂O); amine (preferably primary, secondary or tertiary amine), carbamate, nitro, -CN.

In one embodiment the compound according to **Formula I** is characterized in that **R1** can be the same or different and is selected from the group of H, OH, halogen (preferably Br, Cl or F), alkoxy (preferably OCH₃, OCH₂CH₃) and amine (preferably primary, secondary or tertiary amine).

In one embodiment the compound according to **Formula I** is characterized in that **R2** is alkyl (preferably C1 to C6-alkyl), carbonyl, amine (preferably primary, secondary or tertiary amine), aryl (preferably phenyl or naphtyl) or a 5 or 6-membered heterocyclyl or heteroaryl comprising one or more of N, O and/or S.

In one embodiment the compound according to **Formula I** is characterized in that R3 is H, alkoxy (preferably OCH₃, OCH₂CH₃), heterocyclyl or heteroaryl comprising one or more of N, O and/or S, amine (preferably primary, secondary or tertiary amine).

In one embodiment the compound according to **Formula I** is characterized in that at least one of **X1**, **X2** and **X3** is N. In a preferred embodiment the compound according to Formula I is characterized in that **X1**, **X2** and **X3** are N.

Because of the wide-reaching physiological roles of the NF-κB pathways, all of which depend on IKK, direct inhibition of the IKK complex or other downstream molecules leads to intolerable side effects including broad immunosuppression, which harbors high risks of infections and might lead to escape of cancer cells from immune surveillance. These disadvantages of known NF-κB pathway inhibitors are overcome by the means of the present invention, in particular those compounds described herein under **Formulae I, II, III, IV, V, VI, VII, VII** and **IX.**

In the prior art, general IKK inhibitors have been described. In contrast, the compounds of the present invention are pathway-selective inhibitors of IKK-NF-κB, which do not directly act on IKK. Previously described IKK inhibitors are direct IKK inhibitors and do not discriminate between gentotoxic stress-induced IKK-NF-κB signaling and the many other pathways that activate NF-κB through IKK such as activation by cytokines such as TNF-α.

The compounds according to **Formula I** predominantly inhibit activation of the genotoxic-stress-induced IKK/NF-kB pathway, but not any other pathways of NF-kB activation (or to some negligible or minor extent, and not to a large extent or as large an extent as the genotoxic-stress-induced IKK/NF-kB pathway). The inhibition shown by these compounds is therefore "genotoxic-stress-induced IKK/NF-kB pathway-specific", such that this pathway is inhibited more than other IKK/NF-kB pathways. This selective inhibition has the advantage that side-effects resulting from inhibition of other NF-kB activation pathways can be excluded or reduced. This makes it possible to tolerate treatment with a compound according to **Formula I** over prolonged periods of multiple days, weeks or even years without suffering from (or thus reducing) disadvantageous side effects.

In WO 2018/087389, compounds that selectively inhibiting genotoxic-stress-induced IKK/NF-kB activation have been disclosed (MW01 and derivatives thereof). However, it was entirely surprising that the structurally distinct compounds of the present invention selectively inhibit genotoxic-stress-induced IKK/NF-kB activation.

Surprisingly, the compounds of the present invention do not directly inhibit previously identified regulators of the IKK/NF-κB pathway, most critically including IκB kinase (IKK). The compounds according to the present invention inhibit signal transmission between ATM, PARP1 and IKK by inhibiting cdc-like kinase 2 (CLK2) and/or cdc-like kinase 4 (CLK4), as essential regulators of DNA damage- induced IKK and NF-κB activity.

Thereby, the compounds advantageously sensitize tumor cells to DNA damaging agents by increasing p53-induced apoptosis, thereby reducing cancer cell viability. It is shown that combinatorial treatment between the compounds according to the present invention and commercial chemotherapeutic drugs, such as etoposide and cisplatin, and irradiation therapy, unbalances the molecular axis of NF-κB-p53, down-regulating NF-κB activation and activating a p53 signature. These effects make the compounds according to the present invention valuable drugs in the treatment, or co-treatment, of many aggressive tumors, sensitizing them to chemotherapeutic treatments that are otherwise ineffective. Thus, the compounds according to the present invention can advantageously be used in context of genotoxic therapy-induced, or ongoing, DNA damage in order to increase tumor cell apoptosis, which is beneficial in cancer treatment. The compounds can therefore reduce chemotherapeutic burden while treating cancer in the clinic.

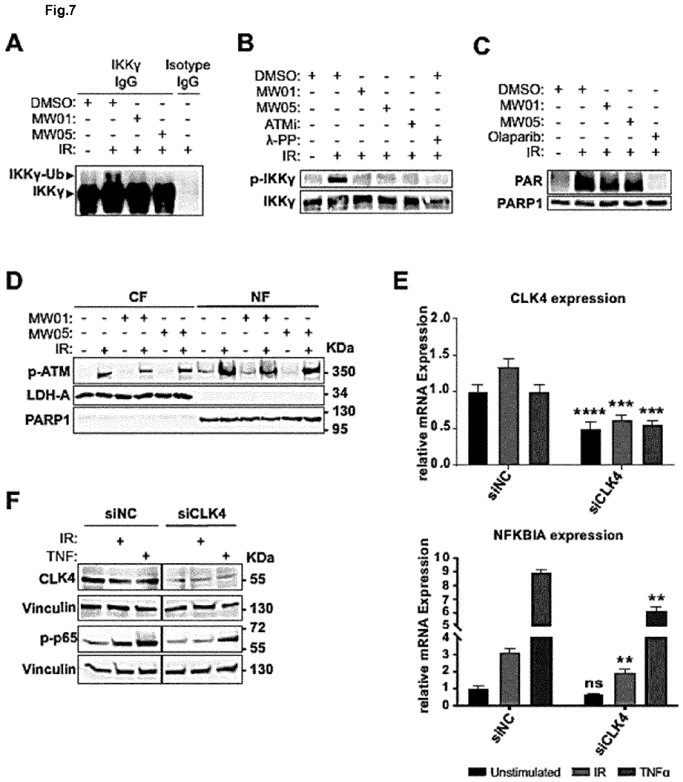

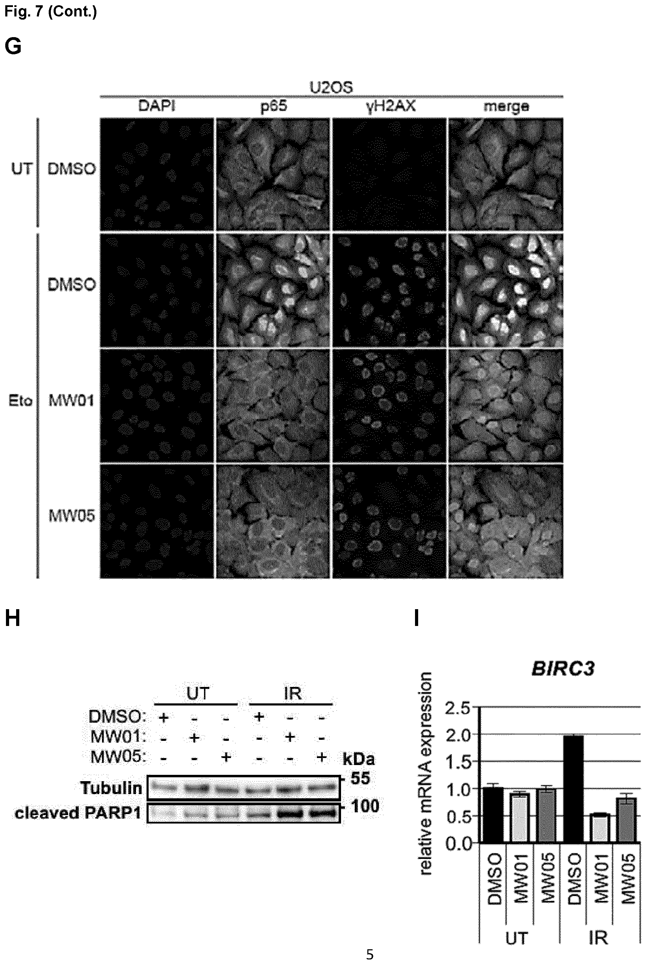

The identification of CLK2 and CLK4 as the shared target of the compounds according to the present invention was entirely surprising, since this kinase has no previously reported role in genotoxic stress signaling or NF-κB pathways. It has been reported that CLK2 phosphorylates SR proteins that modulate RNA splicing (Iwai et al., 2018; Salvador and Gomis, 2018; Yoshida et al., 2015). A splicing-related function of CLK2 and CLK4 in the regulation of IKK signaling can however be excluded, because already short-term pre-incubation with the inhibitors blocks DNA damage-induced NF-κB activation. Re-inspection of the global data set of a genome-wide siRNA screen for regulators of etoposide-induced NF-κB (Tufan et al., 2022), revealed that CLK2 and CLK4 scored similar to IKK or ATM, additionally confirming a function for CLK2/CLK4 in NF-κB signalling. It was shown by the inventors that CLK2 and 4 act downstream of ATM and PARP1, but not directly at IKKα/CHUK or IKKβ, as indicated by the examples below (Fig. 7A-C, 7G, Table 6) and the compounds abrogate the signature Ser-85 phosphorylation of IKKγ. Silencing of the CLKs revealed that they are essential to promote the phosphorylation at Ser-85 by ATM, perhaps through a priming phosphorylation for ATM. The compounds according to the present invention inhibiting CLK2 and/or CLK4 thus represent a new therapeutic modality to achieve DNA damage-specific NF-κB inhibition. A skilled person would have had no reasonable expectation of success that the compounds of the present invention exhibit this action, nor that CLK2 or CLK4 is the target of these compounds. The present invention thus represents a beneficial and unexpected finding of chemical compounds and structures that exhibit this unique function.

Advantageously, the compounds of the present invention show a kinase-specific profile of selective inhibition of CLK2 and/or CLK4. In contrast, the compounds disclosed in WO 2018/087389 (MWO1 and derivatives) show some PI3K isoform off-target and kinase inhibitor promiscuity, which is a potential problem, which is however common to kinase inhibitors as a drug class (Cohen et al., 2021).

In one embodiment the invention relates to a compound according to **Formula II** for use in the treatment of a cancer exhibiting genotoxic stress-induced IKK/NF-κB activation 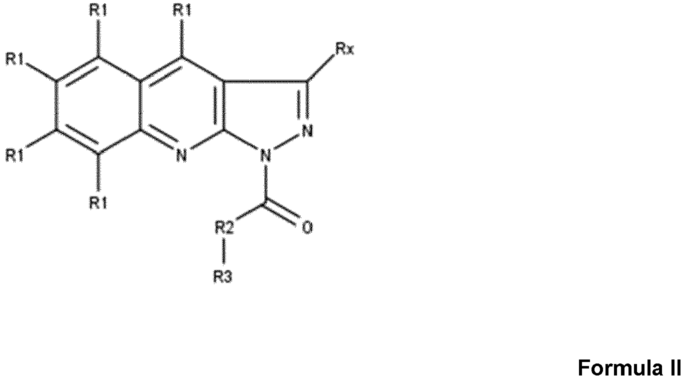 wherein
**R1** = can be the same or different, H, OH, halogen (preferably Br, Cl or F), alkyl (preferably C1 to C3 alkyl), alkenyl, alkynyl, alkoxy (preferably OCH₃, OCH₂CH₃), alkyl carbonyl, carboxyl, alkoxy carbonyl, amine (preferably primary, secondary or tertiary amine),
**Rx** = amine (preferably primary, secondary or tertiary amine), OH, H, alkyl (preferably C1 to C3 alkyl),
**R2** = alkyl (preferably C1 to C3-alkyl), alkenyl, alkynyl, alkoxy, carbonyl, carboxyl, alkyl carbonyl alkoxy carbonyl, amine (preferably primary, secondary or tertiary amine), aryl (preferably phenyl or naphtyl), C3 to C8-cycloalkyl, a 5- or 6-membered heterocyclyl or heteroaryl comprising one or more of N, O and/or S, and
**R3** = H, halogen (preferably Cl, Br, F), alkyl (preferably C1 to C3 alkyl), haloalkyl, OH, alkoxy (preferably OCH₃, OCH₂CH₃), alkoxy carbonyl (preferably - COOCH₃, -CH₂COOCH₃, - CH₂CH₂COOCH₃), cycloalkyl (preferably C3 to C6 cycloalkyl), aryl (preferably phenyl), alkyl-cycloalkyl, C1 to C6 alkyl-aryl, heterocyclyl, heteroaryl, carboxamide, CO-Cx alkyl carboxamide (preferably CON(CH₂CH₂)₂); amine (preferably primary, secondary or tertiary amine), carbamate, nitro, -CN.

In one embodiment the invention relates to a compound according to **Formula III** for use in the treatment of a cancer exhibiting genotoxic stress-induced IKK/NF-κB activation 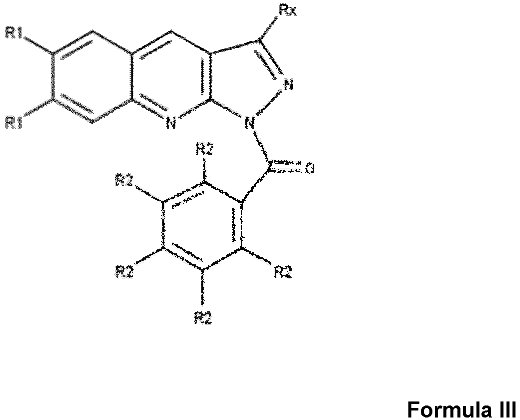 wherein
**R1** = can be the same or different, H, OH, halogen (preferably Br, Cl or F), alkyl (preferably C1 to C3 alkyl), alkenyl, alkynyl, alkoxy, alkyl carbonyl, carboxyl, alkoxy carbonyl, amine (preferably primary, secondary or tertiary amine),
**Rx** = amine (preferably primary, secondary or tertiary amine), OH, H, alkyl (preferably C1 to C3 alkyl), and
**R2** = can be the same or different, halogen (preferably Cl, Br, F), alkyl (preferably C1 to C6 alkyl), haloalkyl, OH, alkoxy (preferably OCH₃, OCH₂CH₃), alkoxy carbonyl (preferably - COOCH₃, - CH₂COOCH₃, -CH₂CH₂COOCH₃), cycloalkyl (preferably C3 to C6 cycloalkyl), aryl (preferably phenyl), alkyl-cycloalkyl, C1 to C5 alkyl-aryl, heterocyclyl, heteroaryl, carboxamide, CO-Cx alkyl carboxamide (preferably CON(CH₂CH₂)₂O); amine (preferably primary, secondary or tertiary amine), carbamate, nitro, -CN, O.

In one embodiment the invention relates to a compund according to **Formula III** for use in the treatment of a cancer exhibiting genotoxic stress-induced IKK/NF-κB activation, wherein
**R1** = can be the same or different, H, OH, halogen (preferably Br, Cl or F), alkoxy, amine (preferably primary, secondary or tertiary amine),
**Rx** = amine (preferably primary, secondary or tertiary amine), OH, H, alkyl (preferably C1 to C3 alkyl), and
**R2** = can be the same or different, H, alkoxy (preferably OCH₃, OCH₂CH₃), cycloalkyl (preferably C3 to C6 cycloalkyl), aryl (preferably phenyl), alkyl-cycloalkyl, heterocyclyl, heteroaryl, amine (preferably primary, secondary or tertiary amine), carboxamide.

In one embodiment the invention relates to a compund according to **Formula IV** for use in the treatment of a cancer exhibiting genotoxic stress-induced IKK/NF-κB activation, 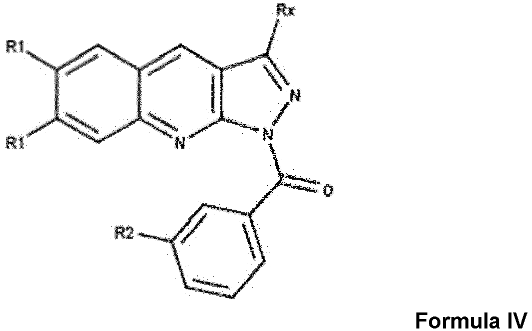 wherein
**R1** = can be the same or different, H, OH, halogen (preferably Br, Cl or F), alkyl (preferably C1 to C3 alkyl), alkenyl, alkynyl, alkoxy (preferably OCH₃, OCH₂CH₃), alkyl carbonyl, carboxyl, alkoxy carbonyl, amine (preferably primary, secondary or tertiary amine),
**Rx** = amine (preferably primary, secondary or tertiary amine), H, OH, alkyl (preferably C1 to C3 alkyl), and
**R2** = halogen (preferably Cl, Br, F), alkyl (preferably C1 to C6 alkyl), haloalkyl, OH, alkoxy (preferably OCH₃, OCH₂CH₃), alkoxy carbonyl (preferably - COOCH₃, -CH₂COOCH₃, - CH₂CH₂COOCH₃), cycloalkyl (preferably C3 to C6 cycloalkyl), aryl (preferably phenyl), alkyl-cycloalkyl, C1 to C3 alkyl-aryl, heterocyclyl, heteroaryl, carboxamide, CO-Cx alkyl carboxamide (preferably CON(CH₂CH₂)₂O); amine (preferably primary, secondary or tertiary amine), carbamate, nitro, -CN, O.

In one embodiment the compound according to **Formula IV** is characterized in that **R1** can be the same or different and is selected from the group of H, OH, halogen (preferably Br, Cl or F), alkoxy (preferably OCH₃, OCH₂CH₃) and amine (preferably primary, secondary or tertiary amine).

In one embodiment the compound according to **Formula IV** is characterized in that **R2** is H, alkoxy (preferably OCH₃, OCH₂CH₃), heterocyclyl or heteroaryl comprising one or more of N, O and/or S, amine (preferably primary, secondary or tertiary amine).

In one embodiment the invention relates to a compund according to **Formula V** for use in the treatment of a cancer exhibiting genotoxic stress-induced IKK/NF-κB activation 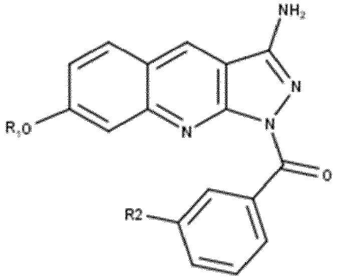 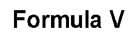 wherein
**R1=** H, alkyl (preferably C1 to C6 alkyl), alkenyl, alkynyl, alkoxy, carbonyl, carboxyl, alkoxy carbonyl, carboxamide, CO-Cx alkyl carboxamide (preferably CON(CH₂CH₂)₂O), and
**R2** = H, alkyl (preferably C1 to C6 alkyl), OH, alkoxy (preferably OCH₃, OCH₂CH₃), cycloalkyl (preferably C3 to C6 cycloalkyl), aryl (preferably phenyl), alkyl-cycloalkyl, heterocyclyl, heteroaryl, amine (preferably primary, secondary or tertiary amine), carboxamide.

In one embodiment the compound according to **Formula V** is characterized in that **R1** is H or alkyl (preferably C1 to C6 alkyl).

In one embodiment the compound according to **Formula V** is characterized in that **R2** is H, alkoxy (preferably OCH₃, OCH₂CH₃), heterocyclyl or heteroaryl comprising one or more of N, O and/or S, amine (preferably primary, secondary or tertiary amine).

In a preferred embodiment the compound of the invention is selected from the group provided in **Table 1**. In a preferred embodiment the invention relates to the compounds in **Table 1** for use in the treatment of a cancer exhibiting gentoxic stress-induced IKK/NF- κB activation.

**Table 1** shows exemplary compounds of the invention suitable for the medical use described herein. Any structure may represent an embodiment of the invention and/or be employed in any of the aspects or embodiments of the invention described herein.

**Table 1: Preferred compounds of the present invention.**

| **Entry** | **Name** | **Example** - **SMILES** | **Structure** |
|---|---|---|---|
| 1 | MW05 | COC1=CC2=C(C=C1) C=C1C(N)=NN(C(=O )C3=CC(=CC=C3)N( C)C)C1=N2 | 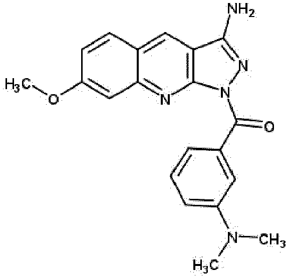 |
| 2 | MW05-E1 | COC1=CC2=C(C=C1) C=C1C(N)=NN(CC3= CC(=CC=C3)N(C)C)C 1=N2 | 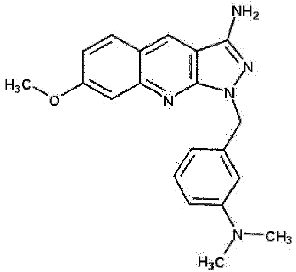 |
| 3 | MW05-E2 | COC1=CC2=C(C=C1) N=C1N(N=C(N)C1= C2)C(=O)C1=CC(=C C=C1)N(C)C | 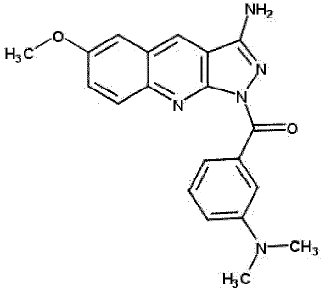 |
| 4 | MW05-E3 | CCN(CC)C1=CC(=CC =C1)C(=O)N1N=C(N )C2=C1N=C1C=C(O C)C=CC1=C2 | 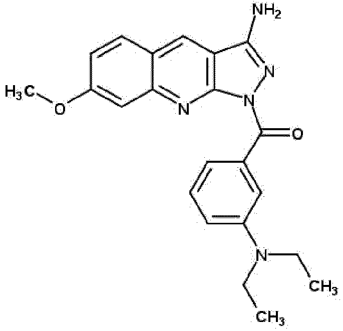 |
| 5 | MW05-E4 | COC1=CC2=C(C=C1) C=C1C(N)=NN(C(=O )C3=CC=C(C=C3)N( C)C)C1=N2 | 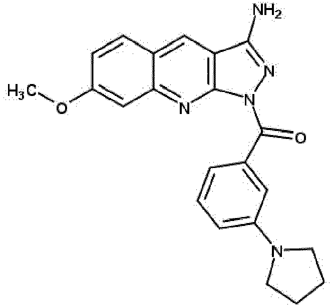 |
| 6 | MW05-E5 | COC1=CC2=C(C=C1) C=C1C(N)=NN(C(=O )C3=CC(N)=CC=C3) C1=N2 | 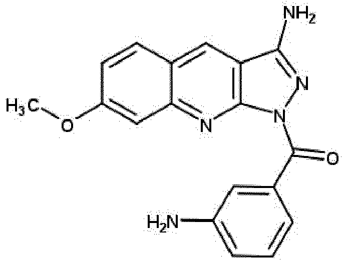 |
| 7 | MW05-E6 | COC1=CC2=C(C=C1) C=C1C(N)=NN(C(=O )C3=C(C=CC=C3)N( C)C)C1=N2 | 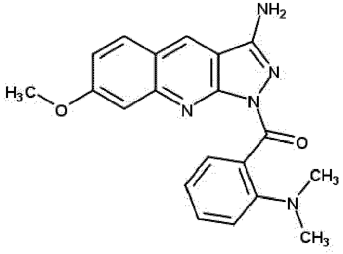 |
| 8 | MW05-E7 | CN(C)C1=CC=C(C=C 1)C(=O)N1N=C(N)C 2=C1N=C1C=C(F)C= CC1=C2 | 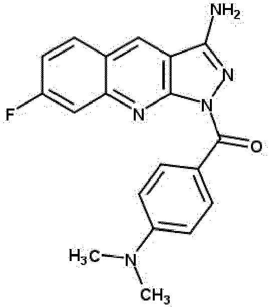 |
| 9 | MW05-E8 | COC1=CC2=C(C=C1) C=C1C(=NN(C(=O)C 3=CC(=CC=C3)N(C) C)C1=N2)N(C)C | 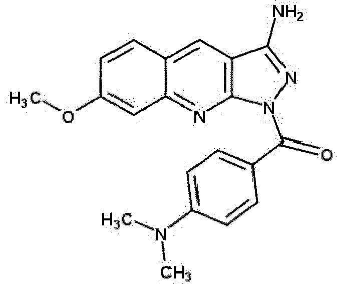 |
| 10 | MW05-E9 | CNC1=CC(=CC=C1)C (=O)N1N=C(N)C2=C 1N=C1C=C(OC)C=C C1=C2 | 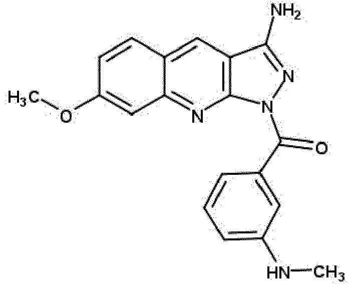 |
| 11 | MW05-E10 | COC1=CC2=C(C=C1) C=C1C(=NN(C(=O)C 3=CC(=CC=C3)N(C) C)C1=N2)N(C)C | 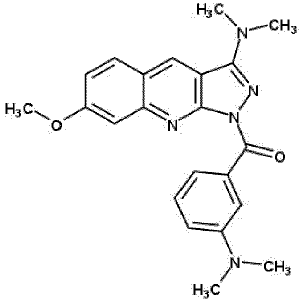 |
| 12 | MW05-E11 | COC1=CC2=C(C=C1) C=C1C(N)=NN(C1= N2)C1=CC(=CC=C1) N(C)C | 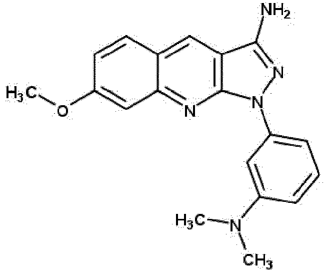 |
| 13 | MW05-E13 | COC1=CC2=C(C=C1) C=C1C(N)=NN(C(=O )C3=C(OC)C(OC)=C C=C3)C1=N2 | 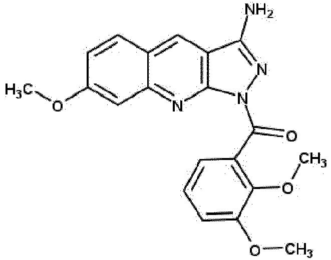 |
| 14 | MW05-E14 | COC1=CC2=C(C=C1) C=C1C(N)=NN(C(=O )CN3CCOCC3)C1=N 2 | 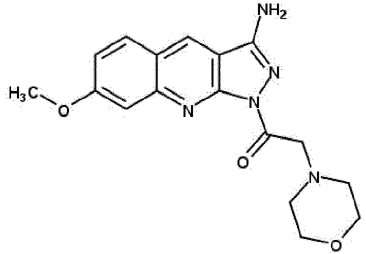 |
| 15 | MW05-E15 | COC1=CC2=C(C=C1) C=C1C(N)=NN(C(=O )C3=CN=CC=C3)C1= N2 | 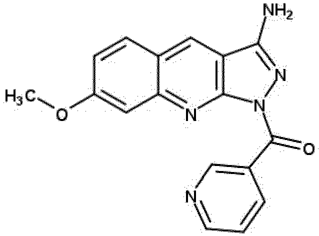 |
| 16 | MW05-E16 | NC1=NN(C(=O)C2= CN=CC=C2)C2=C1C =C1C=CC(O)=CC1= N2 | 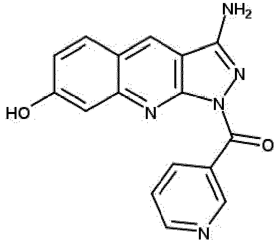 |
| 17 | MW05-E17 | COC1=CC2=C(C=C1) C=C1C(N)=NN(C(=O )C3=CC(=NC=C3)N3 CCCC3)C1=N2 | 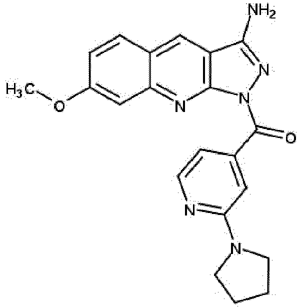 |
| 18 | MW05-E18 | COC1=CC2=C(C=C1) C=C1C(N)=NN(C(=O )C3=CC(=NC=C3)N( C)C)C1=N2 | 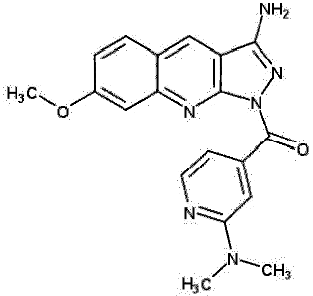 |
| 19 | MW05-E19 | COC1=CC2=C(C=C1) C=C1C(C)=NN(C(=O )C3=CC(=CC=C3)N( C)C)C1=N2 | 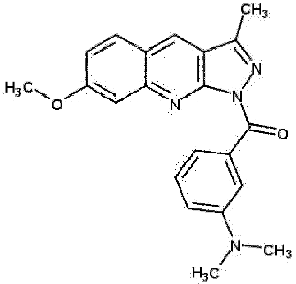 |
| 20 | MW05-E20 | COC1=CC2=C(C=C1) C=C1C=NN(C(=O)C3 =CC(=CC=C3)N(C)C) C1=N2 | 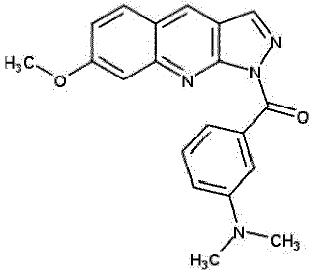 |
| 21 | MW05-E21 | CN(C)C1=CC(=CC=C 1)C(=O)N1N=C(N)C 2=C1N=C1C=CC=CC 1=C2 | 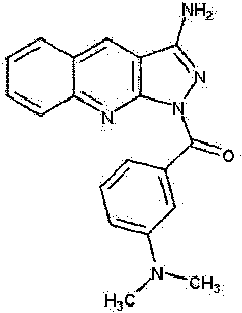 |
| 22 | MW05-E22 | CN(C)C1=CC(=CC=C 1)C(=O)N1N=C(N)C 2=C1N=C1C=C(O)C =CC1=C2 | 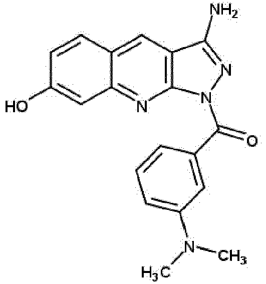 |
| 23 | MW05-E23 | CN(C)C1=CC(=CC=C 1)C(=O)N1N=C(N)C 2=C1N=C1C=C(C=C C1=C2)N(C)C | 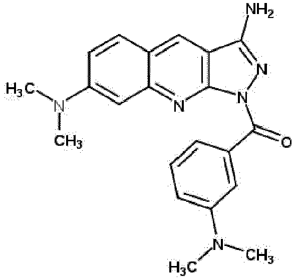 |
| 24 | MW05-E24 | COC1=CC2=C(C=C1) C=C1C(N)=NN(CC(= O)C3=CC(=CC=C3)N (C)C)C1=N2 | 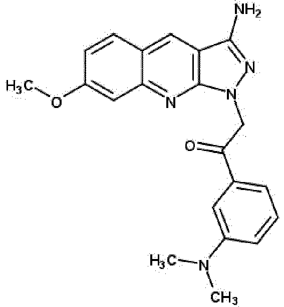 |
| 25 | MW05-E25 | COC1=CC2=C(C=C1) C=C1C(N)=NN(C1= N2)S(=O)(=O)C1=C C(=CC=C1)N(C)C | 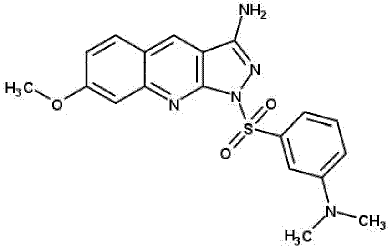 |
| 26 | MW05-E26 | COC1=CC2=C(C=C1) C=C1C(N)=NN(C(=O )C3=CC(=CC=C3)N3 CCOCC3)C1=N2 | 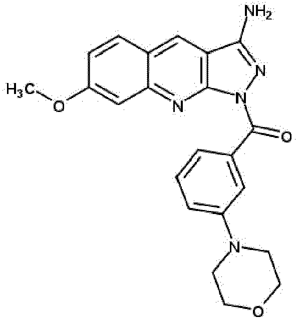 |
| 27 | MW05-E27 | COC1=CC2=C(C=C1) C=C1C(N)=NN(C(=O )NC3=CC(=CC=C3)N (C)C)C1=N2 | 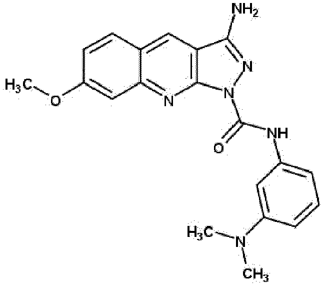 |
| 28 | MW05-E28 | COC1=CC2=C(C=C1) C=C1C(N)=NN(C(C) C3=CC(=CC=C3)N(C )C)C1=N2 | 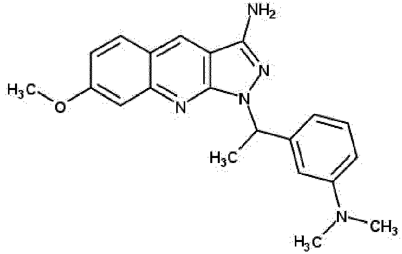 |
| 29 | MW05-E29 | CCC(N1N=C(N)C2= C1N=C1C=C(OC)C= CC1=C2)C1=CC(=CC =C1)N(CC)CC | 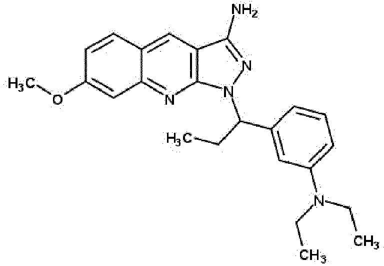 |

In one embodiment the compound of the invention is preferably selected from the group of MW05 and MW05-E09.

As described in more detail herein, the invention relates to the following compounds as such, and their use in treating one or more of the medical indications described herein. The compounds of the Formulae **VI, VII, VIII** and **IX,** as represented by the exemplary compounds listed in Table 2, are considered novel as such and exhibit structures not previously disclosed. These compounds also exhibit the benefical properties of the invention and are intended for the medical use disclosed herein, but are not limited thereto.

In one embodiment the invention trelates to a compound according to **Formula VI,** 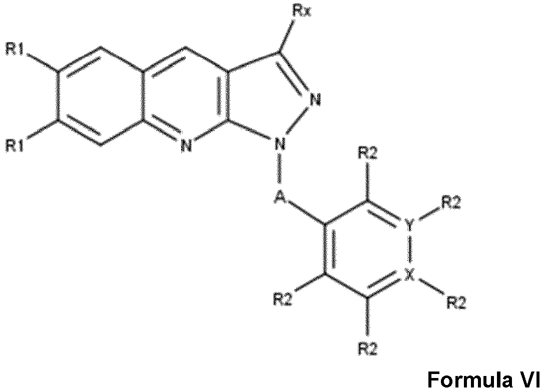 wherein
**R1** = can be the same or different, H, OH, halogen (preferably Br, Cl or F), alkyl (preferably C1 to C6 alkyl), alkoxy (preferably OCH₃, OCH₂CH₃), amine (preferably primary, secondary or tertiary amine),
**Rx** = amine (preferably primary, secondary or tertiary amine), OH, H, alkyl (preferably C1 to C3 alkyl),
**A=** alkyl (preferably branched C1 to C6 alkyl), alkenyl, alkynyl, alkoxy, alkyl carbonyl, carbonyl, carboxyl, cycloalkyl (preferably C3 to C6 cycloalkyl), aryl (preferably phenyl), sulfonyl, carboxamide,
**X** and **Y** are the same or different = C or N, and
**R2** = can be the same or different, H, alkoxy (preferably OCH₃, OCH₂CH₃), alkyl (preferably C1 to C6 alkyl), haloalkyl, OH, cycloalkyl (preferably C3 to C6 cycloalkyl), aryl (preferably phenyl), alkyl-cycloalkyl, heterocyclyl, heteroaryl, carboxamide, CO-Cx alkyl carboxamide (preferably C=ON(CH₂CH₂)₂°), amine (preferably primary, secondary or tertiary amine), nitro, -CN, O,
wherein when **Rx** is a primary or secondary amine and **R1** is OCH₃
   - **X** and/or **Y** are N and at least one of **R2** is alkoxy (preferably OCH₃, OCH₂CH₃), alkyl (preferably C1 to C6 alkyl), haloalkyl, OH, cycloalkyl (preferably C3 to C6 cycloalkyl), aryl (preferably phenyl), alkyl-cycloalkyl, heterocyclyl, heteroaryl, carboxamide, CO-Cx alkyl carboxamide (preferably C=ON(CH₂CH₂)₂°), amine (preferably primary, secondary or tertiary amine), nitro, -CN, O, and/or
   - **A** is alkyl (preferably branched C1 to C6 alkyl), alkenyl, alkynyl, alkoxy (preferably OCH₃, OCH₂CH₃), alkyl carbonyl, carboxyl, cycloalkyl (preferably C3 to C6 cycloalkyl), aryl (preferably phenyl), sulfonyl, carboxamide, and/or
   - at least one of **R2** is alkyl (preferably C1 to C6 alkyl), haloalkyl, OH, cycloalkyl (preferably C3 to C6 cycloalkyl), aryl (preferably phenyl), alkyl-cycloalkyl, heteroaryl, carboxamide, CO-Cx alkyl carboxamide (preferably C=ON(CH₂CH₂)₂), nitro, -CN, O, and/or
   - at least one of **R2** is a 5-membered heterocyclyl comprising one or more of N, S and/or O and at least one of **X** or **Y** is N.

In one embodiment the invention relates to a compound according to **Formula VII,** 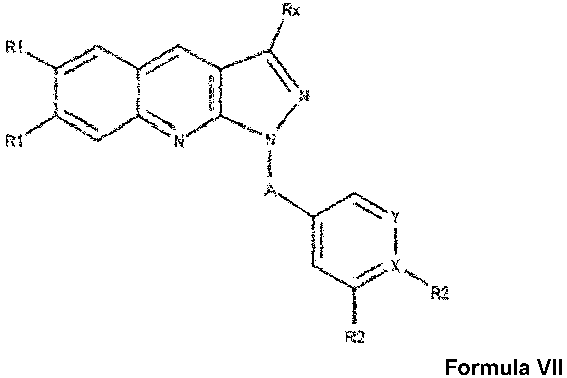 wherein
**R1** = can be the same or different, H, OH, halogen (preferably Br, Cl or F), alkoxy (preferably OCH₃, OCH₂CH₃), amine (preferably primary, secondary or tertiary amine),
**Rx** = amine (preferably primary, secondary or tertiary amine), OH, H, alkyl (preferably C1 to C3 alkyl),
**A=** alkyl (preferably branched C1 to C6 alkyl), alkenyl, alkynyl, alkoxy, alkyl carbonyl, carbonyl, carboxyl, cycloalkyl (preferably C3 to C6 cycloalkyl), aryl (preferably phenyl), sulfonyl, carboxamide, and
**R2** = can be the same or different, H, alkoxy (preferably OCH₃, OCH₂CH₃), alkyl (preferably C1 to C3 alkyl), cycloalkyl (preferably C3 to C6 cycloalkyl), aryl (preferably phenyl), alkyl-cycloalkyl, heterocyclyl (preferably C4 to C6 heterocyclyl), heteroaryl (preferably C4 to C6 heteroaryl), amine (preferably primary, secondary or tertiary amine),
wherein when **Rx** is a primary or secondary amine and **R1** is OCH₃
   - **X** and/or **Y** are N and at least one of **R2** is alkoxy (preferably OCH₃, OCH₂CH₃), alkyl (preferably C1 to C3 alkyl), cycloalkyl (preferably C3 to C6 cycloalkyl), aryl (preferably phenyl), alkyl-cycloalkyl, (preferably C4 to C6 heterocyclyl), heteroaryl (preferably C4 to C6 heteroaryl), amine (preferably primary, secondary or tertiary amine), and/or
   - - **A** is alkyl (preferably branched alkyl), alkenyl, alkynyl, alkoxy, alkyl carbonyl, carboxyl, cycloalkyl (preferably C3 to C6 cycloalkyl), aryl (preferably phenyl), sulfonyl, carboxamide, and/or
   - at least one of **R2** is alkyl (preferably C1 to C3 alkyl), cycloalkyl (preferably C3 to C6 cycloalkyl), aryl (preferably phenyl), alkyl-cycloalkyl, heteroaryl, a 6-membered heterocyclyl, and/or
   - at least one of **R2** is a 5-membered heterocyclyl comprising one or more of N, S and/or O and at least one of **X** or **Y** is N.

In one embodiment the invention relates to a coumpound according to **Formula VII,** wherein
**R1** = can be the same or different, H, OH, halogen (preferably Br, Cl or F), alkoxy (preferably OCH₃, OCH₂CH₃), amine (preferably primary, secondary or tertiary amine),
**Rx** = amine (preferably primary, secondary or tertiary amine), OH, H, alkyl (preferably C1 to C3 alkyl),
**A=** alkyl (preferably branched alkyl), alkyl carbonyl, carbonyl, sulfonyl, carboxamide, and
**R2** = can be the same or different, H, alkoxy (preferably OCH₃, OCH₂CH₃), heterocyclyl (preferably C4 to C6 heterocyclyl), amine (preferably primary, secondary or tertiary amine),
wherein when Rx is a primary or secondary amine and **R1** is OCH₃
   - **X** and/or **Y are** N and at least one of **R2** is alkoxy (preferably OCH₃, OCH₂CH₃), amine (preferably primary, secondary or tertiary amine), and/or
   **- - A** is alkyl (preferably branched alkyl), alkyl carbonyl, sulfonyl, carboxamide and/or
   - at least one of **R2** a 6-membered heterocyclyl comprising one or more of N, S and/or O, and/or
   - at least one of **R2** is a 5-membered heterocyclyl comprising one or more of N, S and/or O and at least one of **X** or **Y** is N.!n one embodiment the invention relates to a coumpound according to **Formula III,** wherein

In one embodiment the invention relates to a compound according to **Fomula VIII** 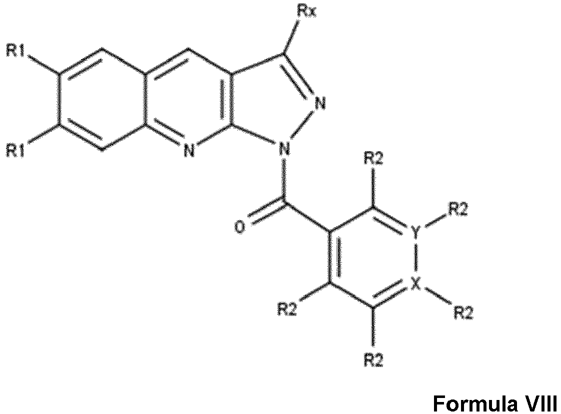 wherein
**R1** = can be the same or different, H, OH, halogen (preferably Br, Cl or F), alkyl (preferably C1 to C6 alkyl), alkoxy (preferably OCH₃, OCH₂CH₃), amine (preferably primary, secondary or tertiary amine),
**Rx** = amine (preferably primary, secondary or tertiary amine), OH, H, alkyl (preferably C1 to C3 alkyl),
**X** and **Y** are the same or different = C or N, and
**R2** = can be the same or different, H, alkoxy (preferably OCH₃, OCH₂CH₃), alkyl (preferably C1 to C6 alkyl), haloalkyl, OH, cycloalkyl (preferably C3 to C6 cycloalkyl), aryl (preferably phenyl), alkyl-cycloalkyl, heterocyclyl, heteroaryl, carboxamide, CO-Cx alkyl carboxamide (preferably C=ON(CH₂CH₂)₂), amine (preferably primary, secondary or tertiary amine), nitro, - CN, O,
wherein when **Rx** is a primary or secondary amine and **R1** is OCH₃
   - **X** and/or **Y** are N and at least one of **R2** is alkoxy (preferably OCH₃, OCH₂CH₃), alkyl (preferably C1 to C6 alkyl), haloalkyl, OH, cycloalkyl (preferably C3 to C6 cycloalkyl), aryl (preferably phenyl), alkyl-cycloalkyl, heterocyclyl, heteroaryl, carboxamide, CO-Cx alkyl carboxamide (preferably C=ON(CH₂CH₂)₂), amine (preferably primary, secondary or tertiary amine), nitro, -CN, O, and/or
   - at least one of **R2** is alkyl (preferably C1 to C6 alkyl), haloalkyl, OH, cycloalkyl (preferably C3 to C6 cycloalkyl), aryl (preferably phenyl), alkyl-cycloalkyl, heteroaryl, carboxamide, CO-Cx alkyl carboxamide (preferably C=ON(CH₂CH₂)₂), nitro, -CN, O, and/or
   - at least one of **R2** is a 5-membered heterocyclyl comprising one or more of N, S and/or O and at least one of **X** or **Y** is N.

In one embodiment the invention relates to a compound according to **Fomula IX,** 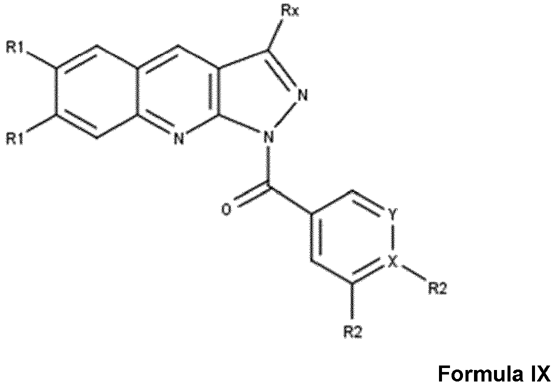 wherein
**R1** = can be the same or different, H, OH, halogen (preferably Br, Cl or F), alkoxy (preferably OCH₃, OCH₂CH₃), amine (preferably primary, secondary or tertiary amine),
**Rx** = amine (preferably primary, secondary or tertiary amine), OH, H, alkyl (preferably C1 to C3 alkyl), and
**R2** = can be the same or different, H, alkoxy (preferably OCH₃, OCH₂CH₃), alkyl (preferably C1 to C3 alkyl), cycloalkyl (preferably C3 to C6 cycloalkyl), aryl (preferably phenyl), alkyl-cycloalkyl, heterocyclyl (preferably C4 to C6 heterocyclyl), heteroaryl (preferably C4 to C6 heteroaryl), amine (preferably primary, secondary or tertiary amine),
wherein when **Rx** is a primary or secondary amine and **R1** is OCH₃
   - **X** and/or **Y are** N and at least one of **R2** is alkoxy (preferably OCH₃, OCH₂CH₃), alkyl (preferably C1 to C3 alkyl), cycloalkyl (preferably C3 to C6 cycloalkyl), aryl (preferably phenyl), alkyl-cycloalkyl, (preferably C4 to C6 heterocyclyl), heteroaryl (preferably C4 to C6 heteroaryl), amine (preferably primary, secondary or tertiary amine), and/or
   - at least one of **R2** is alkyl (preferably C1 to C3 alkyl), cycloalkyl (preferably C3 to C6 cycloalkyl), aryl (preferably phenyl), alkyl-cycloalkyl, heteroaryl, a 6-membered heterocyclyl, and/or
   - at least one of **R2** is a 5-membered heterocyclyl comprising one or more of N, S and/or O and at least one of **X** or **Y** is N.

In one embodiment the invention relates to a coumpound according to **Formula IX,** wherein
**R1** = can be the same or different, H, OH, halogen (preferably Br, Cl or F), alkoxy (preferably OCH₃, OCH₂CH₃), amine (preferably primary, secondary or tertiary amine),
**Rx** = amine (preferably primary, secondary or tertiary amine), OH, H, alkyl (preferably C1 to C3 alkyl), and
**R2** = can be the same or different, H, alkoxy (preferably OCH₃, OCH₂CH₃), heterocyclyl (preferably C4 to C6 heterocyclyl), amine (preferably primary, secondary or tertiary amine),
wherein when **Rx** is a primary or secondary amine and **R1** is OCH₃
   - **X** and/or **Y** are N and at least one of **R2** is alkoxy (preferably OCH₃, OCH₂CH₃), amine (preferably primary, secondary or tertiary amine), and/or
   - at least one of **R2** a 6-membered heterocyclyl comprising one or more of N, S and/or O, and/or
   - at least one of **R2** is a 5-membered heterocyclyl comprising one or more of N, S and/or O and at least one of **X** or **Y** is N.

In a preferred embodiment the compound of the invention is selected from the group provided in **Table 2.** In a preferred embodiment the invention relates to the compounds in **Table 2** useful for inhibition of gentoxic stress-induced IKK/NF- κB activation.

**Table 2** shows exemplary compounds of the invention suitable for the medical use described herein. Any structure may represent an embodiment of the invention and/or be employed in any of the aspects or embodiments of the invention described herein.

**Table 2: Preferred compounds of the present invention.**

| **Entry** | **Name** | **Example** - **SMILES** | **Structure** |
|---|---|---|---|
| 1 | MW05-E7 | CN(C)C1=CC=C(C=C 1)C(=O)N1N=C(N)C 2=C1N=C1C=C(F)C= CC1=C2 | 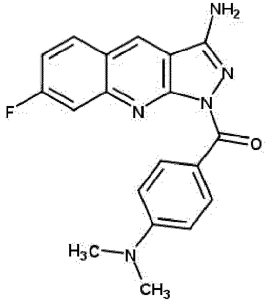 |
| 2 | MW05-E16 | NC1=NN(C(=O)C2= CN=CC=C2)C2=C1C =C1C=CC(O)=CC1= N2 | 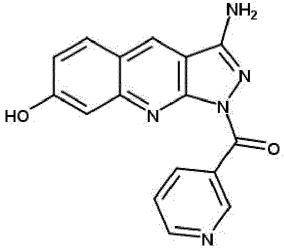 |
| 3 | MW05-E17 | COC1=CC2=C(C=C1) C=C1C(N)=NN(C(=O )C3=CC(=NC=C3)N3 CCCC3)C1=N2 | 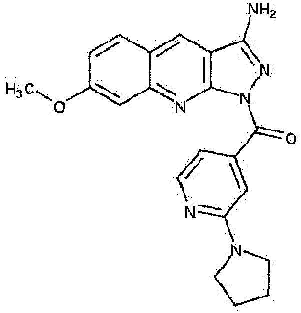 |
| 3 | MW05-E18 | COC1=CC2=C(C=C1) C=C1C(N)=NN(C(=O )C3=CC(=NC=C3)N( C)C)C1=N2 | 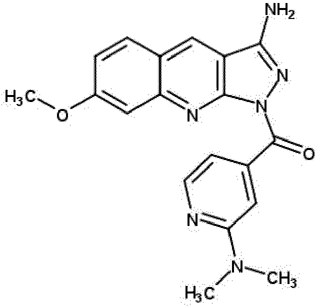 |
| 4 | MW05-E19 | COC1=CC2=C(C=C1) C=C1C(C)=NN(C(=O )C3=CC(=CC=C3)N( C)C)C1=N2 | 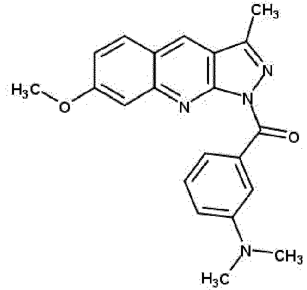 |
| 5 | MW05-E20 | COC1=CC2=C(C=C1) C=C1C=NN(C(=O)C3 =CC(=CC=C3)N(C)C) C1=N2 | 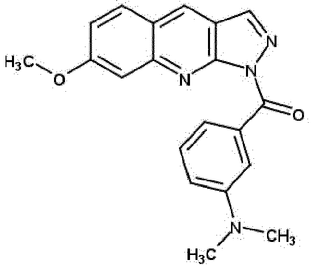 |
| 6 | MW05-E21 | CN(C)C1=CC(=CC=C 1)C(=O)N1N=C(N)C 2=C1N=C1C=CC=CC 1=C2 | 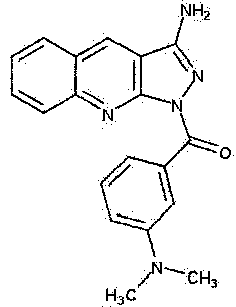 |
| 7 | MW05-E22 | CN(C)C1=CC(=CC=C 1)C(=O)N1N=C(N)C 2=C1N=C1C=C(O)C =CC1=C2 | 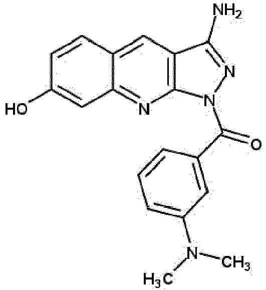 |
| 8 | MW05-E23 | CN(C)C1=CC(=CC=C 1)C(=O)N1N=C(N)C 2=C1N=C1C=C(C=C C1=C2)N(C)C | 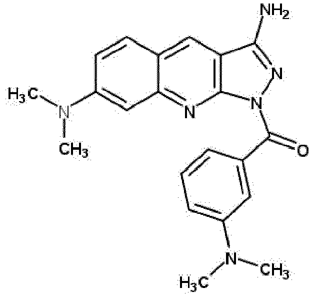 |
| 9 | MW05-E24 | COC1=CC2=C(C=C1) C=C1C(N)=NN(CC(= O)C3=CC(=CC=C3)N (C)C)C1=N2 | 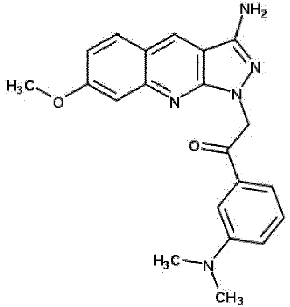 |
| 10 | MW05-E25 | COC1=CC2=C(C=C1) C=C1C(N)=NN(C1= N2)S(=O)(=O)C1=C C(=CC=C1)N(C)C | 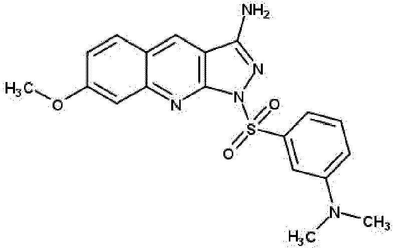 |
| 11 | MW05-E26 | COC1=CC2=C(C=C1) C=C1C(N)=NN(C(=O )C3=CC(=CC=C3)N3 CCOCC3)C1=N2 | 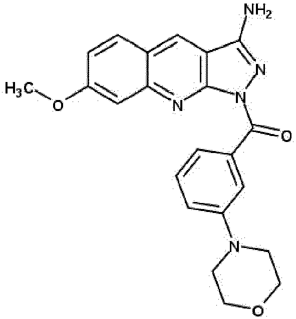 |
| 12 | MW05-E27 | COC1=CC2=C(C=C1) C=C1C(N)=NN(C(=O )NC3=CC(=CC=C3)N (C)C)C1=N2 | 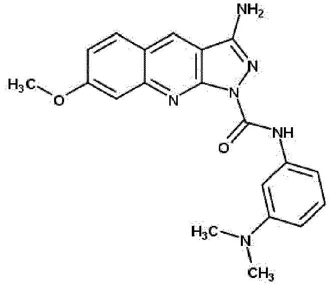 |
| 13 | MW05-E28 | COC1=CC2=C(C=C1) C=C1C(N)=NN(C(C) C3=CC(=CC=C3)N(C )C)C1=N2 | 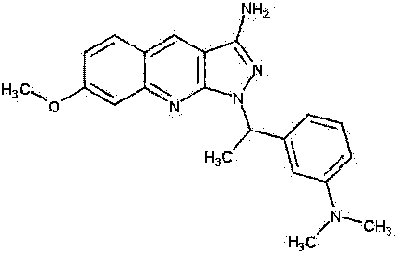 |
| 14 | MW05-E29 | CCC(N1N=C(N)C2= C1N=C1C=C(OC)C= CC1=C2)C1=CC(=CC =C1)N(CC)CC | 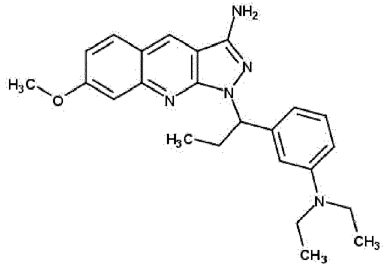 |

In one embodiment the invention relates to the compound according to any one of **Formulas I, II, III, IV, V, VI, VII, VII** and **IX** for use in the treatment of a subject suffering from cancer exhibiting genotoxic stress-induced IKK/NF-κB activation.

In one embodiment the invention relates to a method of treating and/or preventing a cancer exhibiting genotoxic stress-induced IKK/NF-κB activation, said method comprising the administration of one or more of the above compounds or compounds falling under the formulae described above to a subject in need thereof, in particular the compounds of **Formula I, II, III, IV, V, Vi, VII, VII** and **IX,** and preferred embodiments and combinations thereof described above.

In one embodiment, the invention relates to a method for inhibiting genotoxic stress-induced IKK/NF-κB activation in a subject, preferably by inhibiting CLK2 and/or CLK4, comprising administering a compound as described herein to a subject in need thereof.

In one embodiment the invention relates to a method of treatment of a cancer in a subject in need thereof comprising
(a) Identifying the subject as having a cancer exhibiting genotoxic stress-induced IKK/NF-κB activation that causes growth of the cancer,
(b) Selectively blocking activation of IKK/NF-κB in response to genotoxic stress upstream of the IKK complex in the subject without blocking IKK/NF-κB activation upon signaling caused by other pathways in the subject by administering to the subject a compound according to any one of **Formulas I to IX.**

In one embodiment the compound is more effective in inhibiting NF-κB-signaling induced by genotoxic stress compared to inhibiting NF-κB-signaling induced by TNF-alpha and/or IL-1β. This feature relates to a functional feature of the compound described herein suitable for definition of the compound and for differentiation from other compounds described in the art.

In one embodiment the cancer is associated with genomic instability due to defective DNA-repair mechanisms, and/or said cancer is associated with NF-κB-mediated resistance to therapy-induced tumor cell apoptosis. In one embodiment the cancer associated with genomic instability comprises a mutation of BRAC1 and/or BRAC2.

In one embodiment the compound according to any one of **Formulas I, II, III, IV, V, Vi, VII, VII** and **IX** selectively inhibits CLK2 and/or CLK4. In embodiments the compounds described herein may be termed CLK2 and/or CLK4 inhibitors. In embodiments the compounds according to the present invention exhibit the desired property of inhibiting CLK2 and/or CLK4. A skilled person is capable of determining whether any given compound exhibits the desired properties of the invention.

In embodiments, the compounds of the present invention are defined by the presence of a functional property, namely the inhibition of CLK2 and/or CLK4. Said inhibition may be evident when using one or more of the in vitro (or other) assays described in detail in the examples. A skilled person is capable, without undue effort, of ascertaining whether any given compound within the compounds according to the present invention exhibits the desired properties using the guidance provided herein and their common general knowledge.

In embodiments, the compounds according to the present invention are used to treat a cancer characterized by expression of CLK2 and/or CLK4, preferably pathological and/or overexpression of CLK2 and/or CLK4. A skilled person is capable, without undue effort, of ascertaining whether any given cancer may be considered to express, overexpress and/or pathologically express CLK2 and/or CLK4.

In one embodiment the treatment comprises the inhibition of CLK2 in a subject suffering from a cancer associated with genotoxic stress-induced IKK/NF-κB activation.

In one embodiment the treatment comprises the inhibition of CLK4 in a subject suffering from a cancer associated with genotoxic stress-induced IKK/NF-κB activation.

In one embodiment the treatment comprises the inhibition of CLK2 and/or CLK4 in a subject suffering from a cancer associated with genotoxic stress-induced IKK/NF-κB activation.

In one embodiment the compound is administered in combination with one or more genotoxic stress-inducing (DNA damage-inducing) cancer therapies, such as genotoxic stress-inducing chemotherapy and/or irradiation therapy.

In one embodiment the compound is administered in combination with irradiation therapy. In another embodiment of the present invention the compound is administered in combination with genotoxic stress-inducing chemotherapy.

In one aspect the invention relates to an *in vitro* method for the inhibition of genotoxic stress-induced NF-κB signaling or inhibition of DNA repair mechanisms, preferably in a cell-based assay, comprising the use of the compound according to the present invention.

In one aspect the invention relates to an *in vitro* method for the inhibition of CLK2 and/or CLK4, preferably in a cell-based assay, comprising the use of the compound according to the present invention.

In one embodiment the compound of the present invention is used in an in vitro method for the inhibition of DNA repair mechanisms, preferably in a cell based assay.

Furthermore, the present invention relates to a pharmaceutical composition for the treatment of a subject afflicted by a cancer associated with genotoxic stress-induced IKK/NF-κB activation, said composition comprising a compound according to the present invention and a pharmaceutically acceptable carrier substance.

All features described in the present specification may be employed to define any other embodiment or aspect of the invention, for example, features used to describe the medical use of one compound may be used to describe a medical use of another compound, and vice versa. Features used to describe any one or more compounds may be combined with any one or more of the medical uses of the invention. Similarly, structural features of the compounds may be used to describe the methods of the invention, other compounds of the invention or pharmaceutical composition and vice versa, according to the understanding of a skilled person.

### DETAILED DESCRIPTION OF THE INVENTION

The invention relates to chemical compounds useful as inhibitors of genotoxic stress-induced IKK/NF-κB activation. The invention relates to chemical compounds and their use in the treatment of a cancer associated with genotoxic stress, preferably a cancer associated with genotoxic stress-induced IKK/NF-κB activation.

### Chemical compounds:

With respect to the chemical compounds described herein, the term "alkyl" refers to a branched or unbranched saturated hydrocarbon group of 1 to 7 carbon atoms, such as methyl, ethyl, n-propyl, isopropyl, w-butyl, isobutyl, f-butyl, pentyl, hexyl, heptyl, and the like. Preferred alkyl groups have 1 to 7 carbon atoms, more preferably 1 to 4 carbon atoms. Any one or more of the alkyl groups described herein may be "substituted alkyls", wherein one or more hydrogen atoms are substituted with a substituent such as halogen, cycloalkyl, alkoxy, amino, hydroxyl, aryl, or carboxyl.

The term "alkenyl" refers to a straight, branched or cyclic hydrocarbon configuration and combinations thereof, including preferably 2 to 7 carbon atoms, more preferably 2 to 4 carbon atoms, that would form if a hydrogen atom is removed from an alkene, for example resulting in ethenyl, or the like.

The term "alkynyl" refers a straight, branched or cyclic hydrocarbon configuration and combinations thereof, including preferably 2 to 7 carbon atoms, more preferably 2 to 4 carbon atoms, that would form if a hydrogen atom is removed from an alkyne, for example resulting in ethynyl, or the like.

The term "cycloalkyl" refers to a configuration derived from a cycloalkane by removal of an atom of hydrogen, thereby forming preferably cyclopropyl, cyclobutyl, cyclopentyl or cyclohexyl, or the like.

The term "alkoxy" refers to a straight, branched or cyclic hydrocarbon configuration and combinations thereof, including preferably 1 to 7 carbon atoms, more preferably 1 to 4 carbon atoms, that include an oxygen atom at the point of attachment (such as O-alkyl). An example of an "alkoxy group" is represented by the formula -OR, where R can be an alkyl group, optionally substituted with an alkenyl, alkynyl, aryl, aralkyl, cycloalkyl, halogenated alkyl, or heterocycloalkyl group. Suitable alkoxy groups include methoxy, ethoxy, n-propoxy, i-propoxy, n-butoxy, i-butoxy, sec-butoxy, cyclohexyloxy, and the like.

"Alkoxycarbonyl" refers to an alkoxy substituted carbonyl radical (such as -C(=O)OR), wherein R represents an optionally substituted alkyl, aryl, aralkyl, cycloalkyl, cycloalkylalkyl or similar moiety.

The term "acyl" refers to configurations derived by the removal of one or more hydroxyl groups from an oxoacid containing a double bonded oxygen atom and an alkyl group, forming -RC(=O)-. The acyl therefore comprises carbonyl, which refers to a radical of the formula -C(=O)-. Carbonyl-containing groups include any substituent containing a carbon-oxygen double bond (C=O), including amides, carboxy groups, esters, ureas, carbamates, carbonates and ketones and aldehydes, such as substituents based on -COR or-RCHO where R is alkyl, heteroalkyl, hydroxyl, or a secondary, tertiary, or quaternary amine.

The term "aryl" refers to any carbon-based aromatic group including, but not limited to, benzene, and the like. The term "aromatic" also includes "heteroaryl group," which is defined as an aromatic group that has at least one heteroatom incorporated within the ring of the aromatic group. Examples of heteroatoms include, but are not limited to, nitrogen, oxygen, sulfur. The aryl group can be substituted with one or more groups including, but not limited to, alkyl, alkynyl, alkenyl, aryl, halide, nitro, amino, ester, ketone, aldehyde, hydroxy, carboxylic acid, or alkoxy, or the aryl group can be unsubstituted.

The term "heteroaryl" is further understood to mean saturated (heterocycloalkyl), partly unsaturated (heterocycloalkenyl) or unsaturated (heteroaryl) hydrocarbon rings containing from 3 to 15 carbon atoms in a mono- or bicyclic , fused, bridged or spirocyclic ring in which 1 to 5 carbon atoms of the 3 to 15 ring carbon atoms are replaced by heteroatoms such as nitrogen, oxygen or sulfur in which further the heteroatoms can be oxidized, for example N=O, S=O, SO₂. Non-limiting examples of heterocycles are acridinyl, azaindole (1H-pyrrolopyridinyl), azabenzimidazolyl, azaspirodecanyl, azepinyl, azetidinyl, aziridinyl, benzimidazolyl, benzofuranyl, dihydrobenzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, chromanyl, chromenyl, cinnolinyl, decahydrochinolinyl, 4,5-dihydrooxazolinyl, dioxazolyl, dioxazinyl, 1,3-dioxolanyl, 1,3-dioxolenyl, 3,3-dioxo[1,3,4]oxathiazinyl, 6H-1,5,2-dithiazinyl, dihydrofuro[2,3-b]-tetrahydrofuranyl, furanyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl (benzimidazolyl), isothiazolyl, isothiazolidinyl, isothiazolinyl, isoxazolyl, isoxazolinyl, isoxazolidinyl, 2-isoxazolinyl, ketopiperazinyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, 1,2-oxa-thiepanyl, 1,2-oxathiolanyl, 1,4-oxazepanyl, 1,4-oxazepinyl, 1,2-oxazinyl, 1,3-oxazinyl, 1,4-oxazinyl, oxazolidinyl, oxazolinyl, oxazolyl, oxetanyl, oxocanyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazolyl, pyridinyl, pyridyl, pyrimidinyl, pyrrolidinyl, pyrrolidinonyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisoquinolinyl, tetrahydroquinolinyl, tetrahydrofuranyl, tetrahydropyranyl, tetrahydropyridinyl, tetrahydrothiophenyl, tetrazinyl, tetrazolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, 1,2-thiazinyl, 1,3-thiazinyl, 1,4-thiazinyl, 1,3-thiazolyl, thiazolyl, thiazolidinyl, thiazolinyl, thienyl, thietanyl, thienothiazolyl, thienooxazolyl, thienoimidazolyl, thiomorpholinyl, thiophenolyl, thiophenyl, thiopyranyl, 1,2,3-triazinyl, 1,2,4-triazinyl, 1,3,5-triazinyl, 1,2,3-triazolyl, 1,2,4-triazolyl, 1,2,5-triazolyl, 1,3,4-triazolyl and xanthenyl.

The term 5- or 6-membered ring structure, optionally comprising one or more of N or O, relates preferably a cycloalkyl, cycloalkane and non-aromatic heterocycles (such as morpholine, piperidine, piperazine, thiomorpholine, tetrahydrofuran), aromatic cyclic structures such as phenyl, naphthalene, heterocyclic aromatic rings, such as furan, pyrrole, oxazole, thiophene, thiazole, pyrazole, imidazole, in addition to pyridine, pyrazine, pyrimidine, pyran, thiopyran, oxazine, azepine, thiepine, oxepane, and the like. The 5- or 6-membered cyclic structure preferably forms preferably forming a pyrazolyl, imidazolyl, pyridyl, pyrimidyl, pyridazyl, pyrazinyl ring.

The term "5-membered heterocyclyl or heteroaryl" refers to a configuration comprising a 5-membered heterocyclic optionally aromatic ring structure, comprising preferably C and one or more of N, O and/or S, preferably selected from a configuration if a hydrogen atom is removed from furan, pyrrole, oxazole, thiophene, thiazole, pyrazole, imidazole, and the like.

The "5- or 6-membered heterocyclyl or heteroaryl" as described herein relates preferably to a cycloalkyl, cycloalkane non-aromatic cyclic structures, such as cyclopentyl or cyclohexyl, and optionally to aromatic cyclic structures, such as phenyl, and the like.

The "6-membered heterocycle" as described herein relates preferably to a cycloalkyl, cycloalkane non-aromatic cyclic structures, such as cyclohexyl, or to aromatic cyclic structures, such as phenyl, and the like.

The "6-membered aromatic heterocycle, comprising one or more of N, O and/or S" as described herein refers to a configuration comprising a 6-membered ring structure comprising C and one or more of N, O and/or S, preferably selected from a configuration if a hydrogen atom is removed from pyridine, pyridazine, pyrimidine, pyrazine, pyran, triazine, thiazine, thiopyran, oxazine, and the like.

In some embodiments, the "6-membered aromatic heterocycle" comprises 1 or 2 N atoms, referring to a configuration comprising a 6-membered ring structure comprising C and 1 or 2 N atoms, preferably selected from pyridine, pyridazine, pyrimidine, pyrazine, triazine, thiazine, oxazine, and the like. Preferred heterocycles comprise only 1 or 2 N atoms.

Where reference is made to "C1-C7, C1-C5 orC1-C3" alkyl, cycloalkyl, alkoxy, aryl, or the like, the number of carbon atoms C1-C7 preferably refers to each of the substituents mentioned, although in some embodiments the shorter substituents of C1-C5 or C1-C3 apply to the alkyl, cycloalkyl and/or alkoxy groups, whereby aryl may remain preferably C1-C7, such as C6 phenyl.

The term "amine" refers to a group of the formula -NRR', where R and R' can be, independently, hydrogen or an alkyl, alkenyl, alkynyl, aryl, aralkyl, cycloalkyl, halogenated alkyl, or heterocycloalkyl group described above. The term "amide" or "amido" is represented by the formula -C(O)NRR', where R and R' independently can be a hydrogen, alkyl, alkenyl, alkynyl, aryl, aralkyl, cycloalkyl, halogenated alkyl, or heterocycloalkyl group described above. A suitable amido group is acetamido.

"Carbonyl" refers to a radical of the formula -C(O)-. Carbonyl-containing groups include any substituent containing a carbon-oxygen double bond (C=O), including acyl groups, amides, carboxy groups, esters, ureas, carbamates, carbonates and ketones and aldehydes, such as substituents based on -COR or-RCHO where R is an aliphatic, heteroaliphatic, alkyl, heteroalkyl, hydroxyl, or a secondary, tertiary, or quaternary amine, phenyl, a substituted phenyl (substituted with, for example, halogen, C1-C3 alkyl, alkoxy, amine), carboxyl, alkoxycarbonyl, amine, aryl.

The term "alkyl amino" refers to alkyl groups as defined above where at least one hydrogen atom is replaced with an amino group.

"Aminocarbonyl" alone or in combination, means an amino substituted carbonyl (carbamoyl) radical, wherein the amino radical may optionally be mono- or di-substituted, such as with alkyl, aryl, aralkyl, cycloalkyl, cycloalkylalkyl, alkanoyl, alkoxycarbonyl, aralkoxycarbonyl and the like. An aminocarbonyl group may be -N(R)-C(O)-R (wherein R is a substituted group or H) or-C(O)-N(R).

"Carboxyl" refers to a -COOH radical. Substituted carboxyl refers to -COOR where R is aliphatic, heteroaliphatic, alkyl, heteroalkyl, or a carboxylic acid or ester.

The term "hydroxyl" is represented by the formula -OH.

The term "hydroxyalkyl" refers to an alkyl group that has at least one hydrogen atom substituted with a hydroxyl group. The term "alkoxyalkyl group" is defined as an alkyl group that has at least one hydrogen atom substituted with an alkoxy group described above.

The term "arylalkyl" refers to an aryl group having an alkyl group, as defined above, attached to the aryl group, as defined above. An example of an aralkyl group is a benzyl group.

Optionally substituted groups, such as "optionally substituted alkyl," refers to groups, such as an alkyl group, that when substituted, have from 1-5 substituents, typically 1, 2 or 3 substituents, selected from alkoxy, optionally substituted alkoxy, acyl, acylamino, acyloxy, amino, aminoacyl, aminoacyloxy, aryl, carboxyalkyl, optionally substituted cycloalkyl, optionally substituted cycloalkenyl, halogen, optionally substituted heteroaryl, optionally substituted heterocyclyl, hydroxy, sulfonyl, thiol and thioalkoxy. In particular, optionally substituted alkyl groups include, by way of example, haloalkyl groups, such as fluoroalkyl groups, including, without limitation, trifluoromethyl groups. These potential optional substituents apply to any group of the formula disclosed herein where an optional substituent is recited.

The term "carboxamide" is represented by the formula -C(O)-N(R)-R.

The term "primary, secondary or tertiary amine" is represented by the formula -N(R)-R.

The term "carbamate" is represented by the formula -NR-C(O)-O-R.

The term "amide amine" is represented by the formula -NH-C(O)-NH-R,

The term "sulfide" is represented by the formula -S-R

The terms "amine sulfoxide", "sulfonamide", "sulfonamide amine" are preferably selected from the groups -N(R)-S(O)u-R, wherein u is 1 or 2, or -S(O)v-N(R)-R, wherein v is 1 or 2, preferably from the groups -NHSO₂CH₃, -SO₂NHCH₃, -NHSO₂N(CH₃)₂).

The terms "sulfoxide" or "sulfone are preferably selected from the groups -SOr-R, wherein r is 1-3, preferably from the groups -S(O)R, -SO2R, -SO2H, and SO3H.

The term "imide" is represented by the formula -C(O)-N(R)-C(O)-R'.

The term "sulfide" is represented by the formula -SR.

The term "sulfonyl" is represented by the formula -SO₂R.

For the definitions above, preferably the terms R, R' are independently selected from the group of H, alkyl, alkylhalo, alkoxy, or amine, and wherein X is halogen. The terms R, R' also comprise the possibility of any given group being appended to R.

The term "nitro" refers to an NO₂ group.

Optionally substituted groups, such as "optionally substituted" refers to groups, such as an alkyl group, that when substituted, have from 1-5 substituents, typically 1, 2 or 3 substituents.

Particular examples of the presently disclosed compounds include one or more asymmetric centers; thus these compounds can exist in different stereoisomeric forms. Accordingly, compounds and compositions may be provided as individual pure enantiomers or as stereoisomeric mixtures, including racemic mixtures. In certain embodiments the compounds disclosed herein are synthesized in or are purified to be in substantially enantiopure form, such as in a 90% enantiomeric excess, a 95% enantiomeric excess, a 97% enantiomeric excess or even in greater than a 99% enantiomeric excess, such as in enantiopure form.

Protected derivatives of the disclosed compound also are contemplated, for example for use in the synthesis of the disclosed compounds. A variety of suitable protecting groups for use with the disclosed compounds are disclosed in Greene and Wuts Protective Groups in Organic Synthesis; 3rd Ed.; John Wiley & Sons, New York, 1999. In general, protecting groups are removed under conditions which will not affect the remaining portion of the molecule. These methods are well known in the art and include acid hydrolysis, hydrogenolysis and the like.

The compounds of the invention may also exist in various polymorphous forms, for example as amorphous and crystalline polymorphous forms. All polymorphous forms of the compounds of the invention belong within the framework of the invention and are a further aspect of the invention.

The compound of the invention may also comprise deuterium replacing hydrogen. This replacement may in some circumstances lead to improved metabolic stability (Nature Reviews Drug Discovery 15, 219-221 (2016)).

It is understood that substituents and substitution patterns of the compounds described herein can be selected by one of ordinary skill in the art to provide compounds that are chemically stable and that can be readily synthesized by techniques known in the art and further by the methods set forth in this disclosure.

The present invention relates further to pharmaceutically acceptable salts of the compounds described herein. The term "pharmaceutically acceptable salt" refers to salts or esters of the compounds described herein prepared by conventional means that include basic salts of inorganic and organic acids, including but not limited to hydrochloric acid, hydrobromic acid, sulfuric acid, phosphoric acid, methanesulfonic acid, ethanesulfonic acid, malic acid, acetic acid, oxalic acid, tartaric acid, citric acid, lactic acid, fumaric acid, succinic acid, maleic acid, salicylic acid, benzoic acid, phenylacetic acid, mandelic acid and the like. Any chemical compound recited in this specification may alternatively be administered as a pharmaceutically acceptable salt thereof. Also included are acidic salts of inorganic and organic bases, including but not limited to sodium, potassium, ammonium, triethylamine and the like.

"Pharmaceutically acceptable salts" are also inclusive of the free acid, base, and zwitterionic forms. Descriptions of suitable pharmaceutically acceptable salts can be found in Handbook of Pharmaceutical Salts, Properties, Selection and Use, Wiley VCH (2002). For therapeutic use, salts of the compounds are those wherein the counter-ion is pharmaceutically acceptable. However, salts of acids and bases which are non-pharmaceutically acceptable may also find use, for example, in the preparation or purification of a pharmaceutically acceptable compound.

### Compositions and modes of treatment:

Another aspect of the disclosure includes pharmaceutical compositions prepared for administration to a subject and which include a therapeutically effective amount of one or more of the compounds disclosed herein. In certain embodiments, the pharmaceutical compositions are useful for treating pain.

The therapeutically effective amount of a disclosed compound will depend on the route of administration, the species of subject and the physical characteristics of the subject being treated. Specific factors that can be taken into account include disease severity and stage, weight, diet and concurrent medications. The relationship of these factors to determining a therapeutically effective amount of the disclosed compounds is understood by those of skill in the art.

Pharmaceutical compositions for administration to a subject can include at least one further pharmaceutically acceptable additive such as carriers, thickeners, diluents, buffers, preservatives, surface active agents and the like in addition to the molecule of choice. Pharmaceutical compositions can also include one or more additional active ingredients such as antimicrobial agents, anti-inflammatory agents, anesthetics, and the like. The pharmaceutically acceptable carriers useful for these formulations are conventional. Remington's Pharmaceutical Sciences, by E. W. Martin, Mack Publishing Co., Easton, PA, 19th Edition (1995), describes compositions and formulations suitable for pharmaceutical delivery of the compounds herein disclosed.

In general, the nature of the carrier will depend on the particular mode of administration being employed. For instance, parenteral formulations usually contain injectable fluids that include pharmaceutically and physiologically acceptable fluids such as water, physiological saline, balanced salt solutions, aqueous dextrose, glycerol or the like as a vehicle. For solid compositions (for example, powder, pill, tablet, or capsule forms), conventional non-toxic solid carriers can include, for example, pharmaceutical grades of mannitol, lactose, starch, or magnesium stearate. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

The compound according to the present invention as described herein may comprise different types of carriers depending on whether it is to be administered in solid, liquid or aerosol form, and whether it need to be sterile for such routes of administration as injection. The present invention can be administered by a variety of administration modes, including without limitation parenteral, intravenous, intradermal, intraarterial, intraperitoneal, intralesional, intracranial, intraarticular, intraprostatical, intrapleural, intratracheal, nasal, intravitreal, vaginal, rectal, topical, intratumoral, intramuscular, , subcutaneous, subconjunctival, intravesicularl, mucosal, intrapericardial, intraumbilical, ocular, oral, topical, local, pulmonal (e.g., aerosol inhalation), transdermal administration, administration by injection, infusion, continuous infusion, localized perfusion bathing target cells directly, via a catheter, via a lavage, in cremes, in lipid compositions (e.g., liposomes), or by other method or any combination of the forgoing as would be known to one of ordinary skill in the art (see, for example, Remington's Pharmaceutical Sciences, 18th Ed. Mack Printing Company, 1990, incorporated herein by reference).

The compositions of the disclosure can alternatively contain as pharmaceutically acceptable carrier substances that are required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, and triethanolamine oleate. For solid compositions, conventional nontoxic pharmaceutically acceptable vehicles can be used which include, for example, pharmaceutical grades of mannitol, lactose, starch, magnesium stearate, sodium saccharin, talcum, cellulose, glucose, sucrose, magnesium carbonate, and the like.

"Administration of" and "administering a" compound should be understood to mean providing a compound, a prodrug of a compound, or a pharmaceutical composition as described herein. The compound or composition can be administered by another person to the subject (e.g., intravenously) or it can be self-administered by the subject (e.g., tablets).

Any references herein to a compound for use as a medicament in the treatment of a medical condition also relate to a method of treating said medical condition comprising the administration of a compound, or composition comprising said compound, to a subject in need thereof, or to the use of a compound, composition comprising said compound, in the treatment of said medical condition.

Dosage can be varied by the attending clinician to maintain a desired concentration at a target site (for example, the lungs or systemic circulation). Higher or lower concentrations can be selected based on the mode of delivery, for example, trans-epidermal, rectal, oral, pulmonary, or intranasal delivery versus intravenous or subcutaneous delivery. Dosage can also be adjusted based on the release rate of the administered formulation, for example, of an intrapulmonary spray versus powder, sustained release oral versus injected particulate or transdermal delivery formulations, and so forth.

### Medical conditions and treatment

The present invention also relates to a method of treatment of subjects suffering from the medical conditions disclosed herein. The method of treatment comprises preferably the administration of a therapeutically effective amount of a compound disclosed herein to a subject in need thereof.

The term "subject" includes both human and veterinary subjects. The term "treatment" refers to a therapeutic intervention that ameliorates a sign or symptom of a disease or pathological condition after it has begun to develop.

In the context of the present invention, the term "medicament" refers to a drug, a pharmaceutical drug or a medicinal product used to diagnose, cure, treat, or prevent disease. It refers to any substance or combination of substances presented as having properties for treating or preventing disease. The term comprises any substance or combination of substances, which may be used in or administered either with a view to restoring, correcting or modifying physiological functions by exerting a pharmacological, immunological or metabolic action, or to making a medical diagnosis. The term medicament comprises biological drugs, small molecule drugs or other physical material that affects physiological processes.

According to the present invention, the term "treatment" refers to a therapeutic intervention that ameliorates a sign or symptom of a disease or pathological condition after it has begun to develop. As used herein, the term "ameliorating", with reference to a disease or pathological condition, refers to any observable beneficial effect of the treatment. The beneficial effect can be evidenced, for example, by a delayed onset of clinical symptoms of the disease in a susceptible subject, a reduction in severity of some or all clinical symptoms of the disease, a slower progression of the disease, an improvement in the overall health or well-being of the subject, or by other parameters well known in the art that are specific to the particular disease.

The present invention encompasses both treatment and prophylactic treatment of a subject. A "prophylactic" treatment is a treatment administered to a subject who does not exhibit signs of a disease or exhibits only early signs for the purpose of decreasing the risk of developing pathology.

The term "disease" refers to a particular abnormal condition, a disorder of a structure or function that affects part or all of an organism in the context of the present invention. It refers to any condition that causes pain, dysfunction, distress, or death to the person afflicted and includes injuries, disabilities, disorders, syndromes, infections, isolated symptoms, deviant behaviors, and atypical variations of structure and function. Diseases are associated with dysfunctioning of the body's normal homeostatic processes. Diseases can be acquired, congenital, chronic, acute, genetic, idiopathic, hereditary or inherited. Other equivalent terms in the context of the present invention are illness, disorder, medical condition, syndrome or predisease. A disease can be localized, disseminated or systemic.

When used in the context of the present invention, the term "genotoxic stress" refers to a stress signal, including any given substance, chemical compound, environmental signal, environmental material, irradiation, and/or cellular metabolite, including ROS, which induces damages to genetic material, including all kinds of nucleic acids such as DNA and RNA. The genome is exposed to potentially deleterious genotoxic events during every cell division cycle. This endogenous source of DNA damage results from cellular metabolism or routine errors in DNA replication and recombination. In addition, cellular and organismal exposure to exogenous genotoxic agents including ultraviolet light, oxidative stress, and chemical mutagens, leads to a variety of nucleotide modifications and DNA strand breaks. In order to combat these attacks on the genome, the cell has evolved a response system that induces cell cycle arrest to allow sufficient time to repair the incurred damage. Genotoxic stress induces DNA damage, which leads to the activation of DNA repair. The genotoxic stress response system comprises the DNA repair and activates the appropriate DNA repair pathway, or, in the case of irreparable damage, induces apoptosis. DNA damage in the form of mutations or genomic instability result from genotoxic stress caused by exposure to toxic agents, such as cytotoxic agents administered as anti-cancer drugs, ultraviolet sun light, background ionizing radiation, chemicals in food and the environment and highly reactive molecules produced within cells during metabolism. Similar types of DNA damage occur in response to various agents and include mutations, removal of bases and nucleotides, formation of dimers, strand breaks, cross-links, and chromosomal aberrations. Some of these types of damage accumulate in nuclear or mitochondrial DNA during aging (e.g., point mutations, single-strand breaks, DNA cross-links, additions/deletions, oxidative damage, and methylated bases).

NF-κB (nuclear factor kappa-light-chain-enhancer of activated B cells) is a protein complex that controls, without limitation, transcription of DNA, cytokine production and survival, differentiation and proliferation of cells. NF-κB is found in almost all animal cell types and is involved in cellular responses to stimuli such as stress, cytokines, free radicals, heavy metals, ultraviolet irradiation, oxidized LDL, and bacterial or viral antigens. NF-κB plays a key role in regulating the immune response to infection and plays various important roles in adaptive and innate immunity. Incorrect regulation of NF-κB has been linked to cancer, inflammatory and autoimmune diseases, septic shock, viral infection, and improper immune system development. NF-κB has also been implicated in processes of synaptic plasticity and memory. All proteins of the NF-κB family share a Rel homology domain in their N-terminus.

A subfamily of NF-κB proteins, including RelA, RelB, and c-Rel, have a transactivation domain in their C-termini. In contrast, the NF-κB1 and NF-κB2 proteins are synthesized as large precursors, p105, and p100, which undergo processing to generate the mature NF-κB subunits, p50 and p52, respectively. The processing of p105 and p100 is mediated by the ubiquitin/proteasome pathway and involves selective degradation of their C-terminal region containing ankyrin repeats. Whereas the generation of p52 from p100 is a tightly regulated process, p50 is produced from constitutive processing of p105. The p50 and p52 proteins have no intrinsic ability to activate transcription and thus have been proposed to act as transcriptional repressors when binding κB elements as homodimers. Indeed, this confounds the interpretation of p105-knockout studies, where the genetic manipulation is removing an IκB (full-length p105) and a likely repressor (p50 homodimers) in addition to a transcriptional activator (the RelA-p50 heterodimer).

NF-κB is important in regulating cellular responses because it belongs to the category of "rapidacting" primary transcription factors, i.e., transcription factors that are present in cells in an inactive state and do not require new protein synthesis in order to become activated. This allows NF-κB to be a fast responder to harmful cellular stimuli. Known inducers of NF-κB activity are highly variable and include reactive oxygen species (ROS), tumor necrosis factor alpha (TNFα), interleukin 1-beta (IL-1β), bacterial lipopolysaccharides (LPS), isoproterenol, cocaine, and ionizing radiation. Many bacterial products and stimulation of a wide variety of cell-surface receptors lead to NF-κB activation and fairly rapid changes in gene expression. The identification of Toll-like receptors (TLRs) as specific pattern recognition molecules and the finding that stimulation of TLRs leads to activation of NF-κB improved our understanding of how different pathogens activate NF-κB. For example, studies have identified TLR4 as the receptor for the LPS component of Gram-negative bacteria. TLRs are key regulators of both innate and adaptive immune responses.

In unstimulated cells, the NF-κB dimers are sequestered in the cytoplasm by a family of inhibitors, called IκBs (Inhibitor of κB), which are proteins that contain multiple copies of a sequence called ankyrin repeats. By virtue of their ankyrin repeat domains, the IκB proteins mask the nuclear localization signals (NLS) of NF-κB proteins and keep them sequestered in an inactive state in the cytoplasm. IκBs are a family of related proteins that have an N-terminal regulatory domain, followed by six or more ankyrin repeats and a PEST domain near their C terminus. Although the IκB family consists of IκBα, IκBβ, IκBε, and Bcl-3, the best-studied and major IκB protein is IκBα. Due to the presence of ankyrin repeats in their C-terminal halves, p105 and p100 also function as IκB proteins. The c-terminal half of p100, that is often referred to as IκBδ, also functions as an inhibitor. IκBδ degradation in response to developmental stimuli, such as those transduced through LTβR, potentiate NF-κB dimer activation in a NIK dependent non-canonical pathway.

Activation of the NF-κB is initiated by the signal-induced degradation of IκB proteins. This occurs primarily via activation of a kinase called "IKK" or IκB kinase. Therefore, the term "IKK/NF-κB activation" as used in the present patent application refers to the activation of NF-κB through activation of IKK.

IKK is composed of a heterodimer of the catalytic IKKα and IKKβ subunits and a "master" regulatory protein termed NEMO (NF-κB essential modulator) or IKKγ. When activated by signals, the IκB kinase phosphorylates two serine residues located in an IκB regulatory domain. Upon phosphorylation of these serines (e.g., serines 32 and 36 in human IκBα), the IκB inhibitor molecules are modified by a process called ubiquitination leading to degradation by the proteasome. With the degradation of IκB, the NF-κB complex is then freed to enter the nucleus where it can 'turn on' the expression of specific genes that have DNA-binding sites for NF-κB nearby. The activation of these genes by NF-κB then leads to the given physiological response, for example, an inflammatory or immune response, a cell survival response, or cellular proliferation. NF-κB turns on expression of its own repressor, IκBα. The newly synthesized IκBα then re-inhibits NF-κB and, thus, forms an auto feedback loop, which results in oscillation, dampening and downregulation of NF-κB activity levels.

According to the present invention, genotoxic stress-induced IKK/NF-κB activation relates to the signaling pathway that is induced through the occurrence of genotoxic stress, which leads to the activation of IKK and consequently to the activation of NF-κB. Genotoxic stress triggers two corresponding signaling axes to activate the IκB kinase (IKK) complex analogously to the canonical NF-κB signaling cascades. The first axis is initiated by the DNA strand break sensor poly(ADP-ribose)-polymerase-1 (PARP-1), which sets up a transient nucleoplasmic complex and triggers PIASy mediated SUMOylation and ataxia telangiectasia mutated (ATM) mediated phosphorylation of nuclear !KKγ. Modified !KKγ shuttles back into the cytoplasm and assembles into newly formed IKK complexes. At the same ATM translocates into the cytoplasm, binds to TRAF6 and triggers its K63-linked polyubiquitination. Activated TRAF6 recruits clAP1 and TAB2-TAK1, resulting in TAK1 activation and IKKβ phosphorylation. However, final activation of the IKK complex requires clAP1-dependent !KKγ mono-ubiquitination of !KKγ at lysine 285, which is dependent on the formation of the nuclear PARP1 signalosome and the activation of the cytosolic signaling axis by the ATM-dependent activation of TRAF6.

cdc-like kinase 2 (CLK2) and cdc-like kinase 4 (CLK4) are dual specificity kinases acting on both serine/threonine and tyrosine-containing substrates. CLK2 and CLK4 phosphorylate serine/arginine rich (SR) proteins of the spliceosomal complex that modulate RNA splicing (Iwai et al., 2018; Salvador and Gomis, 2018; Yoshida et al., 2015). CLK2 and CLK4 are further involved in the regulation of several cellular processes and may serve as a link between cell cycle progression, apoptosis, and telomere length regulation. While the exact molecular role of CLK2 and CLK4 yet remain to be determined, the clinical implications of deregulated CLK2 in a variety of cancers have been illuminated by numerous recent studies. CLK2 has an oncogenic role in many cancers, such as colorectal cancer, non-small cell lung cancer, glioblastoma, and breast cancer and elevated CLK2 expression is associated with their occurrence, progression, and poor prognosis (Lin et al., 2022; Liu et al., 2021; Park et al., 2016). In addition, pharmacological abrogation of CLK2 has been shown to inhibit tumor xenografts in vivo in breast cancer models (Iwai et al., 2018; Riggs et al., 2017; Salvador and Gomis, 2018). CLK4 is comparatively less well understood, but recent studies have illustrated a role in breast cancer as well as downstream targets which are not splicing factors (Němec et al., 2019; Martin-Moyano et al., 2020).

Diseases associated with genotoxic stress-induced IKK/NF-κB activation comprise, without limitation, cancer, either during development of the disease, in the established disease or as a consequence of chemotherapy or radiation therapy of the disease, particularly colon cancer, gastric cancer, breast cancer, melanoma, myelodysplastic syndrome, acute myeloid leukemia (AML), tumors with increased PARP-1 expression, including Ewing's sarcoma, malignant lymphomas, the early stage of colorectal carcinogenesis, hepatocellular carcinoma, nonatypical and atypical endometrial hyperplasia, breast, uterine, lung, and ovarian cancers. Non-cancer diseases and conditions associated with genotoxic stress-induced IKK/NF-κB activation comprise, without limitation, diabetes type 1, diabetes type 2, stroke, subarachnoid hemorrhage (SAH), reperfusion damage, in particular of the kidney and heart, atherosclerosis, progeriod syndrome and aging.

A person skilled in the art can identify a subject suffering from cancer exhibiting genotoxic stress-induced IKK/NF-κB activation by employing standard means of analysis. There are multiple assays to identify genotoxic stress-induced NF-κB activation in tumor samples from a cancer patient in order to identify subjects of the present invention intended for treatment, some of which are indicated below. The following methods represent examples and are not be understood as an exhaustive list of assays for identifying subjects suffering from cancer exhibiting genotoxic stress-induced IKK/NF-κB activation:
The following five protein-modifications indicate that IKK/NF-κB activation was induced by genotoxic stress, such as DNA double-strand breaks (DSBs), which can be generated for example by chemotherapeutical drugs or irradiation: Phospho-Ser 139 γH2A.X, Phospho-Ser 1981-ATM, Phospho-Ser 85 !KKγ, Mono-ubiquitination at Lys 285 of !KKγ and Phospho-Ser536-RelA (references for the modifications are found in Hinz et al., (2010) Mol Cell). The indicated protein modifications represent an exemplary, non-exhaustive and non-limiting list. These modifications can be assayed by established methods using commercially available antibodies, e.g. using Western blot analyses or other antibody-based techniques. Further methods to detect these modifications are mass-spectrometry techniques.
1.) Phospho-Ser 139 γH2A.X: Phosphorylation of H2A.X at residue Ser-139 by the PI3K-like kinases ATM, ATR and DNA-PK, is an early readout for the cellular response to the generation of DSBs by chemotherapeutical drugs or irradiation.
2.) Phospho-Ser 1981-ATM: This modification indicates activation of ATM by DSBs generated by chemotherapeutical drugs or irradiation.
3.) Phospho-Ser 85 !KKγ: To the current knowledge, this modification is only detected in cells with DSBs and the presence of DSB-activated ATM. It promotes NF-κB activation by DSBs.
4.) Mono-ubiquitination at Lys 285 of !KKγ: The ubiquitination at this residue is higher in genotoxic stress (i.e. DSB) -induced NF-κB compared to cytokine-induced NF-κB.
5.) Phospho-Ser536-RelA: This modification indicates NF-κB activation through diverse activating pathways, not limited to the genotoxic stress-induced pathway.

Patients that are suitable for the application of the compounds of the present invention and its use in the treatment of a cancer exhibiting genotoxic stress-induced IKK/NF-κB activation comprise subjects suffering from any cancer type which is being or has been treated with DNA-damage-inducing chemotherapy or irradiation.

The use of the compounds of the invention can in some embodiments be employed as "add-on" drugs in genotoxic therapies (chemotherapies, irradiation) to enhance cancer/tumor cell killing by suppression of NF-κB-dependent protection against apoptosis. Thus, there would be a large spectrum of malignancies where treatment success may be improved.

It is anticipated that the PARP1-PIASy-ATM- !KKγ complex and ATM-TRAF6 axis will be activated by chemotherapy and/or irradiation in a number of different cancer types. The assays described above may be used to affirm activation of the genotoxic stress induced NF-κB pathway by the respective standard chemotherapy or irradiation protocol in a given disease. The assays can also be used for therapy resistant cancers to decide to apply compounds of the present invention. The assays can also be applied prior to any treatment with cancers expected to have high level unrepaired DNA damage (e.g., when mutations in DNA repair genes have been documented).

Cancer according to the present invention refers to all types of cancer or neoplasm or malignant tumors found in mammals, including leukemias, lymphomas, sarcomas, melanomas and carcinomas. Examples of cancers are cancer of the breast, pancreas, colon, lung, non-small cell lung, ovary, and prostate.

In the context of the present invention, leukemias include, but are not limited to acute nonlymphocytic leukemia, chronic lymphocytic leukemia, acute granulocytic leukemia, chronic granulocytic leukemia, acute promyelocytic leukemia, adult T-cell leukemia, aleukemic leukemia, a leukocythemic leukemia, basophylic leukemia, blast cell leukemia, bovine leukemia, chronic myelocytic leukemia, leukemia cutis, embryonal leukemia, eosinophilic leukemia, Gross' leukemia, hairy-cell leukemia, hemoblastic leukemia, hemocytoblastic leukemia, histiocytic leukemia, stem cell leukemia, acute monocytic leukemia, leukopenic leukemia, lymphatic leukemia, lymphoblastic leukemia, lymphocytic leukemia, lymphogenous leukemia, lymphoid leukemia, lymphosarcoma cell leukemia, mast cell leukemia, megakaryocytic leukemia, micromyeloblastic leukemia, monocytic leukemia, myeloblastic leukemia, myelocytic leukemia, myeloid granulocytic leukemia, myelomonocytic leukemia, Naegeli leukemia, plasma cell leukemia, plasmacytic leukemia, promyelocytic leukemia, Rieder cell leukemia, Schilling's leukemia, stem cell leukemia, subleukemic leukemia, and undifferentiated cell leukemia.

According to the present invention, lymphomas include Hodgkin and non-Hodgkin lymphoma (B-cell and T-cell lymphoma) including, but not limited to Diffuse large B-cell lymphoma (DLBCL), primary mediastinal B-cell lymphoma, Follicular lymphoma, Chronic lymphocytic leukemia, small lymphocytic lymphoma, Mantle cell lymphoma, Marginal zone B-cell lymphomas, Extranodal marginal zone B-cell lymphomas, also known as mucosa-associated lymphoid tissue (MALT) lymphomas, Nodal marginal zone B-cell lymphoma and Splenic marginal zone B-cell lymphoma, Burkitt lymphoma, Lymphoplasmacytic lymphoma (Waldenstrom macroglobulinemia), Hairy cell leukemia Primary central nervous system (CNS) lymphoma, Precursor T-lymphoblastic lymphoma/leukemia, Peripheral T-cell lymphomas, Cutaneous T-cell lymphomas (mycosis fungoides, Sezary syndrome, and others), Adult T-cell leukemia/lymphoma including the smoldering, the chronic, the acute and the lymphoma subtype, Angioimmunoblastic T-cell lymphoma, Extranodal natural killer/T-cell lymphoma, nasal type, Enteropathy-associated intestinal T-cell lymphoma (EATL), Anaplastic large cell lymphoma (ALCL), and unspecified Peripheral T-cell lymphoma.

Sarcomas as defined in the context of the present invention include, but are not limited to a chondrosarcoma, fibrosarcoma, lymphosarcoma, melanosarcoma, myxosarcoma, osteosarcoma, Abernethy's sarcoma, adipose sarcoma, liposarcoma, alveolar soft part sarcoma, ameloblastic sarcoma, botryoid sarcoma, chloroma sarcoma, chorio carcinoma, embryonal sarcoma, Wilms' tumor sarcoma, endometrial sarcoma, stromal sarcoma, Ewing's sarcoma, fascial sarcoma, fibroblastic sarcoma, giant cell sarcoma, granulocytic sarcoma, Hodgkin's sarcoma, idiopathic multiple pigmented hemorrhagic sarcoma, immunoblastic sarcoma of B cells, lymphoma, immunoblastic sarcoma of T-cells, Jensen's sarcoma, Kaposi's sarcoma, Kupffer cell sarcoma, angiosarcoma, leukosarcoma, malignant mesenchymoma sarcoma, parosteal sarcoma, reticulocytic sarcoma, Rous sarcoma, serocystic sarcoma, synovial sarcoma, and telangiectaltic sarcoma.

Melanomas according to the present invention include, but are not limited to include, for example, acral-lentiginous melanoma, amelanotic melanoma, benign juvenile melanoma, Cloudman's melanoma, S91 melanoma, Harding-Passey melanoma, juvenile melanoma, lentigo maligna melanoma, malignant melanoma, nodular melanoma, subungal melanoma, and superficial spreading melanoma.

Carcinomas as defined by the present inventin include, but are not limited to acinar carcinoma, acinous carcinoma, adenocystic carcinoma, adenoid cystic carcinoma, carcinoma adenomatosum, carcinoma of adrenal cortex, alveolar carcinoma, alveolar cell carcinoma, basal cell carcinoma, carcinoma basocellulare, basaloid carcinoma, basosquamous cell carcinoma, bronchioalveolar carcinoma, bronchiolar carcinoma, bronchogenic carcinoma, cerebriform carcinoma, cholangiocellular carcinoma, chorionic carcinoma, colloid carcinoma, comedo carcinoma, corpus carcinoma, cribriform carcinoma, carcinoma en cuirasse, carcinoma cutaneum, cylindrical carcinoma, cylindrical cell carcinoma, duct carcinoma, carcinoma durum, embryonal carcinoma, encephaloid carcinoma, epiermoid carcinoma, carcinoma epitheliale adenoides, exophytic carcinoma, carcinoma exulcere, carcinoma fibrosum, gelatiniform carcinoma, gelatinous carcinoma, giant cell carcinoma, carcinoma gigantocellulare, glandular carcinoma, granulosa cell carcinoma, hair-matrix carcinoma, hematoid carcinoma, hepatocellular carcinoma, Hurthle cell carcinoma, hyaline carcinoma, hypernephroid carcinoma, infantile embryonal carcinoma, carcinoma in situ, intraepidermal carcinoma, intraepithelial carcinoma, Krompecher's carcinoma, Kulchitzky-cell carcinoma, large-cell carcinoma, lenticular carcinoma, carcinoma lenticulare, lipomatous carcinoma, lymphoepithelial carcinoma, carcinoma medullare, medullary carcinoma, melanotic carcinoma, carcinoma molle, mucinous carcinoma, carcinoma muciparum, carcinoma mucocellulare, mucoepidermoid carcinoma, carcinoma mucosum, mucous carcinoma, carcinoma myxomatodes, nasopharyngeal carcinoma, oat cell carcinoma, carcinoma ossificans, osteoid carcinoma, papillary carcinoma, periportal carcinoma, preinvasive carcinoma, prickle cell carcinoma, pultaceous carcinoma, renal cell carcinoma of kidney, reserve cell carcinoma, carcinoma sarcomatodes, schneiderian carcinoma, scirrhous carcinoma, carcinoma scroti, signet-ring cell carcinoma, carcinoma simplex, small-cell carcinoma, solanoid carcinoma, spheroidal cell carcinoma, spindle cell carcinoma, carcinoma spongiosum, squamous carcinoma, squamous cell carcinoma, string carcinoma, carcinoma telangiectaticurn, carcinoma telangiectodes, transitional cell carcinoma, carcinoma tuberosum, tuberous carcinoma, verrucous carcinoma, and carcinoma villosum.

Additional cancers according to the present invention include, but are not limited to multiple myeloma, neuroblastoma, breast cancer, ovarian cancer, lung cancer, rhabdomyosarcoma, primary thrombocytosis, primary macroglobulinemia, small-cell lung tumors, primary brain tumors, stomach cancer, colon cancer, malignant pancreatic insulanoma, malignant carcinoid, urinary bladder cancer, premalignant skin lesions, testicular cancer, lymphomas, thyroid cancer, esophageal cancer, genitourinary tract cancer, malignant hypercalcemia, cervical cancer, endometrial cancer, adrenal cortical cancer, and prostate cancer.

In the context of the present invention, the term "DNA damage" refers to alteration in the chemical structure of DNA, such as a break in a strand of DNA, a base missing from the backbone of DNA, or a chemically changed base. Damage to DNA that occurs naturally can result from metabolic or hydrolytic processes. Metabolism releases compounds that damage DNA including reactive oxygen species, reactive nitrogen species, reactive carbonyl species, lipid peroxidation products and alkylating agents, among others, while hydrolysis cleaves chemical bonds in DNA. While most DNA damages can undergo DNA repair, such repair is not 100% efficient. Un-repaired DNA damages accumulate in non-replicating cells, such as cells in the brains or muscles of adult mammals and can cause aging. In replicating cells, such as cells lining the colon, errors occur upon replication of past damages in the template strand of DNA or during repair of DNA damages. These errors can give rise to mutations or epigenetic alterations. Both of these types of alteration can be replicated and passed on to subsequent cell generations. These alterations can change gene function or regulation of gene expression and possibly contribute to progression to cancer. Failure to repair DNA lesions may result in blockages of transcription and replication, mutagenesis, and/or cellular cytotoxicity. In humans, DNA damage has been shown to be involved in a variety of genetically inherited disorders, in aging, and in carcinogenesis.

All eukaryotic cells have evolved a multifaceted response to counteract the potentially deleterious effects of DNA damage. Upon sensing DNA damage or stalls in replication, cell cycle checkpoints are activated to arrest cell cycle progression to allow time for repair before the damage is passed on to daughter cells. In addition to checkpoint activation, the DNA damage response leads to induction of transcriptional programs, enhancement of DNA repair pathways, and when the level of damage is severe, to initiation of apoptosis. All of these processes are carefully coordinated so that the genetic material is faithfully maintained, duplicated, and segregated within the cell.

The term "DNA repair" refers to a number of cellular processes or pathways to restore lost information after DNA damage, when used in the context of the present invention. These processes and pathways comprise, without limitation, cell cycle check points such as the G1 checkpoint, S-phase checkpoint, G2-M checkpoint, and DNA repair pathways such as direct reversal, base excision repair, nucleotide excision repair, DNA mismatch repair and double strand break repair. The rate of DNA repair is dependent on many factors, including the cell type, the age of the cell, and the extracellular environment.

A cell that has accumulated a large amount of DNA damage, or one that no longer effectively repairs damage incurred to its DNA, can undergo different cellular processes including an irreversible state of dormancy, known as senescence, apoptosis, which is a programmed cell death program, other cell death programs, such as necrosis, non-apoptotic programmed celldeath or necroptosis, unregulated cell division, which can lead to the formation of a tumor that is cancerous.

In the sense of the present invention, the term "DNA repair gene" refers to all genes, which are involved in the control or modulation of DNA repair mechanisms and pathways. These include, without limitation, for base excision repair (BER), UNG, SMUG1, MBD4, TDG, OGG1, MUTYH (MYH), NTHL1 (NTH1), MPG, NEIL1, NEIL2, NEIL3, APEX1 (APE1), APEX2, LIG3, XRCC1, PNKP, APLF (C2ORF13); for Poly(ADP-ribose) polymerase (PARP) enzymes that bind to DNA, PARP1 (ADPRT), PARP2 (ADPRTL2), PARP3 (ADPRTL3); for activation of PARP1, TSG101; for direct reversal of damage MGMT, ALKBH2 (ABH2), ALKBH3 (DEPC1); for repair of DNA-topoisomerase crosslinks TDP1, TDP2 (TTRAP); for mismatch excision repair (MMR) MSH2, MSH3, MSH6, MLH1, PMS2, MSH4, MSH5, MLH3, PMS1, PMS2L3; for nucleotide excision repair (NER) XPC, RAD23B, CETN2, RAD23A, XPA, DDB1, DDB2 (XPE), RPA1, RPA2, RPA3, TFIIH, ERCC3 (XPB), ERCC2 (XPD), GTF2H1, GTF2H2, GTF2H3, GTF2H4, GTF2H5 (TTDA), CDK7, CCNH, MNAT1, ERCC5 (XPG), ERCC1, ERCC4 (XPF), LIG1; NER-related ERCC8 (CSA), ERCC6 (CSB), UVSSA (KIAA1530), XAB2 (HCNP), MMS19; for homologous recombination RAD51, RAD51B, RAD51D, DMC1, XRCC2, XRCC3, RAD52, RAD54L, RAD54B, BRCA1, SHFM1 (DSS1), RAD50, MRE11A, NBN (NBS1), RBBP8 (CtIP), MUS81, EME1 (MMS4L), EME2, GIYD1 (SLX1A), GIYD2 (SLX1B), GEN1; for fanconi anemia FANCA, FANCB, FANCC, BRCA2 (FANCD1), FANCD2, FANCE, FANCF, FANCG (XRCC9), FANCI (KIAA1794), BRIP1 (FANCJ), FANCL, FANCM, PALB2 (FANCN), RAD51C (FANCO), BTBD12 (SLX4) (FANCP), FAAP20 (C1orf86), FAAP24 (C19orf40); for non-homologous end-joining XRCC6 (Ku70), XRCC5 (Ku80), PRKDC, LIG4, XRCC4, DCLRE1C (Artemis), NHEJ1 (XLF, Cernunnos); for modulation of nucleotide pools NUDT1 (MTH1), DUT, RRM2B (p53R2); for DNA polymerases (catalytic subunits) POLB, POLG, POLD1, POLE, PCNA, REV3L (POLZ), MAD2L2 (REV7), REV1L (REV1), POLH, POLI (RAD30B), POLO, POLK (DINB1), POLL, POLM, POLN (POL4P); for editing and processing nucleases FEN1 (DNase IV), FAN1 (MTMR15), TREX1 (DNase III), TREX2, EXO1 (HEX1), APTX (aprataxin), SPO11, ENDOV; for Ubiquitination and modification UBE2A (RAD6A), UBE2B (RAD6B), RAD18, SHPRH, HLTF (SMARCA3), RNF168, SPRTN (c1orf124), RNF8, RNF4, UBE2V2 (MMS2), UBE2N (UBC13); for Chromatin Structure and Modification H2AFX (H2AX), CHAF1A (CAF1), SETMAR (METNASE); for genes defective in diseases associated with sensitivity to DNA damaging agents BLM, WRN, RECQL4, ATM, TTDN1 (C7orf11); for other identified genes with known or suspected DNA repair function DCLRE1A (SNM1), DCLRE1B (SNM1B), RPA4, PRPF19 (PSO4), RECQL (RECQ1), RECQL5, HELQ (HEL308), RDM1 (RAD52B), OBFC2B (SSB1); other conserved DNA damage response genes ATR, ATRIP, MDC1, RAD1, RAD9A, HUS1, RAD17 (RAD24), CHEK1, CHEK2, TP53, TP53BP1 (53BP1), RIF1, TOPBP1, CLK2, PER1.

In one embodiment of the present invention the compound is more effective in inhibiting NF-κB-signaling induced by preferably genotoxic stress compared to inhibiting NF-κB-signaling induced by TNF-alpha (TNFα) and/or IL-1β.

According to the present invention, TNFα or tumor necrosis factor alpha is a cell signaling protein (cytokine) involved in systemic inflammation and is one of the cytokines that make up the acute phase reaction. TNFα regulates immune cells, is able to induce fever, apoptotic cell death, cachexia, inflammation and to inhibit tumorigenesis and viral replication and respond to sepsis via IL1 & IL6 producing cells. Dysregulation of TNFα-production has been implicated in a variety of human diseases including Alzheimer's disease, cancer, major depression, Psoriasis and inflammatory bowel disease (IBD). TNFα can bind two receptors, TNFR1 (TNF receptor type 1; CD120a; p55/60) and TNFR2 (TNF receptor type 2; CD120b; p75/80). TNFR signaling induces activation of several intracellular signaling pathways, including activation of NF-κB.

In the sense of the present invention, IL-1β is also known as "leukocytic pyrogen", "leukocytic endogenous mediator", "mononuclear cell factor", "lymphocyte activating factor" among other names and is a cytokine protein that in humans is encoded by the IL1B gene. IL-1β is a member of the interleukin 1 family of cytokines. This cytokine is produced by activated macrophages as a proprotein, which is proteolytically processed to its active form by caspase 1 (CASP1/ICE). This cytokine is an important mediator of the inflammatory response, and is involved in a variety of cellular activities, including cell proliferation, differentiation, and apoptosis.

In the context of the present invention, the term "NF-κB signaling induced by TNFα and/or IL-1β" refers to the activation of the classical or canonical NF-κB signaling pathway, which gets activated upon stimulation with TNFα and/or IL-1β In the canonical signaling pathway, NF-κB/Rel proteins are bound and inhibited by IκB proteins. Proinflammatory cytokines such as TNFα and IL-1β, LPS, growth factors, and antigen receptors induce signaling cascades that lead to IKK complex activation (IKKβ, IKKα, and NEMO), which phosphorylates IκB proteins. Phosphorylation of IκB leads to its ubiquitination and proteasomal degradation, freeing NF-κB/Rel complexes. Active NF-κB/Rel complexes are further activated by post-translational modifications (phosphorylation, acetylation, glycosylation, ubiquitination) and translocate to the nucleus where, either alone or in combination with other transcription factors including AP-1, Ets, and Stat, they induce target gene expression.

The present invention can also relate to the treatment of a cancer, which is associated with genomic instability due to defective DNA-repair mechanisms.

The term "genomic instability", as used in the context of the present invention, refers to a high frequency of mutations within the genome of a cellular lineage. Such mutations can include changes in nucleic acid sequences, chromosomal rearrangements or aneuploidy. Genome instability does occur in bacteria. In multicellular organisms, genome instability is central to carcinogenesis and occurs in many types of cancer. The skilled person can easily identify cancers that are associated with genomic instability by routine testing. Other diseases than cancer associated with genomic instability comprise neuronal diseases, including neurodegenerative diseases such as amyotrophic lateral sclerosis and the neuromuscular disease myotonic dystrophy.

In cancer, genome instability can occur prior to or as a consequence of transformation. Genome instability can refer to, without limitation, the accumulation of extra copies of DNA or chromosomes, chromosomal translocations, chromosomal inversions, chromosome deletions, single-strand breaks in DNA, double-strand breaks in DNA, the intercalation of foreign substances into the DNA double helix, or any abnormal changes in DNA tertiary structure that can cause either the loss of DNA, or the misexpression of genes. The unpredictable nature of these events is also a main contributor to the heterogeneity observed among tumour cells.

Cancers that are associated with genomic instability in the sense of the present invention include, but are not limited to BRCA1 or BRCA2 mutant ovarial carcinoma, breast carcinoma, cervical carcinoma, gastric carcinoma, pancreatic carcinoma or prostate carcinoma.

In the context of the present invention, the term "defective" refers to that something, for example a cellular system such as the DNA repair system or the DNA damage response system, which has a problem or fault that prevents it from working correctly.

In the context of the present invention the term "alteration" refers to any kind of change, modification or adjustment that is made so that the original state of something is changed or altered, when used in the context of the present invention. Genetic alterations therefore refer to changes that are occurring on genetic material, including changes that occur with respect to the nucleotide sequence of a nucleic acid molecule. Epigenetic alterations refer to changes of the epigenetic state of a nucleic acid molecule, for example a DNA molecule, which do not change the nucleotide sequence of the molecule. Epigenetic modifications can occur on the nucleic acid or on the chromatin, which includes histones and histone modifications. Epigenetic modifications or alterations include, without limitation, acetylation, methylation, ubiquitination, phosphorylation, sumoylation, ribosylation and citrullination.

The term "resistance" in the sense of the present invention refers to the reduction in effectiveness of a drug such as an antimicrobial, anthelmintic or an antineoplastic in treating a disease or condition. The term is used in the context of, for example, pathogens or cancer cells, which have "acquired" resistance to a drug or to another treatment or mechanism that is directed against the pathogen or the cancer cell. Antimicrobial resistance and antineoplastic resistance challenge when an organism or cancer cell is resistant to more than one drug, it is said to be multidrugresistant.

According to the present invention, cancer therapeutic resistance refers to the development of resistance to treatments such as chemotherapy, radiotherapy, irradiation therapy, cell therapy and targeted therapies by cancer cells through different mechanisms. These mechanisms include specific genetic and epigenetic changes in the cancer cell and/or the microenvironment in which the cancer cell resides. Also, activation of different signaling pathways, including the NF-κB pathway, can contribute to the development of cancer therapeutic resistance. The term "NF-κB-mediated resistance to apoptosis" refers to cellular mechanisms, by which the genotoxic stressactivated NF-κB pathway inhibits the induction of apoptosis, when used in the context of the present invention. NF-κB activation in response to DNA damaging cancer therapy is a principal mechanism of inducible tumour cell resistance.

Cancers that are associated with NF-κB-mediated resistance to therapy-induced tumor cell apoptosis in the sense of the present invention include, but are not limited to BRCA1 or BRCA2 mutant ovarial carcinoma, breast carcinoma, cervical carcinoma, gastric carcinoma, pancreatic carcinoma or prostate carcinoma.

In the context of the present invention, the term "cancer therapy" refers to any kind of treatment of cancer, including, without limitation, surgery, chemotherapy, radiotherapy, irradiation therapy, hormonal therapy, targeted therapy, cellular therapy, cancer immunotherapy, monoclonal antibody therapy.

Administration of the compound can be individual as mono-therapy or in combination with one or more other cancer therapies. In the context of the present invention the term "in combination" indicates that an individual that receives the compound according to the present invention also receives other cancer therapies, which does not necessarily happen simultaneously, combined in a single pharmacological composition or via the same route of administration. "In combination" therefore refers the treatment of an individual suffering from cancer with more than one cancer therapy. Combined administration encompasses simultaneous treatment, co-treatment or joint treatment, whereby treatment may occur within minutes of each other, in the same hour, on the same day, in the same week or in the same month as one another.

DNA damage-inducing cancer therapies in the sense of the present invention include, but are not limited to irradiation therapy and chemotherapy and work by overwhelming the capacity of the cell to repair DNA damage, resulting in cell death.

In this context, chemotherapy refers to a category of cancer treatment that uses one or more anti-cancer drugs (chemotherapeutic agents) as part of a standardized chemotherapy regimen. Chemotherapy may be given with a curative intent (which almost always involves combinations of drugs), or it may aim to prolong life or to reduce symptoms (palliative chemotherapy). Chemotherapy is one of the major categories of medical oncology (the medical discipline specifically devoted to pharmacotherapy for cancer). Chemotherapeutic agents are used to treat cancer and are administered in regimens of one or more cycles, combining two or more agents over a period of days to weeks. Such agents are toxic to cells with high proliferative rates - e.g., to the cancer itself, but also to the GI tract (causing nausea and vomiting), bone marrow (causing various cytopenias) and hair (resulting in baldness).

Chemotherapeutic agents comprise, without limitation, Actinomycin, All-trans retinoic acid, Azacitidine, Azathioprine, Bleomycin, Bortezomib, Carboplatin, Capecitabine, Cisplatin, Chlorambucil, Cyclophosphamide, Cytarabine, Daunorubicin, Docetaxel, Doxifluridine, Doxorubicin, Epirubicin, Epothilone, Etoposide, Fluorouracil, Gemcitabine, Hydroxyurea, Idarubicin, Imatinib, Irinotecan, Mechlorethamine, Mercaptopurine, Methotrexate, Mitoxantrone, Oxaliplatin, Paclitaxel, Pemetrexed, Teniposide, Tioguanine, Topotecan, Valrubicin, Vinblastine, Vincristine, Vindesine, Vinorelbine.

Irradiation or radiation therapy or radiotherapy in the context of the present invention relates to a therapeutic approach using ionizing or ultraviolet-visible (UV/Vis) radiation, generally as part of cancer treatment to control or kill malignant cells such as cancer cells or tumor cells. Radiation therapy may be curative in a number of types of cancer, if they are localized to one area of the body. It may also be used as part of adjuvant therapy, to prevent tumor recurrence after surgery to remove a primary malignant tumor (for example, early stages of breast cancer). Radiation therapy is synergistic with chemotherapy, and can been used before, during, and after chemotherapy in susceptible cancers. Radiation therapy is commonly applied to the cancerous tumor because of its ability to control cell growth. Ionizing radiation works by damaging the DNA of cancerous tissue leading to cellular death. Radiation therapy can be used systemically or locally.

Radiation therapy works by damaging the DNA of cancerous cells. This DNA damage is caused by one of two types of energy, photon or charged particle. This damage is either direct or indirect ionization of the atoms which make up the DNA chain. Indirect ionization happens as a result of the ionization of water, leading to the formation of free radicals, including hydroxyl radicals, which then damage the DNA. In photon therapy, most of the radiation effect is mediated through free radicals. Cells have mechanisms for repairing single-strand DNA damage and double-stranded DNA damage. However, double-stranded DNA breaks are much more difficult to repair, and can lead to dramatic chromosomal abnormalities and genetic deletions. Targeting double-stranded breaks increases the probability that cells will undergo cell death.

The amount of radiation used in photon radiation therapy is measured in gray (Gy), and varies depending on the type and stage of cancer being treated. For curative cases, the typical dose for a solid epithelial tumor ranges from 60 to 80 Gy, while lymphomas are treated with 20 to 40 Gy. Preventive (adjuvant) doses are typically around 45-60 Gy in 1.8-2 Gy fractions (for breast, head, and neck cancers.)

Different types of radiation therapy are known such as external beam radiation therapy, including conventional external beam radiation therapy, stereotactic radiation (radiosurgery), virtual simulation, 3-dimensional conformal radiation therapy, and intensity-modulated radiation therapy, intensity-modulated radiation therapy (IMRT), volumetric modulated arc therapy (VMAT), Particle therapy, auger therapy, brachytherapy, intraoperative radiotherapy, radioisotope therapy and deep inspiration breath-hold.

External beam radiation therapy comprises X-ray, gamma-ray and charged particles and can be applied as a low-dose rate or high dose rate depending on the overall therapeutic approach.

In internal radiation therapy radioactive substance can be bound to one or more monoclonal antibodies. For example, radioactive iodine can be used for thyroid malignancies. Brachytherapy of High dose regime (HDR) or low dose regime (LDR) can be combined with IR in prostate cancer.

According to the present invention, DNA damage-inducing chemotherapies comprise the administration of chemotherapeutics agents including, but not limited to anthracyclines such as Daunorubicin, Doxorubicin, Epirubicin, Idarubicin, Valrubicin, Mitoxantrone; Inhibitors of topoisomerase I such as Irinotecan (CPT-11) and Topotecan; Inhibitors of topoisomerase **II** including Etoposide, Teniposide and Tafluposide; Platinum-based agents such as Carboplatin, Cisplatin and Oxaliplatin; and other chemotherapies such as Bleomycin.

The instant disclosure also includes kits, packages and multi-container units containing the herein described pharmaceutical compositions, active ingredients, and/or means for administering the same for use in the prevention and treatment of diseases and other conditions in a mammalian subject.

With the above context, the following consecutively numbered embodiments provide further specific aspects of the invention:
1. Compound according to **Formula I** for use in the treatment of a cancer exhibiting genotoxic stress-induced IKK/NF-κB activation, 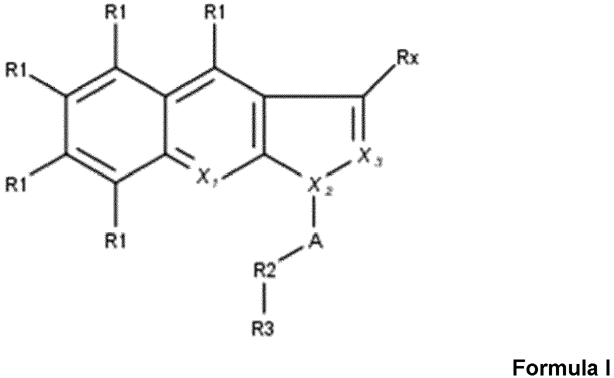 wherein
   **R1** = can be the same or different, H, OH, halogen (preferably Br, Cl or F), alkyl (preferably C1 to C6 alkyl), alkenyl, alkynyl, alkoxy (preferably OCH₃, OCH₂CH₃), alkyl carbonyl, carboxyl, alkoxy carbonyl, amine (preferably primary, secondary or tertiary amine),
   **Rx** = amine (preferably primary, secondary or tertiary amine), OH, H, alkyl (preferably C1 to C6 alkyl),
   **X1, X2, X3** = can be the same or different, N or C,
   **A=** alkyl (preferably C1 to C6 alkyl), alkenyl, alkynyl, alkoxy, carbonyl, carboxyl, cycloalkyl (preferably C3-C6 cycloalkyl), aryl (preferably phenyl), sulfonyl
   **R2** = alkyl (preferably C1 to C6-alkyl), alkenyl, alkynyl, alkoxy, carbonyl, carboxyl, alkyl carbonyl, alkoxy carbonyl, amine (preferably primary, secondary or tertiary amine), aryl (preferably phenyl or naphtyl), C3 to C8 cycloalkyl, a 5 or 6-membered heterocyclyl or heteroaryl comprising one or more of N, O and/or S, and
   **R3** = is absent, H, halogen (preferably Cl, Br, F), alkyl (preferably C1 to C6 alkyl), haloalkyl, OH, alkoxy (preferably OCH₃, OCH₂CH₃), alkoxy carbonyl (preferably - COOCH₃, - CH₂COOCH₃, -CH₂CH₂COOCH₃), cycloalkyl (preferably C3 to C6 cycloalkyl), aryl (preferably phenyl), alkyl-cycloalkyl, C1 to C6 alkyl-aryl, heterocyclyl, heteroaryl, carboxamide, CO-Cx alkyl carboxamide (preferably CON(CH₂CH₂)₂O); amine (preferably primary, secondary or tertiary amine), carbamate, nitro, -CN.
2. Compound according to the preceding embodiment, according to **Formula II** 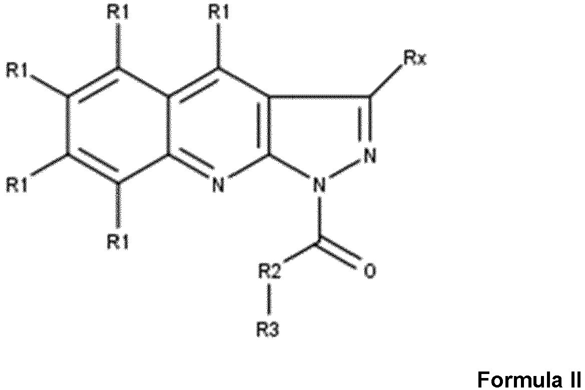 wherein
   **R1** = can be the same or different, H, OH, halogen (preferably Br, Cl or F), alkyl (preferably C1 to C3 alkyl), alkenyl, alkynyl, alkoxy (preferably OCH₃, OCH₂CH₃), alkyl carbonyl, carboxyl, alkoxy carbonyl, amine (preferably primary, secondary or tertiary amine),
   **Rx** = amine (preferably primary, secondary or tertiary amine), OH, H, alkyl (preferably C1 to C3 alkyl),
   **R2** = alkyl (preferably C1 to C3-alkyl), alkenyl, alkynyl, alkoxy, carbonyl, carboxyl, alkyl carbonyl alkoxy carbonyl, amine (preferably primary, secondary or tertiary amine), aryl (preferably phenyl or naphtyl), C3 to C8-cycloalkyl, a 5- or 6-membered heterocyclyl or heteroaryl comprising one or more of N, O and/or S, and
   **R3** = H, halogen (preferably Cl, Br, F), alkyl (preferably C1 to C3 alkyl), haloalkyl, OH, alkoxy (preferably OCH₃, OCH₂CH₃), alkoxy carbonyl (preferably - COOCH₃, -CH₂COOCH₃, - CH₂CH₂COOCH₃), cycloalkyl (preferably C3 to C6 cycloalkyl), aryl (preferably phenyl), alkyl-cycloalkyl, C1 to C6 alkyl-aryl, heterocyclyl, heteroaryl, carboxamide, CO-Cx alkyl carboxamide (preferably CON(CH₂CH₂)₂); amine (preferably primary, secondary or tertiary amine), carbamate, nitro, -CN.
3. Compound according to any one of the preceding embodiments, according to **Formula III** 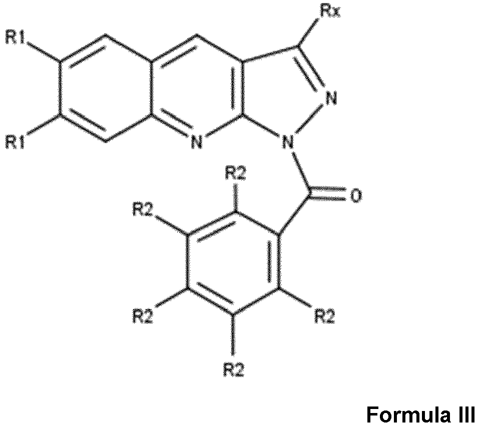 wherein
   **R1** = can be the same or different, H, OH, halogen (preferably Br, Cl or F), alkyl (preferably C1 to C3 alkyl), alkenyl, alkynyl, alkoxy, alkyl carbonyl, carboxyl, alkoxy carbonyl, amine (preferably primary, secondary or tertiary amine),
   **Rx** = amine (preferably primary, secondary or tertiary amine), OH, H, alkyl (preferably C1 to C3 alkyl), and
   **R2** = can be the same or different, halogen (preferably Cl, Br, F), alkyl (preferably C1 to C6 alkyl), haloalkyl, OH, alkoxy (preferably OCH₃, OCH₂CH₃), alkoxy carbonyl (preferably - COOCH₃, -CH₂COOCH₃, -CH₂CH₂COOCH₃), cycloalkyl (preferably C3 to C6 cycloalkyl), aryl (preferably phenyl), alkyl-cycloalkyl, C1 to C5 alkyl-aryl, heterocyclyl, heteroaryl, carboxamide, CO-Cx alkyl carboxamide (preferably CON(CH₂CH₂)₂O); amine (preferably primary, secondary or tertiary amine), carbamate, nitro, -CN, O.
4. Compound according to any one of the preceding embodiments, according to **Formula III** 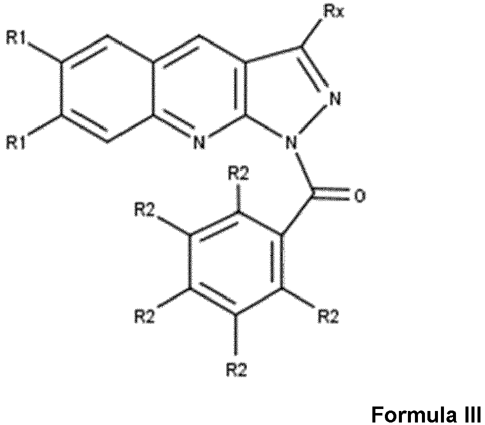 wherein
   **R1** = can be the same or different, H, OH, halogen (preferably Br, Cl or F), alkoxy, amine (preferably primary, secondary or tertiary amine),
   **Rx** = amine (preferably primary, secondary or tertiary amine), OH, H, alkyl (preferably C1 to C3 alkyl), and
   **R2** = can be the same or different, H, alkoxy (preferably OCH₃, OCH₂CH₃), cycloalkyl (preferably C3 to C6 cycloalkyl), aryl (preferably phenyl), alkyl-cycloalkyl, heterocyclyl, heteroaryl, amine (preferably primary, secondary or tertiary amine), carboxamide,
5. Compound according to the preceding embodiment, according to **Formula IV** 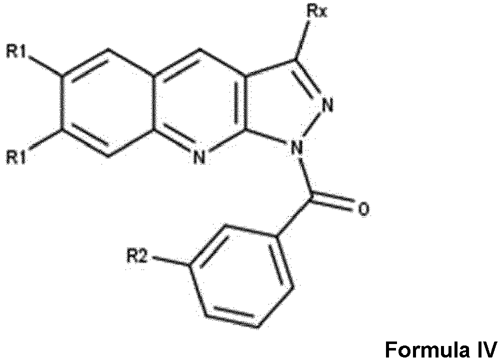 wherein
   **R1** = can be the same or different, H, OH, halogen (preferably Br, Cl or F), alkyl (preferably C1 to C3 alkyl), alkenyl, alkynyl, alkoxy, alkyl carbonyl, carboxyl, alkoxy carbonyl, amine (preferably primary, secondary or tertiary amine),
   **Rx** = amine (preferably primary, secondary or tertiary amine), H, OH, alkyl (preferably C1 to C3 alkyl), and
   **R2** = halogen (preferably Cl, Br, F), alkyl (preferably C1 to C6 alkyl), haloalkyl, OH, alkoxy (preferably OCH₃, OCH₂CH₃), alkoxy carbonyl (preferably - COOCH₃, -CH₂COOCH₃,-CH₂CH₂COOCH₃), cycloalkyl (preferably C3 to C6 cycloalkyl), aryl (preferably phenyl), alkyl-cycloalkyl, C1 to C3 alkyl-aryl, heterocyclyl, heteroaryl, carboxamide, CO-Cx alkyl carboxamide (preferably CON(CH₂CH₂)₂O); amine (preferably primary, secondary or tertiary amine), carbamate, nitro, -CN, O.
6. Compound according to any one of the preceding embodiments, according to **Formula V** 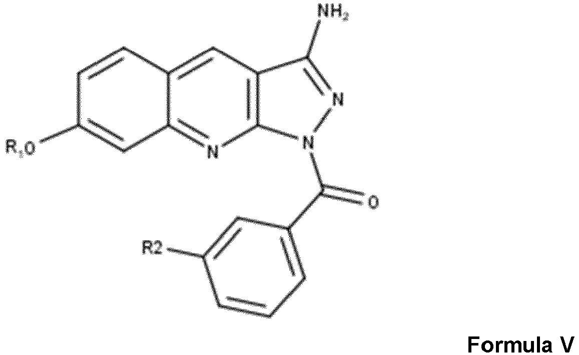 wherein
   **R1=** H, alkyl (preferably C1 to C6 alkyl), alkenyl, alkynyl, alkoxy, carbonyl, carboxyl, alkoxy carbonyl, carboxamide, CO-Cx alkyl carboxamide (preferably CON(CH₂CH₂)₂O), and
   **R2** = H, alkyl (preferably C1 to C6 alkyl), OH, alkoxy (preferably OCH₃, OCH₂CH₃), cycloalkyl (preferably C3 to C6 cycloalkyl), aryl (preferably phenyl), alkyl-cycloalkyl, heterocyclyl, heteroaryl, amine (preferably primary, secondary or tertiary amine), carboxamide.
7. Compound according to **Formula VI** 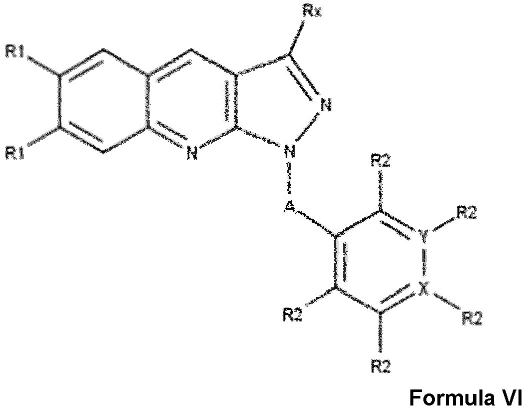 wherein
   **R1** = can be the same or different, H, OH, halogen (preferably Br, Cl or F), alkyl (preferably C1 to C6 alkyl), alkoxy (preferably OCH₃, OCH₂CH₃), amine (preferably primary, secondary or tertiary amine),
   Rx = amine (preferably primary, secondary or tertiary amine), OH, H, alkyl (preferably C1 to C3 alkyl),
   **A=** alkyl (preferably branched C1 to C6 alkyl), alkenyl, alkynyl, alkoxy, alkyl carbonyl, carbonyl, carboxyl, cycloalkyl (preferably C3 to C6 cycloalkyl), aryl (preferably phenyl), sulfonyl, carboxamide,
   **X** and **Y are** the same or different = C or N, and
   **R2** = can be the same or different, H, alkoxy (preferably OCH₃, OCH₂CH₃), alkyl (preferably C1 to C6 alkyl), haloalkyl, OH, cycloalkyl (preferably C3 to C6 cycloalkyl), aryl (preferably phenyl), alkyl-cycloalkyl, heterocyclyl, heteroaryl, carboxamide, CO-Cx alkyl carboxamide (preferably C=ON(CH₂CH₂)₂°), amine (preferably primary, secondary or tertiary amine), nitro, -CN, O,
   wherein when **Rx** is a primary or secondary amine and **R1** is OCH₃
      - **X** and/or **Y** are N and at least one of **R2** is alkoxy (preferably OCH₃, OCH₂CH₃), alkyl (preferably C1 to C6 alkyl), haloalkyl, OH, cycloalkyl (preferably C3 to C6 cycloalkyl), aryl (preferably phenyl), alkyl-cycloalkyl, heterocyclyl, heteroaryl, carboxamide, CO-Cx alkyl carboxamide (preferably C=ON(CH₂CH₂)₂), amine (preferably primary, secondary or tertiary amine), nitro, -CN, O, and/or
      - **A** is alkyl (preferably branched C1 to C6 alkyl), alkenyl, alkynyl, alkoxy (preferably OCH₃, OCH₂CH₃), alkyl carbonyl, carboxyl, cycloalkyl (preferably C3 to C6 cycloalkyl), aryl (preferably phenyl), sulfonyl, carboxamide, and/or
      - at least one of **R2** is alkyl (preferably C1 to C6 alkyl), haloalkyl, OH, cycloalkyl (preferably C3 to C6 cycloalkyl), aryl (preferably phenyl), alkyl-cycloalkyl, heteroaryl, carboxamide, CO-Cx alkyl carboxamide (preferably C=ON(CH₂CH₂)₂), nitro, -CN, O, and/or
      - at least one of **R2** is a 5-membered heterocyclyl comprising one or more of N, S and/or O and at least one of **X** or **Y** is N.
8. Compound according to the preceding embodiment, according to **Formula VII** 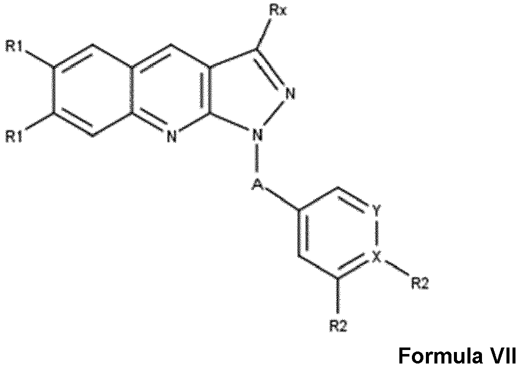 wherein
   **R1** = can be the same or different, H, OH, halogen (preferably Br, Cl or F), alkoxy (preferably OCH₃, OCH₂CH₃), amine (preferably primary, secondary or tertiary amine),
   **Rx** = amine (preferably primary, secondary or tertiary amine), OH, H, alkyl (preferably C1 to C3 alkyl),
   **A=** alkyl (preferably branched C1 to C6 alkyl), alkenyl, alkynyl, alkoxy, alkyl carbonyl, carbonyl, carboxyl, cycloalkyl (preferably C3 to C6 cycloalkyl), aryl (preferably phenyl), sulfonyl, carboxamide, and
   **R2** = can be the same or different, H, alkoxy (preferably OCH₃, OCH₂CH₃), alkyl (preferably C1 to C3 alkyl), cycloalkyl (preferably C3 to C6 cycloalkyl), aryl (preferably phenyl), alkyl-cycloalkyl, heterocyclyl (preferably C4 to C6 heterocyclyl), heteroaryl (preferably C4 to C6 heteroaryl), amine (preferably primary, secondary or tertiary amine),
   wherein when **Rx** is a primary or secondary amine and **R1** is OCH₃
      - **X** and/or **Y** are N and at least one of **R2** is alkoxy (preferably OCH₃, OCH₂CH₃), alkyl (preferably C1 to C3 alkyl), cycloalkyl (preferably C3 to C6 cycloalkyl), aryl (preferably phenyl), alkyl-cycloalkyl, (preferably C4 to C6 heterocyclyl), heteroaryl (preferably C4 to C6 heteroaryl), amine (preferably primary, secondary or tertiary amine), and/or
      - - **A** is alkyl (preferably branched alkyl), alkenyl, alkynyl, alkoxy, alkyl carbonyl, carboxyl, cycloalkyl (preferably C3 to C6 cycloalkyl), aryl (preferably phenyl), sulfonyl, carboxamide, and/or
      - at least one of **R2** is alkyl (preferably C1 to C3 alkyl), cycloalkyl (preferably C3 to C6 cycloalkyl), aryl (preferably phenyl), alkyl-cycloalkyl, heteroaryl, a 6-membered heterocyclyl, and/or
      - at least one of **R2** is a 5-membered heterocyclyl comprising one or more of N, S and/or O and at least one of **X** or **Y** is N.
9. Compound according to any one of embodiments 7 and 8, according to **Formula VII** 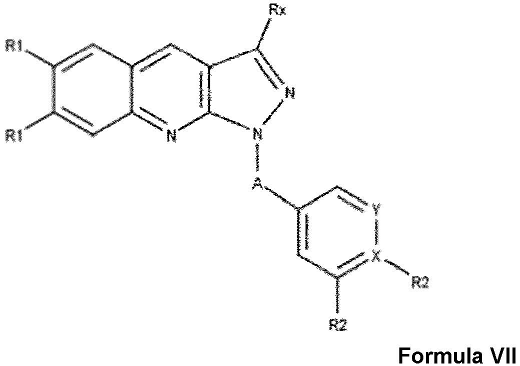 wherein
   **R1** = can be the same or different, H, OH, halogen (preferably Br, Cl or F), alkoxy (preferably OCH₃, OCH₂CH₃), amine (preferably primary, secondary or tertiary amine),
   **Rx** = amine (preferably primary, secondary or tertiary amine), OH, H, alkyl (preferably C1 to C3 alkyl),
   **A=** alkyl (preferably branched alkyl), alkyl carbonyl, carbonyl C=OR, sulfonyl, carboxamide, and
   **R2** = can be the same or different, H, alkoxy (preferably OCH₃, OCH₂CH₃), heterocyclyl (preferably C4 to C6 heterocyclyl), amine (preferably primary, secondary or tertiary amine),
   wherein when **Rx** is a primary or secondary amine and **R1** is OCH₃
      - **X** and/or **Y are** N and at least one of **R2** is alkoxy (preferably OCH₃, OCH₂CH₃), amine (preferably primary, secondary or tertiary amine), and/or
      - - **A** is alkyl (preferably branched alkyl), alkyl carbonyl, sulfonyl, carboxamide and/or
      - at least one of **R2** a 6-membered heterocyclyl comprising one or more of N, S and/or O, and/or
      - at least one of **R2** is a 5-membered heterocyclyl comprising one or more of N, S and/or O and at least one of **X** or **Y** is N.
10. Compound according to any one of embodiments 7 to 9, according to **Formula VIII** 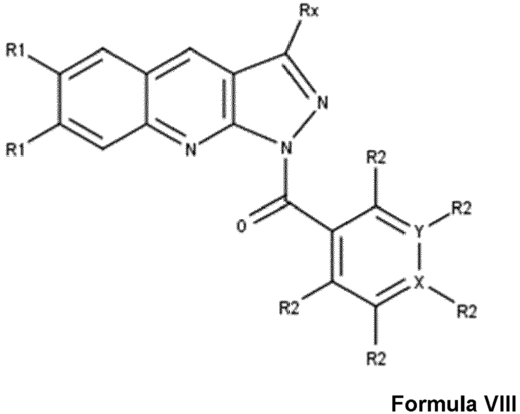 wherein
   **R1** = can be the same or different, H, OH, halogen (preferably Br, Cl or F), alkyl (preferably C1 to C6 alkyl), alkoxy (preferably OCH₃, OCH₂CH₃), amine (preferably primary, secondary or tertiary amine),
   **Rx** = amine (preferably primary, secondary or tertiary amine), OH, H, alkyl (preferably C1 to C3 alkyl),
   **X** and **Y** are the same or different = C or N, and
   **R2** = can be the same or different, H, alkoxy (preferably OCH₃, OCH₂CH₃), alkyl (preferably C1 to C6 alkyl), haloalkyl, OH, cycloalkyl (preferably C3 to C6 cycloalkyl), aryl (preferably phenyl), alkyl-cycloalkyl, heterocyclyl, heteroaryl, carboxamide, CO-Cx alkyl carboxamide (preferably C=ON(CH₂CH₂)₂^{o}), amine (preferably primary, secondary or tertiary amine), nitro, -CN, O,
   wherein when **Rx** is a primary or secondary amine and **R1** is OCH₃
      - **X** and/or **Y** are N and at least one of **R2** is alkoxy (preferably OCH₃, OCH₂CH₃), alkyl (preferably C1 to C6 alkyl), haloalkyl, OH, cycloalkyl (preferably C3 to C6 cycloalkyl), aryl (preferably phenyl), alkyl-cycloalkyl, heterocyclyl, heteroaryl, carboxamide, CO-Cx alkyl carboxamide (preferably C=ON(CH₂CH₂)₂^{o}), amine (preferably primary, secondary or tertiary amine), nitro, -CN, O, and/or
      - at least one of **R2** is alkyl (preferably C1 to C6 alkyl), haloalkyl, OH, cycloalkyl (preferably C3 to C6 cycloalkyl), aryl (preferably phenyl), alkyl-cycloalkyl, heteroaryl, carboxamide, CO-Cx alkyl carboxamide (preferably C=ON(CH₂CH₂)₂), nitro, -CN, O, and/or
      - at least one of **R2** is a 5-membered heterocyclyl comprising one or more of N, S and/or O and at least one of **X** or **Y** is N.
11. Compound according to any one of embodiments 7 to 10, according to Formula IX 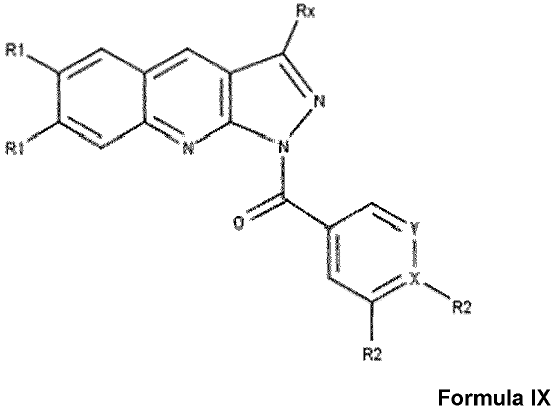 wherein
   **R1** = can be the same or different, H, OH, halogen (preferably Br, Cl or F), alkoxy (preferably OCH₃, OCH₂CH₃), amine (preferably primary, secondary or tertiary amine),
   **Rx** = amine (preferably primary, secondary or tertiary amine), OH, H, alkyl (preferably C1 to C3 alkyl), and
   **R2** = can be the same or different, H, alkoxy (preferably OCH₃, OCH₂CH₃), alkyl (preferably C1 to C3 alkyl), cycloalkyl (preferably C3 to C6 cycloalkyl), aryl (preferably phenyl), alkyl-cycloalkyl, heterocyclyl (preferably C4 to C6 heterocyclyl), heteroaryl (preferably C4 to C6 heteroaryl), amine (preferably primary, secondary or tertiary amine),
   wherein when **Rx** is a primary or secondary amine and **R1** is OCH₃
      - **X** and/or **Y are** N and at least one of **R2** is alkoxy (preferably OCH₃, OCH₂CH₃), alkyl (preferably C1 to C3 alkyl), cycloalkyl (preferably C3 to C6 cycloalkyl), aryl (preferably phenyl), alkyl-cycloalkyl, (preferably C4 to C6 heterocyclyl), heteroaryl (preferably C4 to C6 heteroaryl), amine (preferably primary, secondary or tertiary amine), and/or
      - at least one of **R2** is alkyl (preferably C1 to C3 alkyl), cycloalkyl (preferably C3 to C6 cycloalkyl), aryl (preferably phenyl), alkyl-cycloalkyl, heteroaryl, a 6-membered heterocyclyl, and/or
      - at least one of **R2** is a 5-membered heterocyclyl comprising one or more of N, S and/or O and at least one of **X** or **Y** is N.
12. Compound according to any one of embodiments 7 to 11, according to **Formula IX** 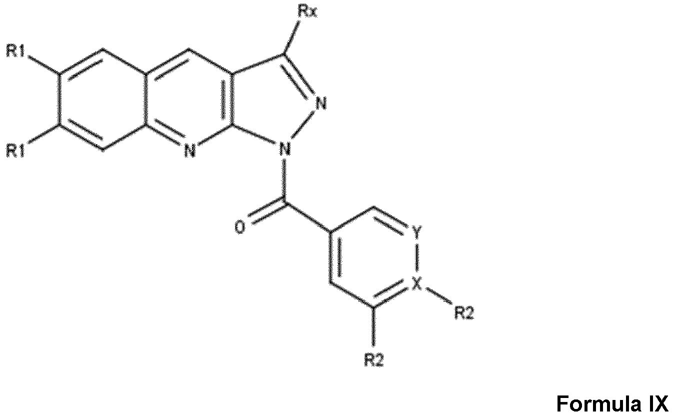 wherein
   **R1** = can be the same or different, H, OH, halogen (preferably Br, Cl or F), alkoxy (preferably OCH₃, OCH₂CH₃), amine (preferably primary, secondary or tertiary amine),
   **Rx** = amine (preferably primary, secondary or tertiary amine), OH, H, alkyl (preferably C1 to C3 alkyl), and
   **R2** = can be the same or different, H, alkoxy (preferably OCH₃, OCH₂CH₃), heterocyclyl (preferably C4 to C6 heterocyclyl), amine (preferably primary, secondary or tertiary amine),
   wherein when **Rx** is a primary or secondary amine and **R1** is OCH₃
      - **X** and/or **Y are** N and at least one of **R2** is alkoxy (preferably OCH₃, OCH₂CH₃), amine (preferably primary, secondary or tertiary amine), and/or
      - at least one of **R2** a 6-membered heterocyclyl comprising one or more of N, S and/or O, and/or
      - at least one of **R2** is a 5-membered heterocyclyl comprising one or more of N, S and/or O and at least one of **X** or **Y** is N.
13. Compound for use in the treatment of a cancer exhibiting genotoxic stress-induced IKK/NF-κB activation according to any one of the preceding embodiments, wherein the compound is more effective in inhibiting NF-κB-signaling induced by genotoxic stress compared to inhibiting NF-κB-signaling induced by TNF-alpha and/or IL-1β.
14. Compound for use in the treatment of a cancer exhibiting genotoxic stress-induced IKK/NF-κB activation according to any one of the preceding embodiments, wherein the disease is associated with genomic instability due to defective DNA-repair mechanisms, and/or wherein said cancer is associated with NF-κB-mediated resistance to therapy-induced tumor cell apoptosis, and/or wherein the compound is administered in combination with one or more genotoxic stress-inducing (DNA damage-inducing) cancer therapies, such as genotoxic stress-inducing chemotherapy and/or irradiation therapy.
15. *In vitro* method for the inhibition of genotoxic stress-induced NF-kB signaling or inhibition of DNA repair mechanisms, preferably in a cell-based assay, comprising the use of a compound according to any one of the preceding embodiments.

### FIGURES

The invention is further described by the following figures. These are not intended to limit the scope of the invention, but represent preferred embodiments of aspects of the invention provided for greater illustration of the invention described herein.

### Brief description of the figures

**Fig.1****:** Identification of inhibitors specific for DNA damage-induced NF-κB activity.
**Fig.2****:** MW01 and MW05 are potent and selective kinase inhibitors.
**Fig.3****:** CLKs are the shared targets of active derivatives of MW01 and MW05.
**Fig.4****:** MW01 and MW05 reduce cell viability and increase p53-mediated apoptosis upon etoposide co-treatment.
**Fig.5****:** Screening, synthesis and characterization of compounds.
**Fig.6****:** ADME studies and degradation of MW01 and MW05.
**Fig.7****:** Investigations on the inhibition of critical signaling events in the genotoxic stress-induced NF-κB pathway by MW01 and MW05.
**Fig.8****:** Investigations on CLK as shared target of MW01 and MW05 and derivatives thereof.
**Fig.9****:** High-resolution mass trace of MW05-E1.
**Fig.10****:** High-resolution mass trace of MW05-E2.
**Fig.11****:** High-resolution mass trace of MW05-E3.
**Fig.12****:** High-resolution mass trace of MW05-E4.
**Fig.13****:** High-resolution mass trace of MW05-E5.
**Fig.14****:** High-resolution mass trace of MW05-E6.
**Fig.15****:** High-resolution mass trace of MW05-E7.
**Fig.16****:** High-resolution mass trace of MW05-E8.
**Fig.17****:** High-resolution mass trace of MW05-E9.
**Fig.18****:** High-resolution mass trace of MW05-E10.
**Fig.19****:** High-resolution mass trace of MW05-E11.
**Fig.20****:** High-resolution mass trace of MW05-E12.
**Fig.21****:** High-resolution mass trace of MW05-E13.
**Fig.22****:** High-resolution mass trace of MW05-E14.
**Fig.23****:** High-resolution mass trace of MW05-E15.

### Detailed description of the figures

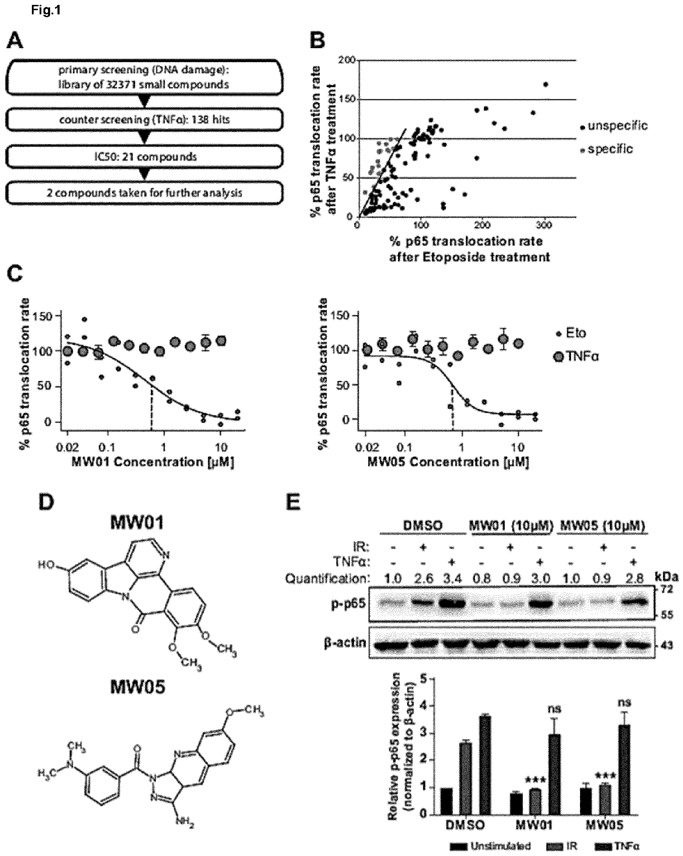

**Fig.1****:** Identification of inhibitors specific for DNA damage-induced NF-κB activity. (A) Flowchart representing the high-throughput chemical library screening. A screen/counter-screen strategy was used by stimulating NF-κB in U2-OS cells with etoposide (50 µM for 90 min) and hits were counter-screened with TNFα (10 ng/ml for 20 min). Cells were pre-treated with compounds (10 µM) for 1 hour before stimulation, fixed, and percent p65 nuclear translocation measured by fluorescence microscopy. IC50 values were used for final selection. (B) Comparison of mean p65 nuclear translocation following either etoposide or TNFa stimulation after treatment with each counter-screened hit compound. Values were normalized to etoposide or TNFα positive controls. Specific inhibitors (red) of DNA damage-induced NF-κB signaling were defined by setting cut-off criteria (black line). To be regarded specific, an inhibitor had to decrease etoposide-induced p65 nuclear translocation by at least 25% and concomitantly the reduction of etoposide-induced p65 translocation had to be at least 1.5 times more effective than the TNFα-induced p65 translocation. (C) IC50 determination for MW01 (left) and MW05 (right) of p65 nuclear translocation, following either etoposide or TNFα treatment. MW01 etoposide-stimulated IC50: 460nM, TNFα stimulated: out of range. MW05 etoposide-stimulated IC50: 690nM, TNFα-stimulated: out of range. IC50 noted with a dotted line. (D) Structures of compounds selected as leads for further analysis, MW01 (5-Hydroxy-10,11-dimethoxy-9H-benzo[c]indolo[3,2,1-ij][1,5]naphthyridin-9-one) and MW05 (3-Amino-7-methoxypyrazolo[3,4-b]quinolin-1-yl)-[3-(dimethylamino)phenyl]methanone). (E) Western blot analysis (top) and quantification (bottom) of NF-κB subunit p65 phosphorylation (S536) following 1-hour pre-treatment with DMSO, MW01 (10 µM), or MW05 (10 µM), in unstimulated, after 90 min irradiation (IR, 20 Gy), or after 20 min TNFα (10 ng/mL) conditions in U2- OS cells. B-actin was used as loading control after. The p-p65 signal was first normalized to β-actin loading control per sample, then each value to DMSO-treated unstimulated to evaluate relative activation of NF-κB. Ns: not significant; ***: p<0.001.

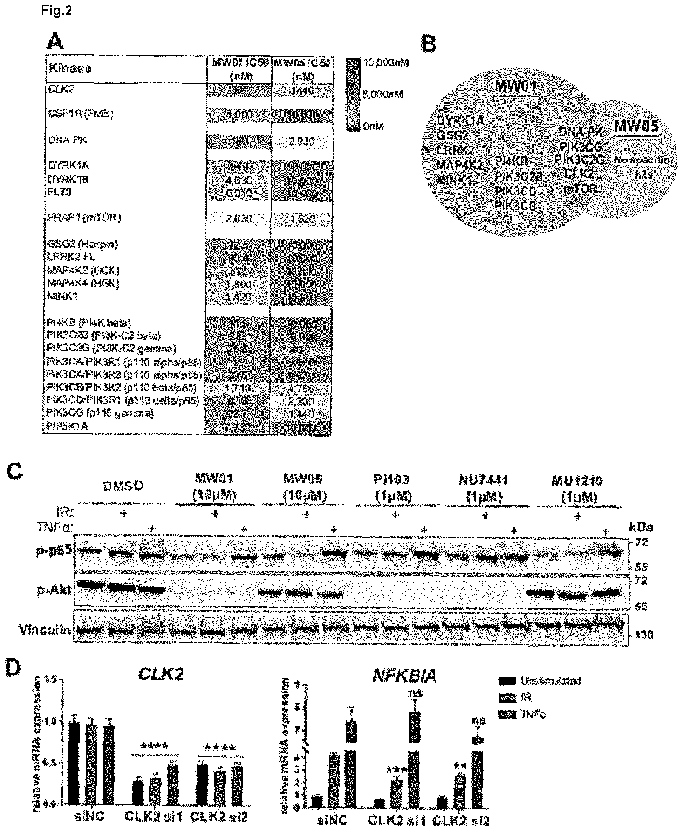

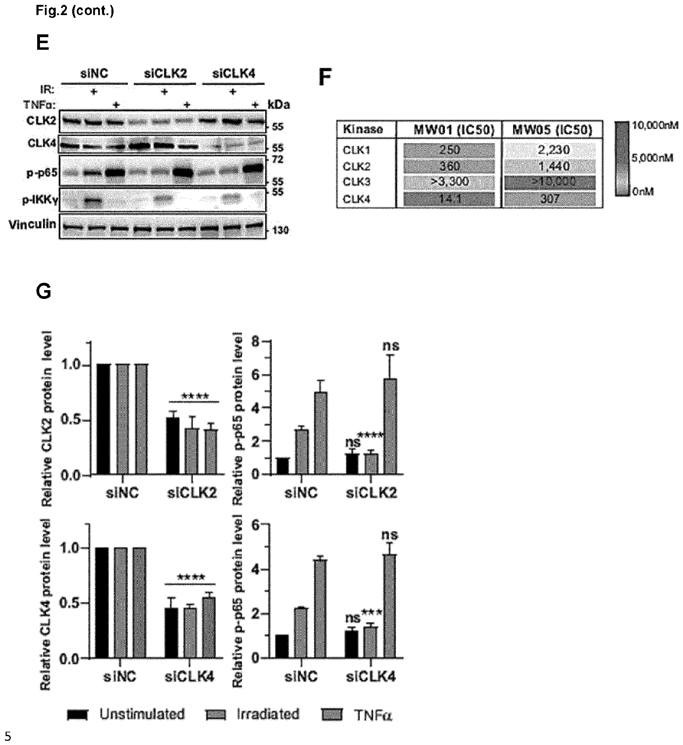

**Fig.2****:** MW01 and MW05 are potent and selective kinase inhibitors. (A) IC50 (nM) of all kinases strongly inhibited by MW01 and MW05. Heatmap indicates lower IC50. (B) Venn diagram depicting strongly inhibited kinases for MW01 and MW05, with shared hits in the overlapping region. (C) Western blot analysis of NF-κB p65 phosphorylation (S536) and Akt phosphorylation (S473) following 1-hour pre-treatment with DMSO, MW01 (10 µM), MW05 (10µM), PI-103 (1 µM), NU7441 (1 µM), or MU1210 (1 µM) in unstimulated, after 90 min irradiation (IR, 20 Gy), or after 20 min TNFα (10 ng/ml) conditions in U2-OS cells. (D) mRNA expression analysis of CLK2 (top), 48 hours after transfection with siNC, CLK2 si1, or CLK2 si2. mRNA expression analysis of NFKBIA (bottom), 48 hours after transfection with siNC, CLK2 si1, orCLK2 si2. Values are normalized to unstimulated siNC expressing cells. Statistical comparison was made between experimental conditions and respective paired siNC expressing controls. ns: not significant; **, ***, ****: p<0.01, p<0.001, p<0.000. (E) Western blot analysis of U2-OS cells after transfection with siNC or CLK2 si2 unstimulated, after irradiation (IR), or after TNFα treatment. Vinculin used as loading control. (F) IC50 (nM) of all CLK isoforms for MW01 and MW05. (G) Quantification from (E) with CLK2 knockdown and p-p65 quantification (top left and top right, respectively) and CLK4 knockdown and p-p65 (bottom left and bottom right, respectively. ns: not significant; **, ***, ****: p<0.01, p<0.001, p<0.0001.

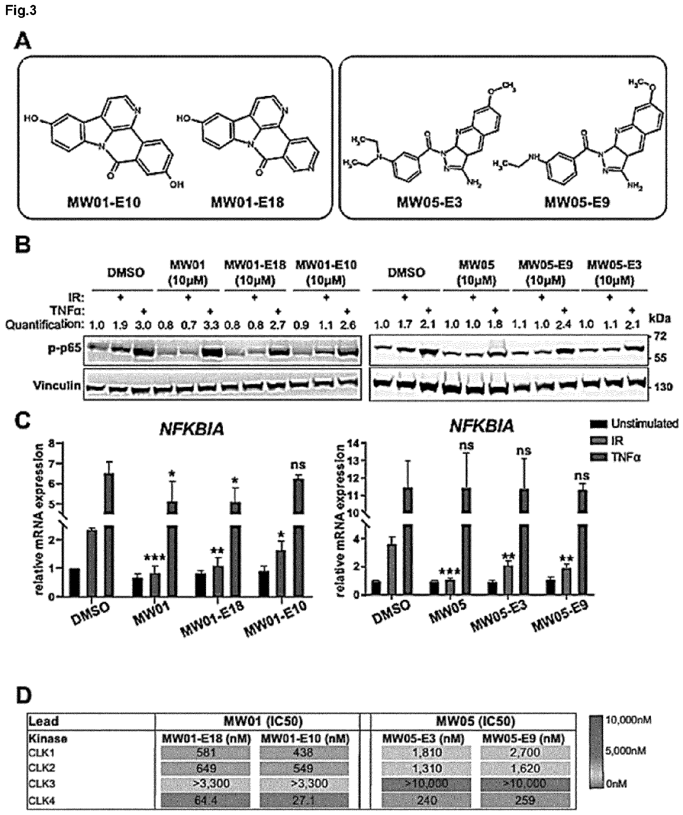

**Fig.3****:** CLKs are the shared targets of derivatives of MW01 and MW05. (A) Selected structural derivatives of MW01 and MW05, as indicated. MW01-E10 (5,16-dihydroxy-1,11-diazapentacyclo[10.7.1.0²,⁷.0⁸,²⁰.0¹³,¹⁸]icosa-2(7),3,5,8(20),9,11,13(18),14,16-nonaen-19-one); MW01-E18 (5-hydroxy-1,11,16-triazapentacyclo[10.7.1.0²,⁷.0⁸,²⁰.0¹³,¹⁸]icosa-2(7),3,5,8(20),9,11,13(18),14,16-nonaen-19-one ); MW05-E3 1-[3-(diethylamino)benzoyl]-7-methoxy-1H-pyrazolo[3,4-b]quinolin-3-amine; MW05-E9 7-methoxy-1-[3-(methylamino)benzoyl]-1Hpyrazolo[3,4-b]quinolin-3-amine. (B) Western blot analysis of p65 phosphorylation (S536) following 1-hour pre-treatment with DMSO, MW01 (10 µM), MW01-E18 (10 µM), or MW01-E10 (10 µM) in unstimulated, after 90 min irradiation (IR, 20 Gy), or after 20 min TNFα (10 ng/ml) conditions in U2-OS cells (left). Western blot analysis of p65 phosphorylation following 1-hour pre-treatment with DMSO, MW05 (10 µM), MW05-E9 (10 µM), or MW05-E3 (10 µM) in unstimulated, after 90 min irradiation (IR, 20 Gy), or after 20 min TNFα (10 ng/mL) conditions in U2-OS cells (right). Vinculin is used as loading control. (C) mRNA expression analysis of NFKBIA using the same experimental conditions as (B) for MW01 and derivatives (left) and MW05 and derivatives (right). Values were normalized to DMSO-treated, unstimulated cells. Statistical comparison was made between experimental conditions and respective paired DMSO control. ns: not significant; *, **, ***: p<0.05, p<0.01, p<0.001. (D) IC50 (nM) of all CLK isoforms for MW01-E10 and MW01-E18 (left), and MW05-E3 and MW05-E9 (right).

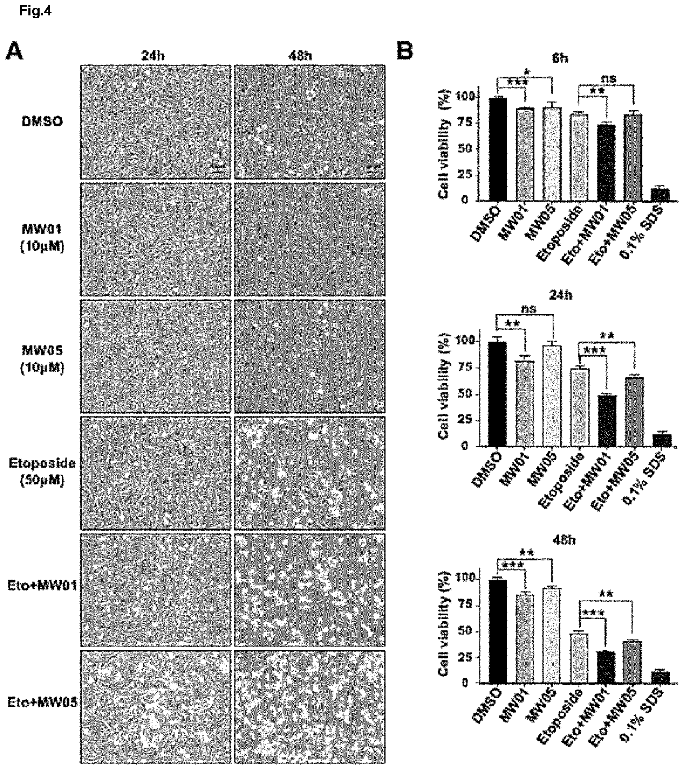

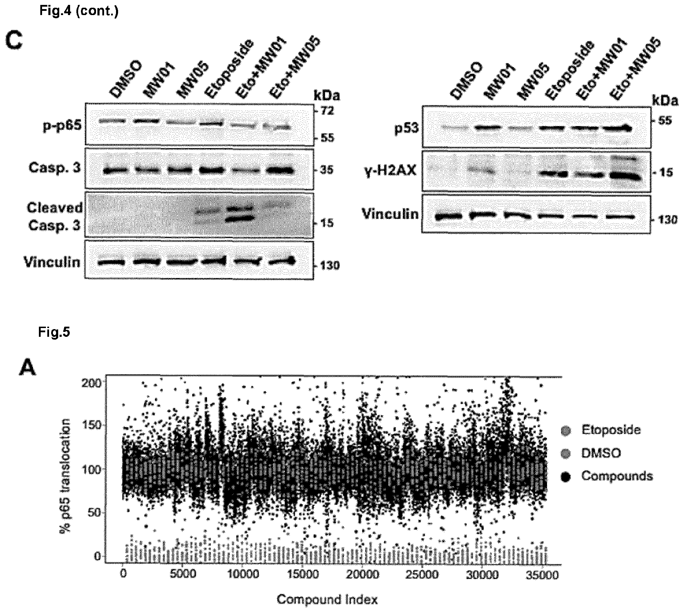

**Fig.4****:** MW01 and MW05 reduce cell viability and increase p53-mediated apoptosis upon etoposide co-treatment. (A) Representative light microscopy images of U2-OS cells following treatment with DMSO, MW01 (10 µM), MW05 (10 µM), or etoposide (50 µM), or co-treatment with MW01 (10 µM) and etoposide (50 µM), or MW05 (10 µM) and etoposide (50 µM) at 24 (left) and 48 hours (right). (B) Quantification of cell viability using CellTiter-Fluor Cell Viability Assay at 6, 24, and 48 hours. Treatment with 0.1% SDS 30 minutes prior to assay was used as positive cell death control. (ns: not significant; *, **, ***: p<0.05, p<0.01, p<0.001). (C) U2-OS cells were treated with DMSO, MW01 (10 µM), MW 05 (10 µM), or etoposide (50 µM), or co-treated with MW01 (10 µM) and etoposide (50 µM), or MW05 (10 µM) and etoposide (50 µM), as indicated. After 24 hours, whole cell lysates were prepared and analyzed by Western blotting for p65 phosphorylation (S536), caspase-3 and cleaved caspase-3 (left panel) and for p53 and γH2AX) (right panel). Vinculin was used as loading control.

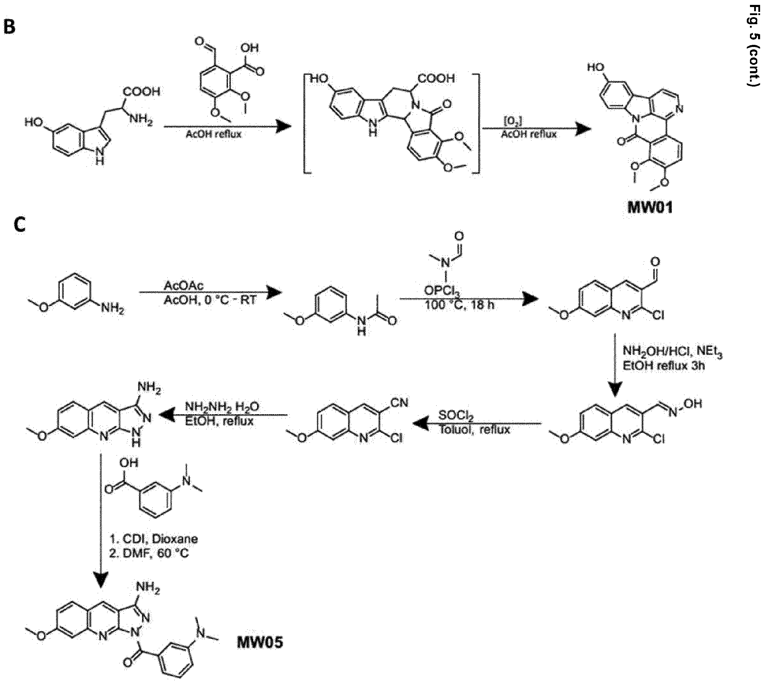

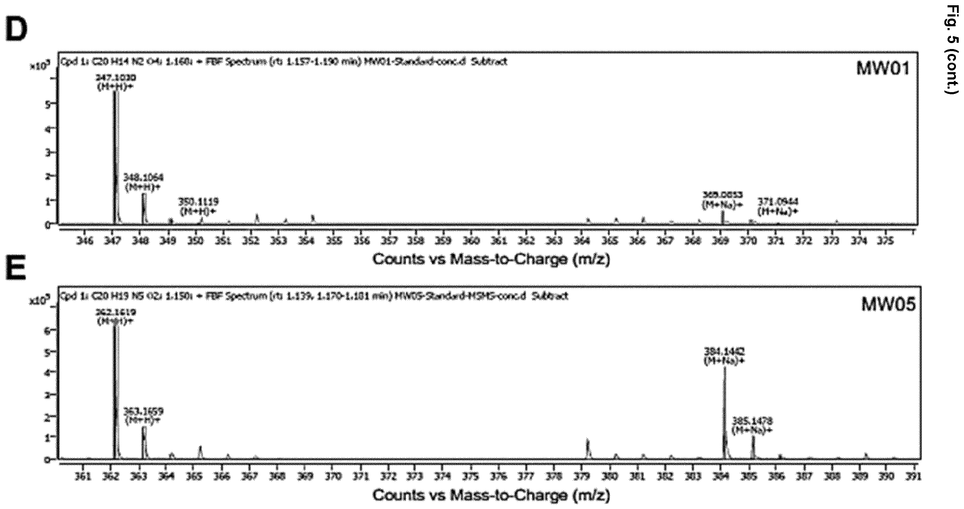

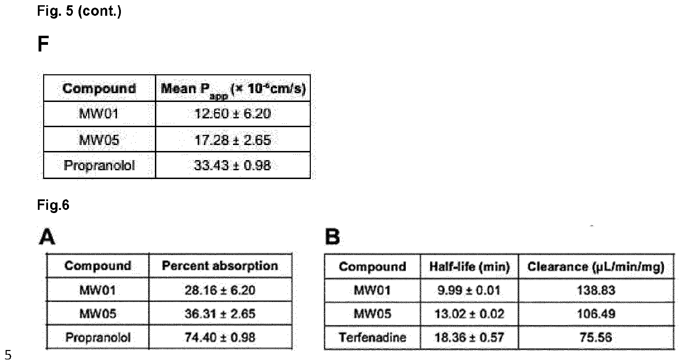

**Fig.5****:** Diagram displaying distribution of all compounds tested in primary screening. The ordinate displays compound-dependent activity of p65 nuclear translocation in correlation to etoposide treated controls. (B) General synthetic scheme for to MW01 (Sen'ko et al., 2017). (C) General synthetic scheme for MW05 (Lapa et al., 2013). (D) High-resolution mass trace of MW01. (E) High-resolution mass trace of MW05. (F) Mean apparent permeability (Pₐₚₚ) of MW01, MW05, or positive control propranolol in CaCo2 cells.

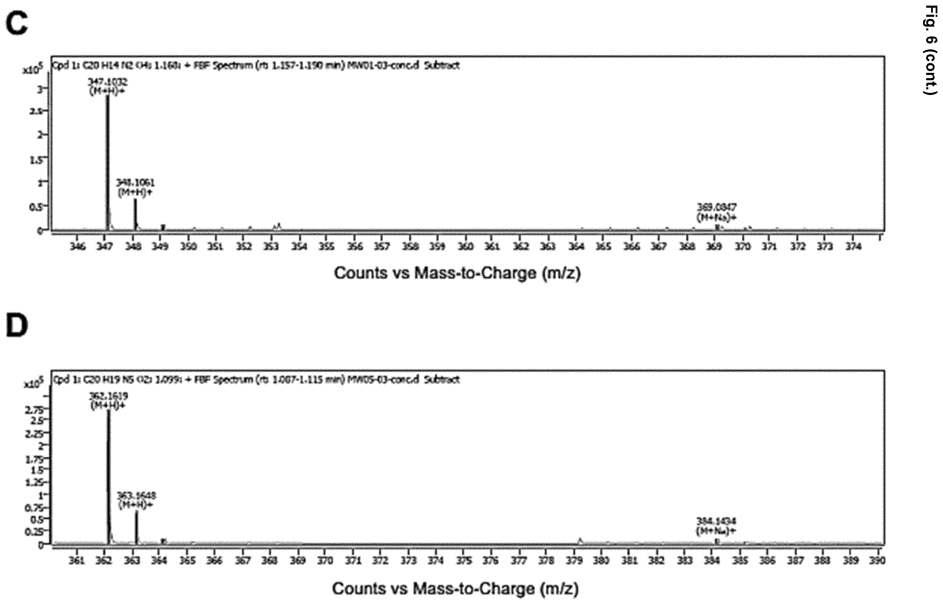

**Fig.6****:** (A) Mean % absorption of MW01, MW05, or positive control propranolol in CaCo2 cells. (B) Half-life (left) and clearance (right) of MW01, MW05, or positive control terfenadine in human liver microsomes. (C) HPLC of media supernatant from U2-OS cell culture one week after MW01 treatment (10µM). (D) HPLC of media supernatant from U2-OS cell culture one week after MW05 treatment (10µM).

**Fig.7****:** (A) Immunoprecipitation of IKKγ from HEK293 lysates pre-treated for 1 hour with DMSO, MW01 or MW05, then irradiated (IR, 20 Gy) and lysed. (B) Western blot analysis of MEF cells pre-treated with DMSO, MW01, MW05 or ATM inhibitor KU55933 and irradiated (IR, 20 Gy). Lysates were subjected to SDS-PAGE/WB. The specific IKKγ S85 band (lower band) was identified by induction following irradiation and by sensitivity to ATMi and λ- phosphatase (λ-PP) treatment. (C) Western blot analysis of U2os cells pre-treated for 1 hour with the indicated substances, irradiated (IR, 20 Gy) and whole cell lysates probed for poly(ADP)-ribose. (D) Western blot analysis of cytoplasmic and nuclear fractions from U2-OS cells for p-ATM (S 1981), after 1 hour pre-treatment with DMSO, MW01 (10 µM), or MW05 (10 µM) and 45 min following irradiation (IR, 20 Gy). PARP1 and LDHA are used as loading controls for nuclear and cytoplasmic fractions respectively. (E) mRNA expression analysis of CLK4 (top), 48 hours after transfection with siNC or siCLK4. mRNA expression analysis of NFKBIA (bottom), 48 hours after transfection with siNC or siCLK4. Values normalized to unstimulated siNC expressing cells. Statistical comparison was made between experimental conditions and paired siNC control. ns: not significant; **, ***, ****: p<0.01, p<0.001, p<0.0001. (F) Western blot analysis of p65 phosphorylation (S536) and CLK4 expression after transfection with siNC or siCLK4 in unstimulated, after 90 min irradiation (IR, 20 Gy), or after 20 min TNFα (10 ng/mL) conditions in U2-OS cells. Vinculin is used as loading control. Note that CLK4 and p-p65 were blotted on separate membranes, with respective vinculin blots underneath. (G) MW01 and MW05 are inhibitors of DNA damage-induced p65 nuclear translocation. U2-OS cells were pre-treated with DMSO, MW01 or MW05. Etoposide was added and after 2 hours cells were fixed and nuclei stained with DAPI. P65 and γH2AX were stained by immunofluorescence and images taken at a confocal Zeiss 710 LSM microscope with a 40x oil objective. (H) Western blot analysis of U2-OS cells 8 hours after 10 Gy irradiation following 1 hour pre-treatment with 10 µM DMSO, MW01, or MW05. Tubulin was used loading control. (I) mRNA expression analysis of BIRC3 in U2-OS cells 8 hours after 10 Gy irradiation following 1 hour pre-treatment with 10 µM DMSO, MW01, or MW05 and. Values were normalized to DMSO-treated unstimulated cells.

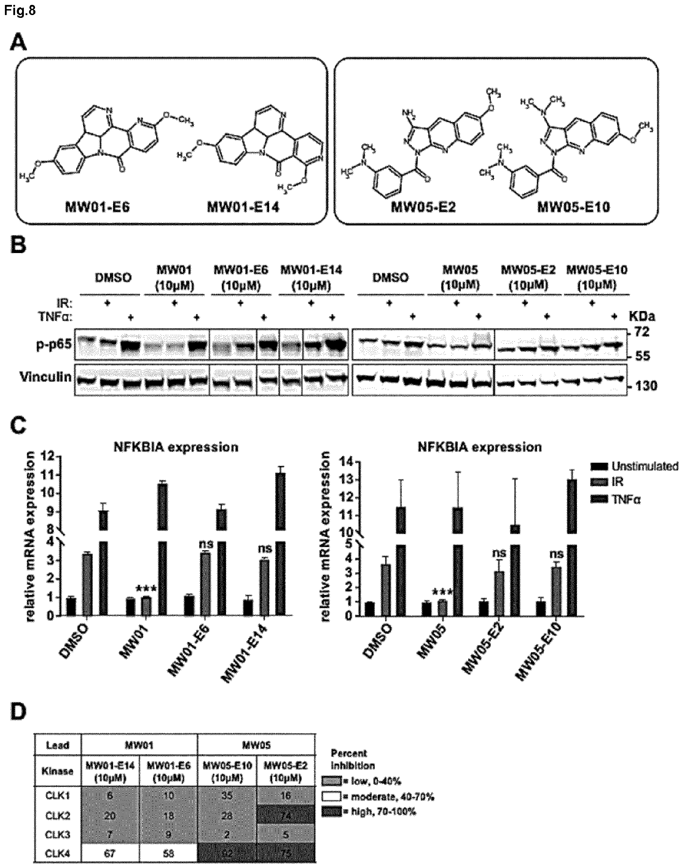

**Fig.8****:** (A) Selected structural derivatives of MW01 and MW05, as indicated. MW01-E6 (5,15-dimethoxy-1,11,14-triazapentacyclo[10.7.1,0²,7.0⁸,²⁰.0¹³,¹⁸]icosa-2(7),3,5,8(20),9,11,13(18),14,16-nonaen-19-one); and MW01-E14 (5,17-dimethoxy-1,11,16-triazapentacyclo[10.7.1.0²,⁷.0⁸,²⁰.0¹³,¹⁸]icosa-2(7),3,5,8(20),9,11,13(18),14,16-nonaen-19-one); MW05-E2 (1-[3-(dimethylamino)benzoyl]-6-methoxy-1H-pyrazolo[3,4-b]quinolin-3-amine); MW05-E10 (1-[3-(dimethylamino)benzoyl]-7-methoxy-N,N-dimethyl-1H-pyrazolo[3,4-b]quinolin-3-amine). (B) Western blot analysis of p65 phosphorylation (S536) following 1 hour pre-treatment with DMSO, MW01 (10µM), MW01-E6 (10 µM), or MW01-E14 (10 µM) in unstimulated, after 90 min irradiation (IR, 20 Gy), or after 20 min TNFα (10 ng/mL) conditions in U2-OS cells (left). Western blot analysis of p65 phosphorylation (S536) following 1 hour pre-treatment with DMSO, MW05 (10 µM), MW05-E2 (10 µM), or MW05-10 (10 µM) in unstimulated, after 90 min irradiation (IR, 20 Gy), or after 20 min TNFα (10 ng/mL) conditions in U2-OS cells (right). Vinculin is used as loading control. (C) mRNA expression analysis of NFKBIA using same experimental conditions as in (B) for MW01 and derivatives (left) and MW05 and derivatives (right). Values normalized to DMSO unstimulated. Statistical comparison was made between experimental conditions and respective paired DMSO control. ns: not significant; *, **, ***: p<0.05, p<0.01, p<0.001. (D) Percent inhibition of all CLK isoforms by derivatives MW01-E6 and MW01-E14 (left), and MW05-E2 and MW05-E10, which were inactive in cell-based assays.

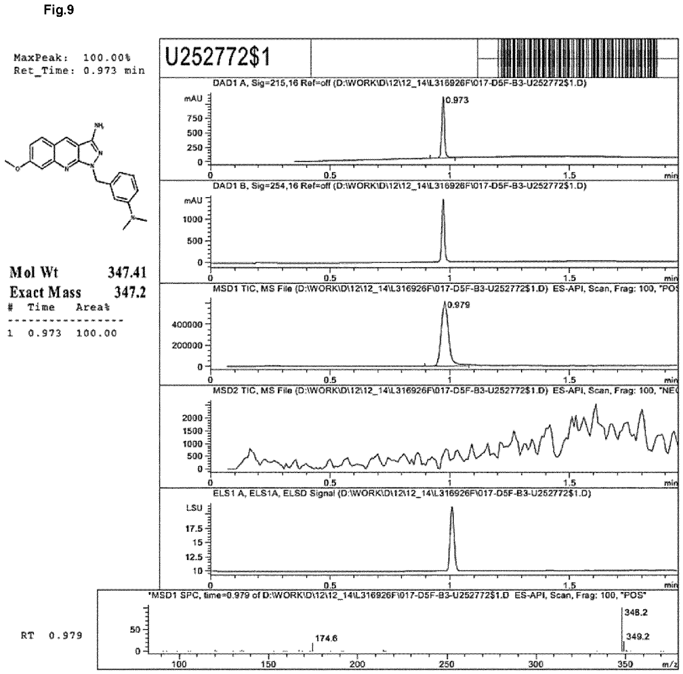

**Fig.9****:** High-resolution mass trace of MW05-E1 measured by LC-MS/MS.

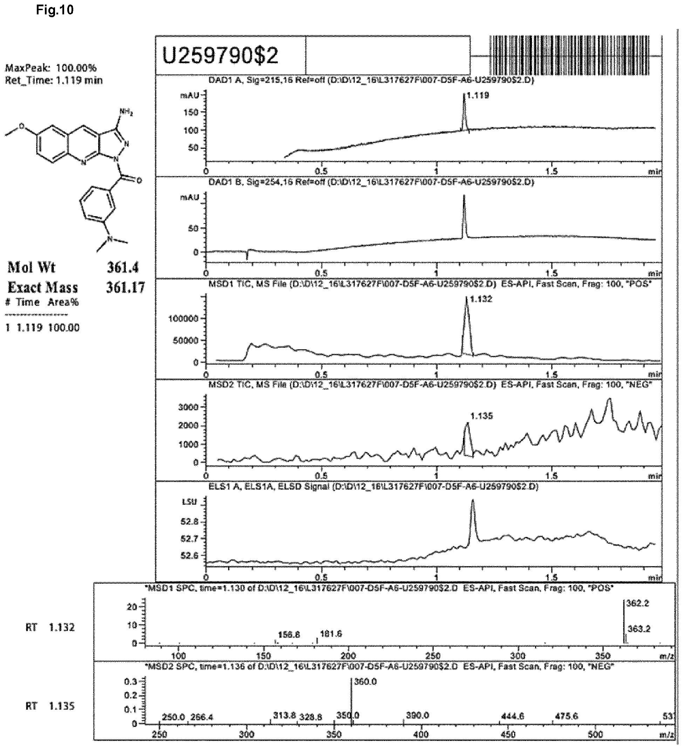

**Fig.10****:** High-resolution mass trace of MW05-E2 measured by LC-MS/MS.

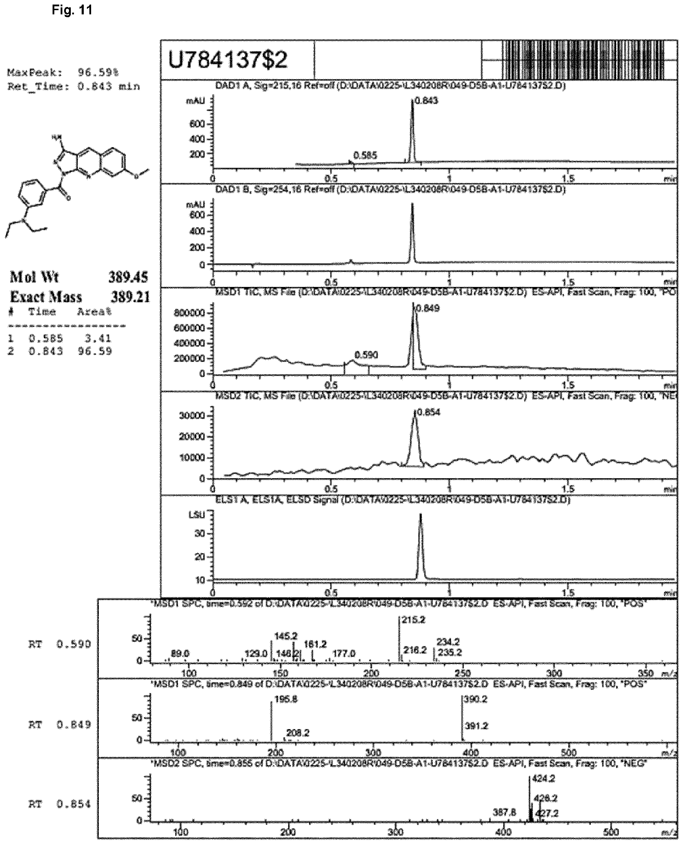

**Fig.11****:** High-resolution mass trace of MW05-E3 measured by LC-MS/MS.

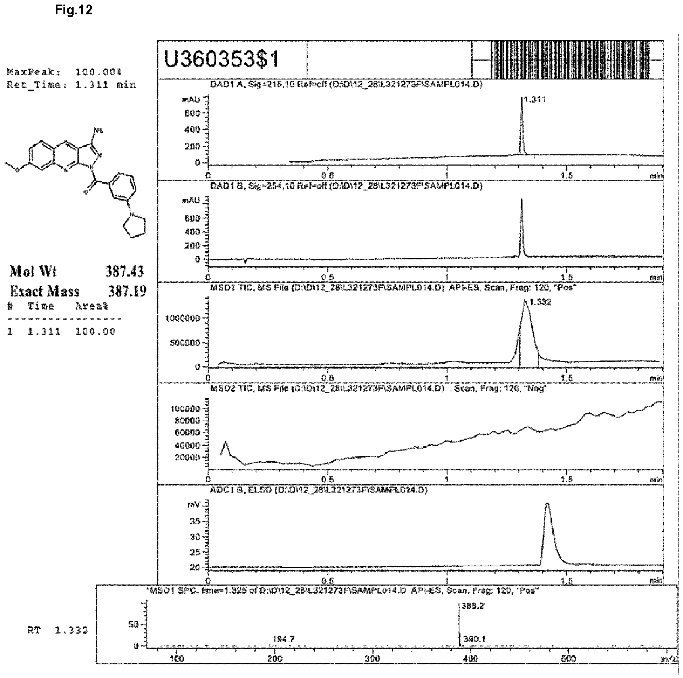

**Fig.12****:** High-resolution mass trace of MW05-E4 measured by LC-MS/MS.

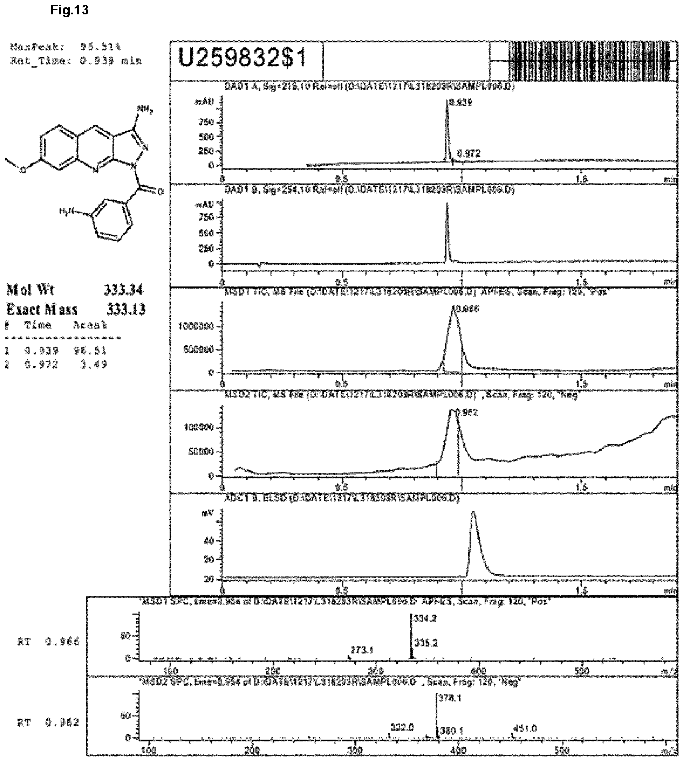

**Fig.13****:** High-resolution mass trace of MW05-E5 measured by LC-MS/MS.

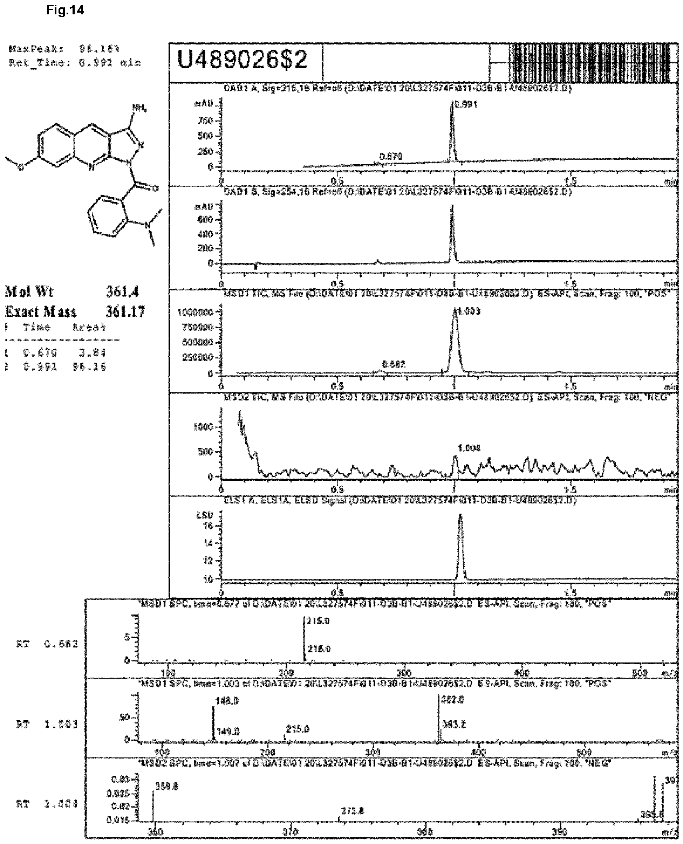

**Fig.14****:** High-resolution mass trace of MW05-E6 measured by LC-MS/MS.

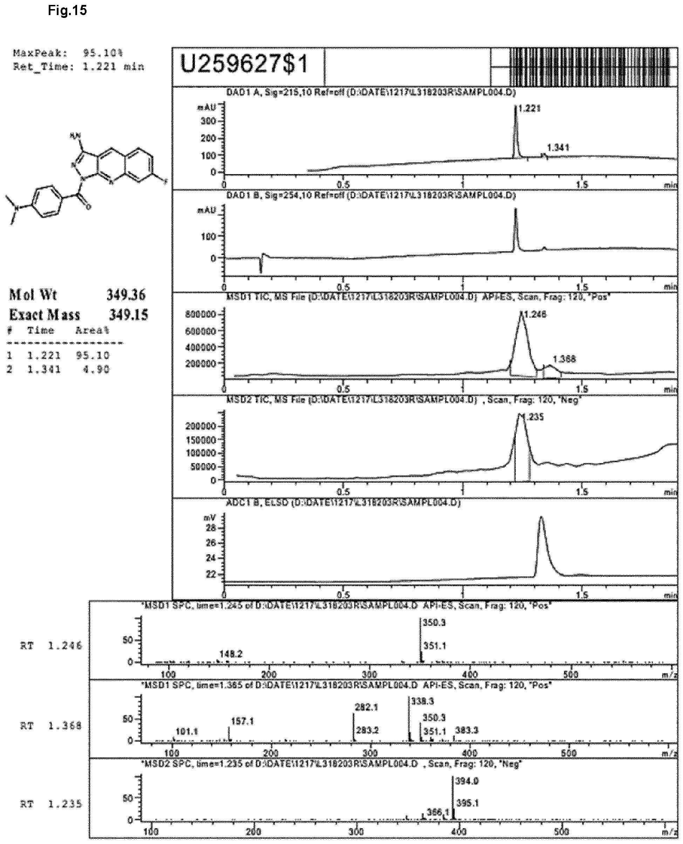

**Fig.15****:** High-resolution mass trace of MW05-E7 measured by LC-MS/MS.

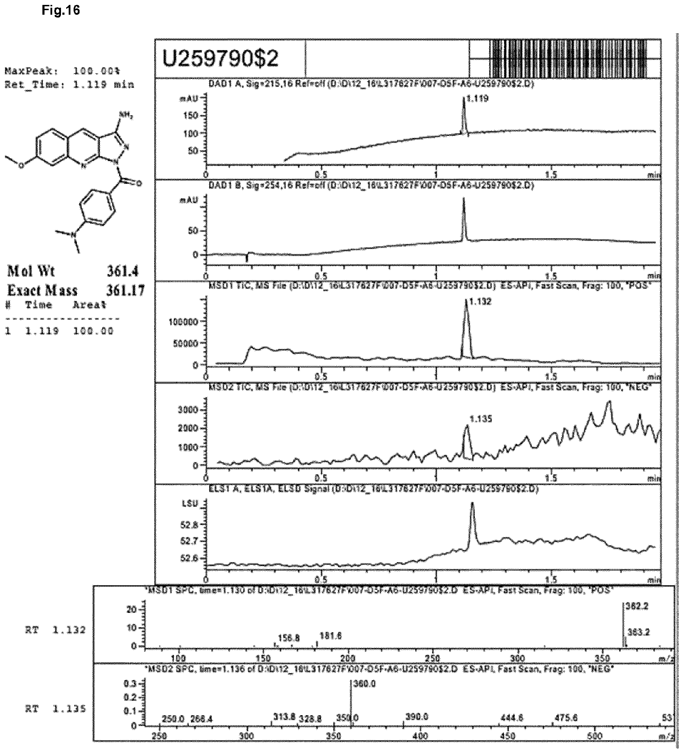

**Fig.16****:** High-resolution mass trace of MW05-E8 measured by LC-MS/MS.

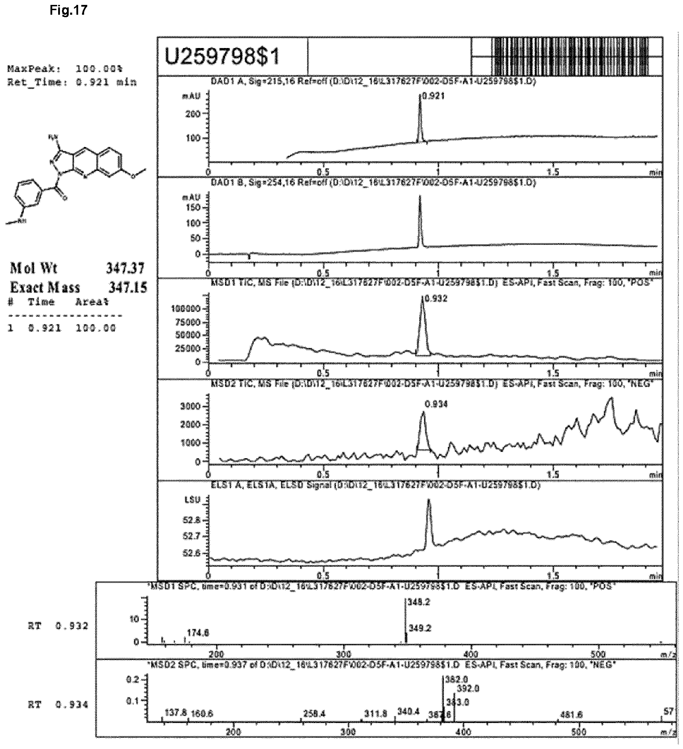

**Fig.17****:** High-resolution mass trace of MW05-E9 measured by LC-MS/MS.

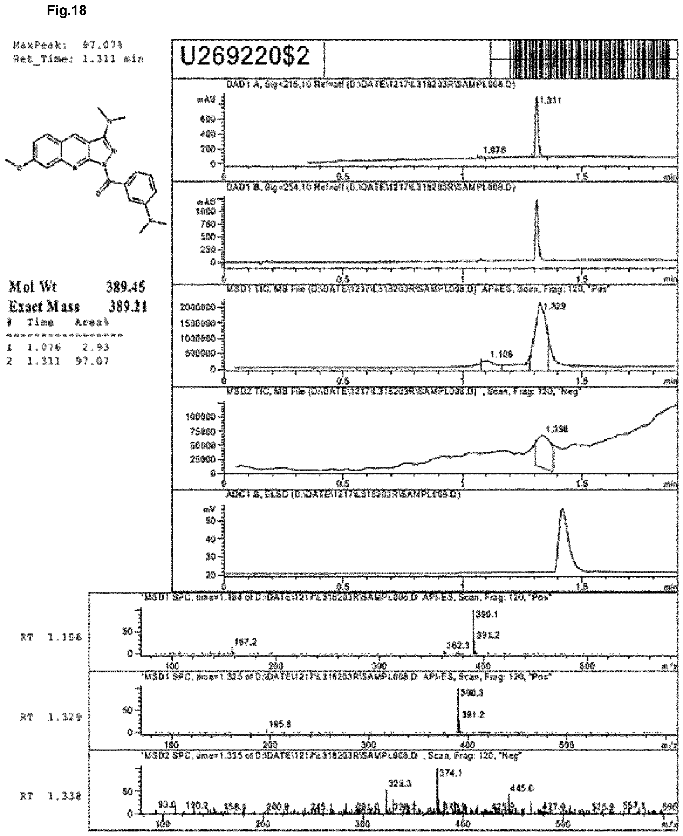

**Fig.18****:** High-resolution mass trace of MW05-E10 measured by LC-MS/MS.

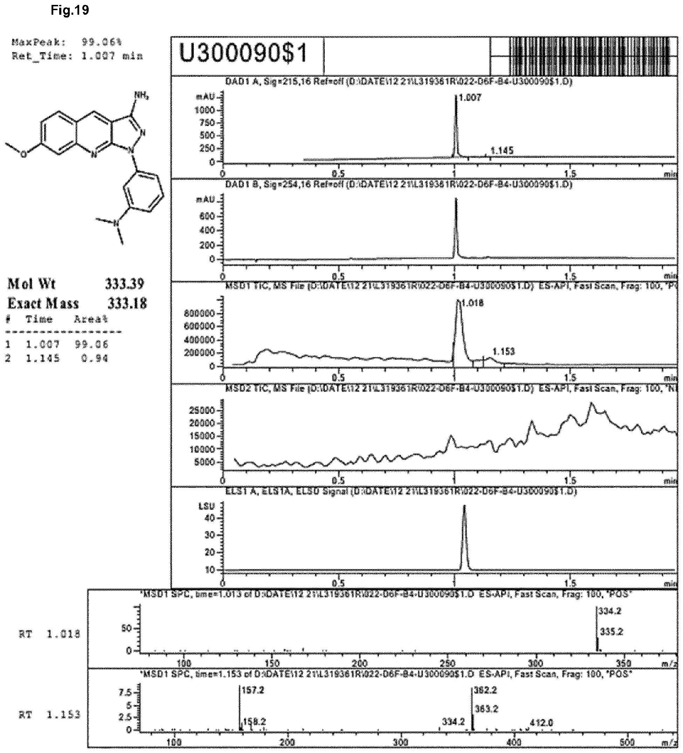

**Fig.19****:** High-resolution mass trace of MW05-E11 measured by LC-MS/MS.

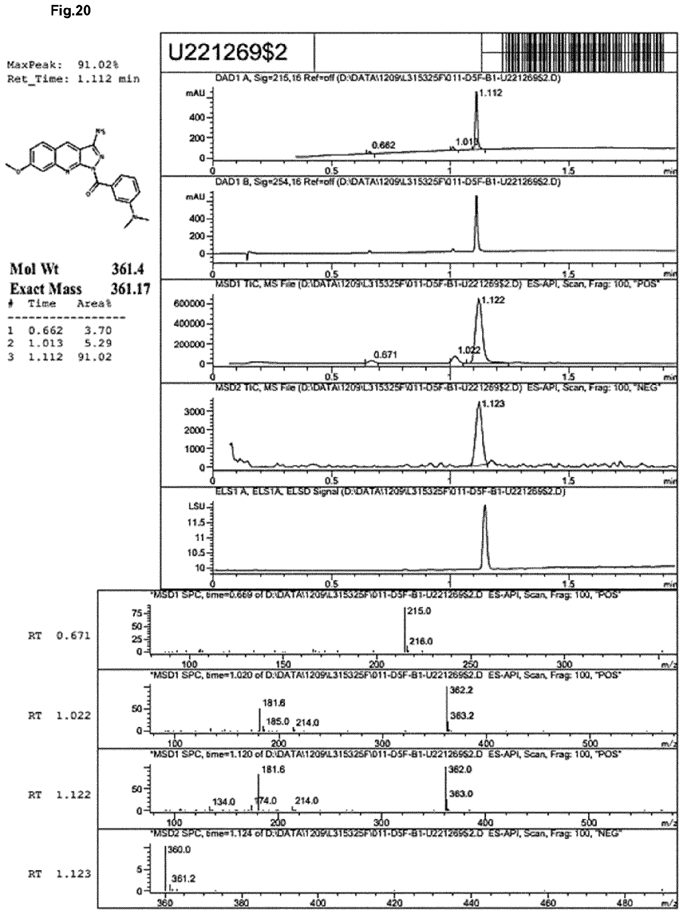

**Fig.20****:** High-resolution mass trace of MW05-E12 measured by LC-MS/MS.

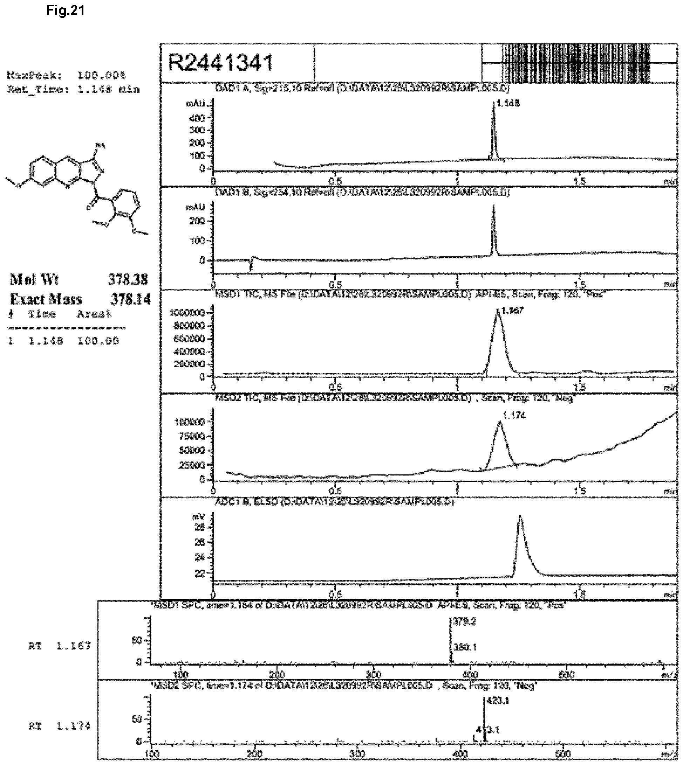

**Fig.21****:** High-resolution mass trace of MW05-E13 measured by LC-MS/MS.

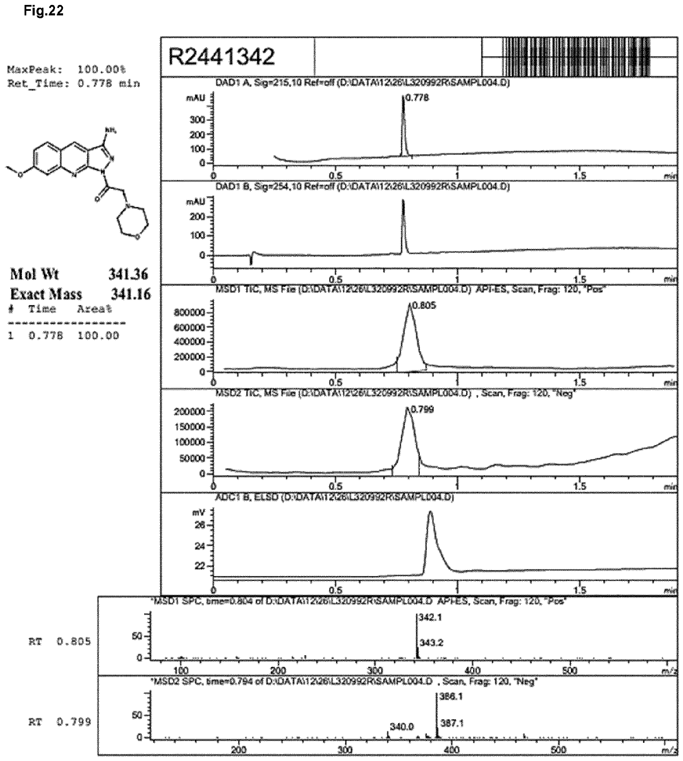

**Fig.22****:** High-resolution mass trace of MW05-E14 measured by LC-MS/MS.

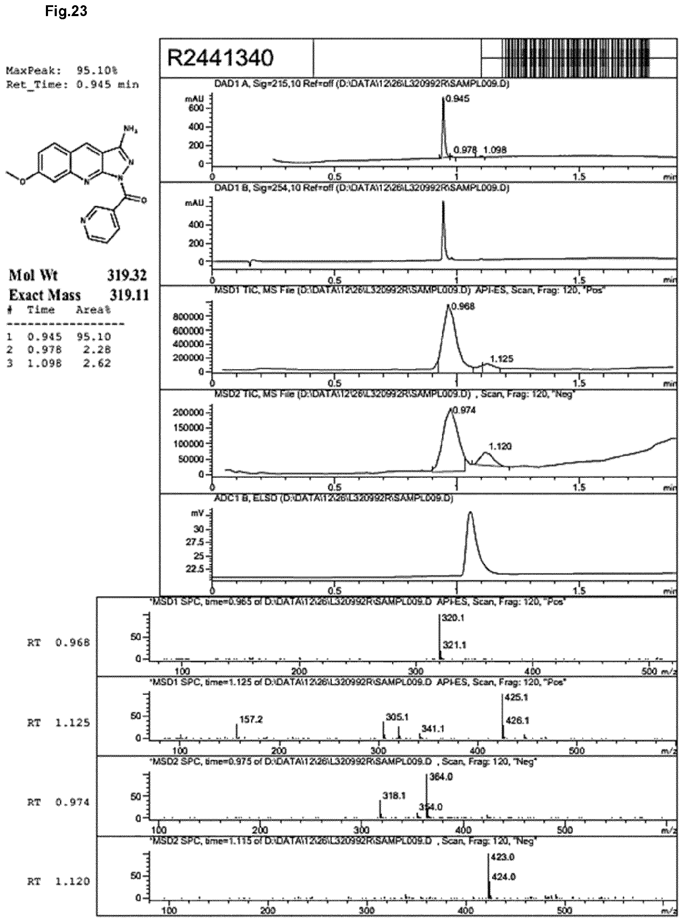

**Fig.23****:** High-resolution mass trace of MW05-E15 measured by LC-MS/MS.

### EXAMPLES

The invention is further described by the following examples. These are not intended to limit the scope of the invention but represent preferred embodiments of aspects of the invention provided for greater illustration of the invention described herein.

### Methods employed in the examples

### Cell lines

U2-OS cells (DSMZ) were propagated in DMEM medium supplemented with 10% FBS, 1% pen-strep, and 1% L-glutamine at 37°C and 5% CO2. In some experiments, cells were silenced with 50nM of CLK2 or CLK4 siRNAs (Table S5) and transfected using Lipofectamine RNAiMAX Trasfection Reagent (Thermo Fisher Scientific, 13778100). All experiments were performed 72h post-silencing.

### HCS screening of NF-κB inhibitor library

U2-OS cells were seeded in a 384-well microplate (Corning) (750 cells/well), and after 48 h they were treated with a library of 32.000 compounds concentrated 10 µM in DMSO. After 2 h of treatment, DNA damage was induced using 50 µM etoposide (Sigma Aldrich, E1383) for 2 h. Cells were washed with PBS 1X, fixed with PFA 4% (Sigma Aldrich, 158127) for 10 minutes and washed again with PBS 1X. Subsequently, cells were incubated with a solution of 0.12% glycine/0.2% saponin in PBS for 10 min and blocked with 10% FCS/0.2% saponin in PBS for 1h. Primary antibody anti-p65 (Santa Cruz Biotechnologies, sc-372) was incubated for 1 h at RT (1:500 dilution in 0.2% saponin in PBS). Cells were washed four times with washing solution (0.2% saponin in PBS). Following washing steps, incubation with the secondary antibody anti mouse conjugated with Alexa 488 (Thermo Fisher Scientific, A-11001) (1:1000 diluted in 0.2% saponin in PBS) was carried out for 1 h at RT. Cells were washed again four times with washing solution and nuclei were stained by incubation with 10 µM Hoechst 33342 (Sigma Aldrich, B2261) in washing solution for 10 min. For data acquisition, 36 images per well were taken using the automated wide field fluorescence microscope ArrayScan VTI HCS reader (Thermo Fisher Scientific) and p65 nuclear translocation rates were calculated by applying masks to define the nuclear and cytoplasmic areas.

### LC-MS/MS measurements

The following LCMS conditions were used: Column: Agilent Poroshell 120 SB-C18 4.6x30mm 2.7 µm; Column temperature: 60°C; Mobile phase: A - water (0.1% formic acid), B - acetonitrile (0.1% formic acid); Flow rate: 3 ml/min; Gradient: 0.01 min - 1% B, 1.5 min - 100% B, 1.73 min - 100% B; MS Ionization mode: Electrospray ionization (ESI); MS Scan range: 83 - 600 m/z; UV detection: 215 nm, 254nm, 280 nm.

The equipment used was: (DAD) Agilent 1100 Series G1315B, (ELSD) Alltech 2000ES, (MASS) Agilent 1100 Series G1956B for compounds MW05-E4, MW05-E5, MW05-E7, MW05-E10, MW05-E13, MW05-E14, and MW05-E15; (DAD) Agilent 1200 Series G1315D, (ELSD) Agilent 1290 Infinity II G7115A, (MASS) Agilent 6100 Series G6120B for compounds MW05-E1, MW05-E6, and MW05-E12, and (DAD) Agilent 1260 Infinity II G7115A, (ELSD) Agilent 1290 Infinity II G7115A, (MASS) Agilent 6100 Series G6125B, for compounds MW05-E2, MW05-E3, MW05-E8, MW05-E9, and MW05-E11.

### NF-κB Stimulation

NF-κB was stimulated by DNA damage through either 20 Gy irradiation (IR) and analyzed after 90 minutes or by 10 ng/ml TNFα and analyzed after 20 minutes unless otherwise noted.

### siRNA Transfection

Cells were silenced with 50 nM of CLK2 or CLK4 siRNAs (Table 3) and transfected using Lipofectamine RNAiMAX Transfection Reagent (Thermo Fisher Scientific, 13778100). All experiments were performed 48 h post-silencing.

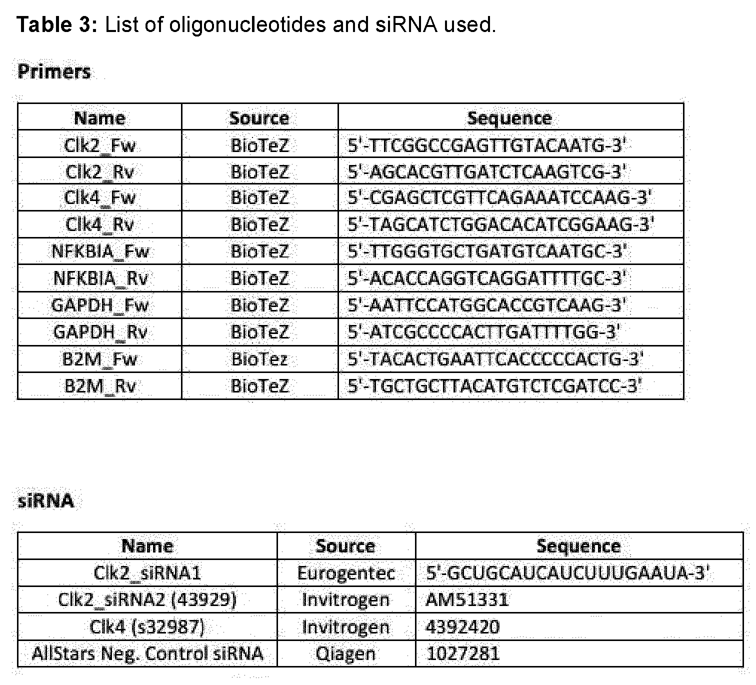

### Drug Treatment

All compounds were pre-treated for 1-hour prior to NF-κB stimulation unless otherwise noted.

### Nuclear/Cytoplasm fractionation

Initially, cells were lysed with Buffer A (20mM Tris-HCl pH 7.9, 1.5mM MgCl₂, 10mM KCI, 8mM β-Glycerol, 20mM NaF, 200mM Na₃VO₄, 1mM DTT, 0.2% NP-40) supplemented with Protease (Thermo Fisher Scientific, 78429) and Phosphatase (Thermo Fisher Scientific, 78420) inhibitors and 50nM Calyculin A. Lysate was vortexed for 10 second and centrifuged. The supernatant, representing the cytoplasm extract (CE), was collected, while the pellet was washed with Buffer A and the suspended with Buffer C (40mM Tris-HCl pH 7.9, 25% Glycerol, 420mM NaCl, 1.5mM MgCl₂, 8mM β-Glycerol, 20mM NaF, 1mM DTT) supplemented with Protease (Thermo Fisher Scientific, 78429) and Phosphatase (Thermo Fisher Scientific, 78420) inhibitors. The pellet was incubated for 20 minutes at 4°C under shaking, centrifuged for 10 minutes at 14,000 rpm and the supernatant, representing the nuclear extract (NE) was collected.

### Whole cell lysate preparation and immunoblot analysis

Cells were collected by scraping and lysed in Baeuerle lysis buffer supplemented with Protease (Thermo Fisher Scientific, 78429) and Phosphatase (Thermo Fisher Scientific, 78420) inhibitors after the appropriate treatment and time point. Proteins were quantified by Bradford assay (Bio-Rad, 5000006) and 20-50 µg of extracted proteins were loaded on 4-20% Protein Gel (Bio-Rad, 4568096) and then transferred onto a PVDF membrane (Bio-Rad, 1620177) using Tris-Glycine buffer with 20% of methanol. Blocking was performed for 1h with 5% not-fat dry milk in PBS 1X. Immunodetection was obtained using primary and secondary antibodies reported in Key Resources Table. The membranes were analyzed by ECL (Thermo Fisher Scientific, 34580) and detected by ChemiDoc XRS+ (Bio-Rad) using ImageLab software (Bio-Rad).

### Absorption Distribution Metabolism Excretion (ADME) study

ADME study was entirely provided by Eurofins using the two lead compounds MW01 and MW05.

### Immunoprecipitation

1.5mg of protein lysate was used for pulldown. Lysates were precleared with 30 µl Sepharose G beads for 30 min and centrifuged for 5 min at 1,500 H*g*. Primary antibody was added to the supernatant for immunoprecipitation overnight while rotating at 4°C. The next day 30 µl of Sepharose beads per sample were used for immobilization of antibodies. IP wash buffer was used to wash the beads four times and precipitated proteins were then eluted by mixing with 3X SDS-buffer and heating to 95°C for 4min.

### Kinase panel study

The Kinase panel study was entirely provided by Thermo Fisher Scientific using the two lead compounds MW01 and MW05 as well as their derivatives (MW01-E06, MW01-E10, MW01-E14, MW01-E18, MW05-E2, MW05-E3, MW05-E9 and MW05-E10. The experiment was performed using the ZLyte or Adapta protocol according to manufacturer standard operating procedures.

### RNA extraction and RT-qPCR

3.5×10⁵ cells per well were seeded in a 6-well plate and incubated overnight. The day after, the appropriate treatment at the appropriate time point was performed and cells were scraped and lysed with β-mercaptoethanol and total RNA was extracted using an RNeasy kit (Qiagen, 74004) following the kit protocol. Then, 500 ng of RNA were retro-transcribed using the iScript cDNA synthesis kit (Bio-Rad, 1708890). Finally, RT-qPCR was performed using 5 ng of cDNA and SYBR Green PCR Master Mix (Thermo Fisher Scientific, 4309155) in Cfx96 Real-Time System Thermocycler (Bio-Rad). The sequence of all primers used is reported in Table 3.

### Cell viability assay

1×10⁴ cells per well were seeded in a 96-well plate and incubated overnight. The day after, cells were treated with 10 µM of compounds under investigation, 50 µM Etoposide or 10 µM Cisplatin, alone or in combination, for appropriate time points. DMSO and 10% of Triton-X100 were used as negative and positive control respectively. Luminescent staining was performed using CellTiter-Glo Luminescent Cell Viability Assay (Promega, G7570), adding in each well a volume of CellTiter-Glo reagent equal to the volume of cell culture medium present into the well. After 1h of incubation at 37°C, luminescence was read by BioTek Cytation 1 Cell Imaging Multimode Reader (Agilent).

### Quantification and Statistical analysis

The results are expressed as mean ± SD of three independent experiments. Graphpad Prism 7.04 was used for statistical analysis using the two-way ANOVA multiple comparison with the default setting (Tukey hypothesis testing with 95% confidence interval). All western blot quantifications were normalized to their respective loading controls. Protein level knockdown efficiency was determined by comparing siCLK2 or siCLK4 to paired siNC-expressing control. Relative NF-κB activation was calculated by comparing the p-p65 signal (for protein level analysis) or NFKBIA expression (for RNA expression analysis) of each sample to the unstimulated control.

### Resources

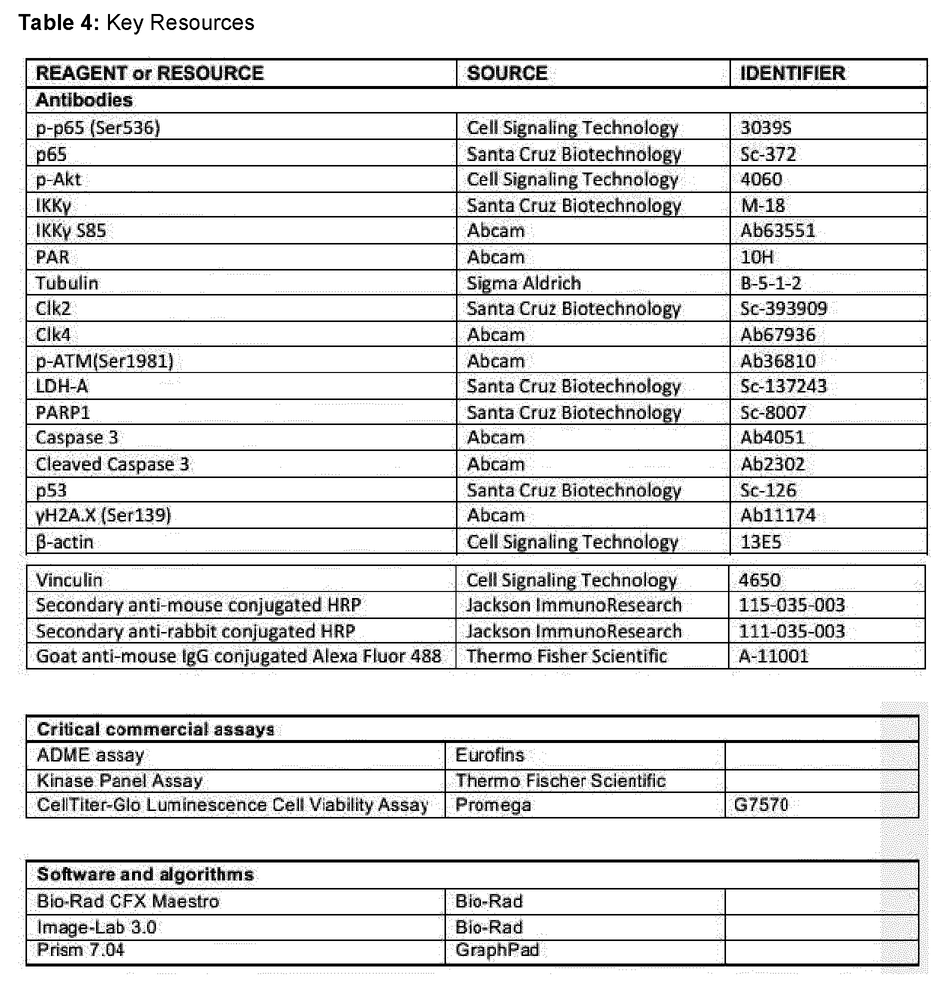

### Results of the examples

### Identification of inhibitors specific for DNA damage-induced NF-κB activity

To identify inhibitors that selectively prevent DNA damage-induced NF-κB activation, we established a high-throughput screening platform quantifying p65 nuclear translocation, an essential step in NF-κB activation (Hayden and Ghosh, 2008). A chemical library of over 32,000 compounds was screened and 138 small molecule inhibitors of etoposide-induced p65 nuclear translocation were identified and used in a subsequent counter-screen using TNFα as inducer (Fig. 1A, 5A). We employed a screen/counter-screen strategy by stimulating NF-κB with either etoposide, to induce DNA double strand breaks, or TNFα and compounds that inhibited NF-κB only after DNA damage were selected for further analysis (Fig. 1B). The IC50s of p65 nuclear translocation following both stimuli were determined for 21 compounds. Among them, two promising structurally distinct compounds, MW01 and MW05, with sub-micromolar IC50's following etoposide without affecting p65 translocation upon TNFα stimulation, were selected for further investigation (Fig. 1C, 1D). We were able to synthesize both compounds with high purity measured by HPLC and fully characterize them by NMR and LC-MS/MS (Fig. 5B, C, D, E, Table 5, Fig. 9 - 23). Moreover, from ADME studies, we found that both MW01 and MW05 show a percentage of absorption in CaCo2 cell line similar to the commercially available drug Terfenadine (Fig. 6A, B). Notably, both small molecules have minimal degradation for 72 hours in or cell culture conditions (Fig. 6C, D). To confirm the efficacy of those compounds in DNA damage-specific NF-κB inhibition, we found by immunoblot that both compounds significantly reduced phosphorylation of p65 at serine 536, the substrate site of IKK (Kolesnichenko et al., 2021), only after DNA damage generation, while leaving TNFα -stimulated and unstimulated levels untouched compared to DMSO-treated control (Fig. 1E). We first re-confirmed the inhibition of p65 nuclear translocation by our two lead compounds (Fig. 7G). In addition, other critical signaling events in the genotoxic stress-induced NF-κB pathway, such as IR-induced IKKy phosphorylation and mono-ubiquitination, were also inhibited by both lead compounds, while others, such as PARP1 activation and poly(ADP-ribose) formation or nuclear ATM activation, were not (Fig. 7A, B, C, D) (Hinz et al., 2010; Li et al., 2001; Piret et al., 1999; Wu et al., 2006). Furthermore, cleavage of PARP1, as apoptotic marker, was potentiated, while upregulation of anti-apoptotic NF-κB target gene BIRC3 was abolished by MW01 or MW05 following irradiation (Figure 7H, I). Thus, both compounds act downstream of the DNA sensors ATM and PARP1 and upstream of IKK. With these lead compounds in hand, we proceeded to investigate the molecular target, mechanism of action, and properties as antitumor drugs of our structurally distinct leads MW01 and MW05.

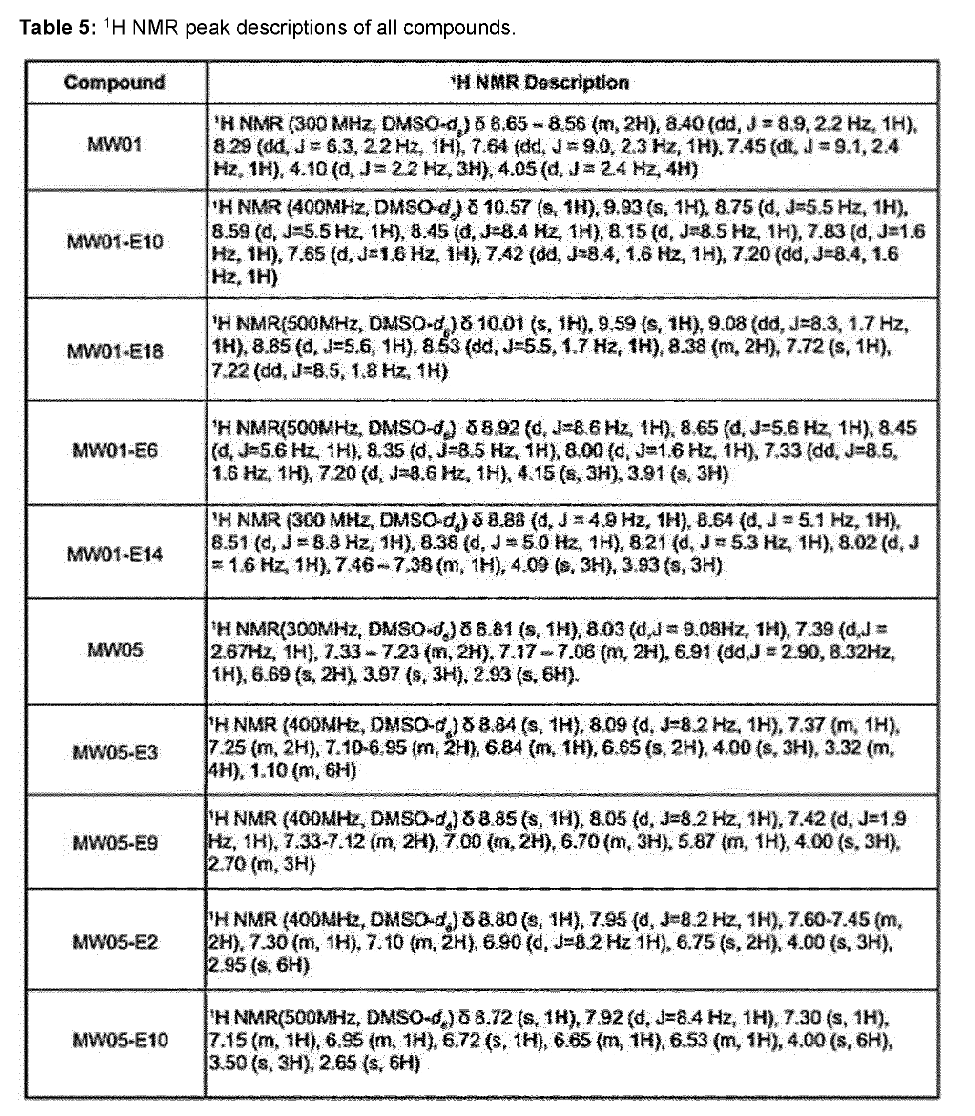

### MW01 and MW05 are potent and selective kinase inhibitors

Based on their similar effects in our screen and molecular analysis, MW01 and MW05 were used in comparative target deconvolution studies seeking common targets despite their distinct structures. To eliminate previously identified kinases regulating NF-κB activity, such as IKK, as possible targets of MW01 and MW05, an in vitro kinase inhibition assay was performed. We tested 275 diverse kinases and found that both MW01 and MW05 are kinase inhibitors with differing kinase inhibition profiles (Table 6). IC50 values were determined for all strongly inhibited kinases contained in the broad panel for both compounds (Fig 2A). Importantly, no previously known kinases within the DNA damage-induced NF-κB pathway were inhibited, suggesting that a potential shared kinase target of MW01 and MW05 could be as-yet undescribed in the context of DNA damage-induced NF-κB activity. Interestingly, several kinases shared by both lead compounds as sensitive targets were found despite the more selective kinase inhibition profile of MW05, including various phosphatidylinositol phosphate kinases and CDC-like kinase 2 (Fig 2B). We next investigated if any of the shared kinase targets of MW01 and MW05 play a role in genotoxic stress-induced NF-κB activity and established several criteria to narrow down pathway-relevant targets. Considering the similar NF-κB inhibition phenotypes of both compounds, we hypothesized first that the target should be shared by both lead compounds. Second, based on the CaCo2 recovery values and experimental treatment concentration of 10 µM), the putative target kinase should have a low IC50 of, at most, 2000 nM for both lead compounds in the in vitro kinase panel (Fig. 2A). Based on these criteria alone, we arrived at a relatively short list of potential targets for further analysis, leaving only PIK3CG, PIK3C2G, and CLK2 (Fig. 2B). IC50 values for PIK3C2G by both lead compounds agreed best with these criteria, but RNA expression analysis indicates high tissue specificity as well as lack of expression of this kinase in our U2-OS model cell line, which we then eliminated from further analysis (Uhlén et al., 2015). DNA-PK and mTOR failed to meet the sub-2000 nM IC50 criteria for MW05 and MW01, respectively, but were scrutinized experimentally (Fig. 2B). Importantly, CLK2 shared the most similar and second-lowest combined IC50 values, suggesting that strong inhibition is likely reached with a treatment concentration of 10 µM in cell-based assays, so we carried out further analysis on PIK3CG, CLK2, DNA-PK, and mTOR.

**Table 6: Percentage of inhibition of the indicated kinases by MW01 or MW05 at either 10 µM or 1 µM. n.d. = not determined.**

| **Kinase** | **MW01 (10000nM)** | **MW01 (1000nM)** | **MW05 (10000nM)** | **MW05 (1000nM)** |
|---|---|---|---|---|
| ABL1 | 27 | 15 | 6 | 4 |
| ABL1 E255K | 11 | 7 | 5 | 6 |
| ABL1 F317I | 19 | 8 | -1 | 6 |
| ABL1 F317L | 13 | 8 | 4 | 6 |
| ABL1 G250E | 32 | 9 | 0 | 12 |
| ABL1 T315I | 34 | 9 | 5 | 8 |
| ABL1 Y253F | 30 | 9 | 7 | -2 |
| ABL2 (Arg) | 21 | 9 | 2 | 5 |
| ACVR1B (ALK4) | -3 | -9 | -8 | 1 |
| ADRBK1 (GRK2) | 17 | 8 | 4 | -1 |
| ADRBK2 (GRK3) | 5 | 0 | -3 | 2 |
| AKT1 (PKB alpha) | 10 | 5 | 3 | 3 |
| AKT2 (PKB beta) | 9 | 1 | -8 | 10 |
| AKT3 (PKB gamma) | 18 | 7 | -6 | 4 |
| ALK | 40 | 18 | -2 | 0 |
| AMPK (A1/B2/G2) | 5 | -6 | 4 | -10 |
| AMPK (A1/B2/G3) | 19 | 3 | -6 | 0 |
| AMPK (A2/B1/G2) | 10 | -12 | -16 | -12 |
| AMPK (A2/B1/G3) | 12 | -2 | -3 | 0 |
| AMPK (A2/B2/G3) | 18 | 8 | -4 | 4 |
| AMPK A1/B1/G1 | 14 | 4 | -3 | 6 |
| AMPKA2/B1/G1 | 32 | 8 | 0 | 5 |
| AURKA (Aurora A) | 18 | 8 | -5 | 7 |
| AURKB (Aurora B) | 19 | 15 | -6 | 8 |
| AURKC (Aurora C) | 10 | 8 | 9 | 9 |
| AXL | 80 | 51 | -8 | 4 |
| BLK | 26 | 8 | -1 | 0 |
| BMX | 6 | -8 | 4 | -10 |
| BRSK1 (SAD1) | 6 | 0 | -6 | -1 |
| BTK | 23 | 11 | 8 | 5 |
| CAMK1 (CaMK1) | -2 | -12 | -5 | -11 |
| CAMK1D (CaMKI delta) | 9 | 5 | -1 | 1 |
| CAMK2A (CaMKII alpha) | 39 | 12 | 16 | 9 |
| CAMK2B (CaMKII beta) | 37 | 9 | 3 | 5 |
| CAMK2D (CaMKII delta) | 60 | 13 | 13 | 3 |
| CAMK4 (CaMKIV) | 16 | -3 | -9 | 2 |
| CDC42 BPA (MRCKA) | 15 | 11 | 2 | 9 |
| CDC42 BPB (MRCKB) | 5 | 0 | -6 | 5 |
| CDC42 BPG (MRCKG) | 5 | 1 | 0 | 0 |
| CDK1/cyclin B | 46 | 20 | 57 | 19 |
| CDK17/cyclin Y | 9 | 3 | 21 | 8 |
| CDK18/cyclin Y | 15 | 5 | 55 | 13 |
| CDK2/cyclin A | 37 | 14 | 25 | 12 |
| CDK4/cyclin D1 | -4 | -8 | -1 | -3 |
| CDK4/cyclin D3 | -6 | -2 | -8 | -6 |
| CDK5/p25 | 12 | 4 | 8 | 4 |
| CDK5/p35 | 14 | 17 | 7 | 5 |
| CDK6/cyclin D1 | -8 | 1 | 0 | 2 |
| CDK7/cyclin H/MNAT1 | 4 | 3 | 29 | 2 |
| CDK9/cyclin T1 | 20 | 11 | 57 | 19 |
| CHEK1 (CHK1) | 13 | 4 | 27 | 16 |
| CHUK (IKK alpha) | 21 | 19 | 18 | 7 |
| CLK1 | 88 | n.d. | 73 | n.d. |
| CLK2 | 91 | 78 | 86 | 40 |
| CLK2 | 91 | 78 | 72 | 40 |
| CLK4 | 91 | n.d. | 94 | n.d. |
| CSF1R (FMS) | 51 | 36 | -6 | 5 |
| CSK | 18 | 11 | 13 | 9 |
| CSNK1A1 (CK1 alpha 1) | 17 | 5 | 1 | 2 |
| CSNK1A1L | 11 | 1 | 1 | 1 |
| CSNK1D (CK1 delta) | 39 | 13 | -1 | -1 |
| CSNK1E (CK1 epsilon) | 32 | 6 | 0 | 2 |
| CSNK1E (CK1 epsilon) R178C | 64 | 23 | 6 | 0 |
| CSNK1G1 (CK1 gamma 1) | 12 | 5 | 6 | 3 |
| CSNK1G2 (CK1 gamma 2) | 41 | 22 | 13 | 4 |
| CSNK1G3 (CK1 gamma 3) | 12 | 4 | 9 | 1 |
| CSNK2A1 (CK2 alpha 1) | 18 | 9 | 5 | 9 |
| CSNK2A2 (CK2 alpha 2) | 29 | 16 | 4 | 7 |
| DAPK1 | 14 | 8 | 3 | 3 |
| DAPK3 (ZIPK) | 9 | 2 | -3 | 2 |
| DNA-PK | 96 | 86 | 61 | 30 |
| DYRK1A | 90 | 80 | 60 | 22 |
| DYRK1B | 82 | 62 | 69 | 28 |
| DYRK3 | 43 | 17 | 7 | 5 |
| DYRK4 | 0 | -2 | 4 | 4 |
| EEF2K | 37 | 11 | 5 | 3 |
| EGFR (ErbB1) | 0 | 17 | 5 | 13 |
| EGFR (ErbB1) C797S | 11 | 4 | -14 | 1 |
| EGFR (ErbB1) G719S | 14 | 5 | -5 | 4 |
| EGFR (ErbB1) L858R | 5 | 3 | 16 | 6 |
| EGFR (ErbB1) L861Q | 8 | -9 | -18 | -6 |
| EGFR (ErbB1) T790M | 19 | 8 | 0 | 4 |
| EGFR (ErbB1) T790M C797S L858R | 12 | 2 | 2 | 1 |
| EGFR (ErbB1) T790M L858R | 29 | 9 | 10 | -5 |
| EPHA1 | 16 | 9 | -5 | 11 |
| EPHA2 | 14 | 6 | -15 | 13 |
| EPHA5 | 7 | -4 | -16 | -8 |
| EPHA8 | 21 | 4 | -9 | -1 |
| EPHB1 | 9 | -3 | -6 | -3 |
| EPHB2 | 17 | 10 | 1 | 6 |
| EPHB3 | 11 | 1 | 5 | 6 |
| EPHB4 | 7 | 5 | 2 | -3 |
| ERBB2 (HER2) | 4 | 2 | -19 | -5 |
| ERBB4 (HER4) | 4 | -3 | -11 | 5 |
| FER | 25 | 11 | 0 | 7 |
| FES (FPS) | 15 | 0 | -1 | 1 |
| FGFR1 | 5 | 6 | -7 | -1 |
| FGFR2 | 11 | 13 | -5 | 0 |
| FGFR2 N549H | 13 | 0 | -22 | -4 |
| FGFR3 | 8 | 4 | -8 | 1 |
| FGFR3 K650E | 16 | 6 | -3 | 8 |
| FGFR3 V555M | 11 | 5 | -9 | -30 |
| FGFR4 | 37 | 13 | -4 | 13 |
| FGR | 26 | 10 | 15 | 5 |
| FLT3 | 86 | 61 | 6 | 2 |
| FLT3 D835Y | 92 | 81 | 15 | 10 |
| FLT4 (VEGFR3) | 31 | 13 | -3 | 7 |
| FRAP1 (mTOR) | 80 | 31 | 81 | 31 |
| FRK (PTK5) | 22 | 15 | 6 | 9 |
| FYN | 15 | 3 | 5 | 4 |
| GRK4 | 12 | 8 | 2 | -1 |
| GRK5 | 0 | -3 | 0 | -2 |
| GRK6 | 5 | 0 | 3 | 6 |
| GRK7 | 0 | -2 | 5 | -2 |
| GSG2 (Haspin) | 83 | 77 | 20 | 4 |
| GSK3A (GSK3 alpha) | 55 | 25 | 18 | 3 |
| GSK3B (GSK3 beta) | 52 | 27 | 14 | 6 |
| HCK | 22 | 13 | 9 | 10 |
| HIPK1 (Myak) | 47 | 21 | -6 | -4 |
| HIPK2 | 74 | 48 | 13 | 4 |
| HIPK4 | 62 | 42 | 8 | 12 |
| IGF1R | 31 | 11 | -12 | -1 |
| IKBKB (IKK beta) | 12 | 4 | 5 | 6 |
| IKBKE (IKK epsilon) | 17 | 11 | 6 | 8 |
| INSR | 11 | 1 | -7 | 8 |
| IRAK1 | 67 | 57 | -7 | 17 |
| IRAK4 | 50 | 19 | 2 | 0 |
| ITK | -2 | 0 | -6 | -10 |
| JAK2 | 48 | 15 | -20 | -3 |
| JAK2 JH1 JH2 V617F | 29 | 15 | -28 | 1 |
| JAK3 | 20 | 9 | -6 | 4 |
| KDR (VEGFR2) | 54 | 24 | -15 | 7 |
| KIT T670I | 13 | 1 | -13 | -1 |
| KIT V559D | 25 | 11 | 15 | 13 |
| KIT V559D V654A | -12 | -10 | -37 | -16 |
| KIT V560G | 7 | 12 | 2 | 11 |
| KSR2 | 8 | 5 | -2 | 7 |
| LCK | 37 | 12 | 5 | 8 |
| LRRK2 | 87 | 83 | 5 | -3 |
| LRRK2 FL | 90 | 85 | 6 | -6 |
| LRRK2 G2019S | 93 | 90 | 16 | -2 |
| LRRK2 G2019S FL | 90 | 87 | 11 | -1 |
| LRRK2 I2020T | 90 | 83 | 5 | -3 |
| LRRK2 R1441C | 86 | 83 | 3 | -2 |
| LTK (TYK1) | 44 | 19 | -9 | 7 |
| LYN A | 18 | 5 | -6 | 1 |
| LYN B | 23 | 6 | 0 | 6 |
| MAP3K19 (YSK4) | 17 | 4 | -5 | -3 |
| MAP4K2 (GCK) | 77 | 50 | 10 | 13 |
| MAP4K4 (HGK) | 80 | 64 | 11 | 9 |
| MAP4K5 (KHS1) | 68 | 39 | 4 | 15 |
| MAPK1 (ERK2) | 9 | 4 | 7 | 5 |
| MAPK12 (p38 gamma) | 11 | 8 | 4 | 4 |
| MAPK13 (p38 delta) | 11 | 13 | 9 | 8 |
| MAPK3 (ERK1) | 4 | 14 | 11 | 14 |
| MAPK7 (ERK5) | 1 | 9 | -7 | 2 |
| MAPKAPK2 | 3 | 3 | -6 | 4 |
| MAPKAPK3 | 24 | 8 | 3 | 10 |
| MAPKAPK5 (PRAK) | 18 | 8 | 7 | 7 |
| MARK1 (MARK) | 6 | 4 | -5 | 3 |
| MARK2 | 5 | 10 | 5 | 3 |
| MARK3 | 7 | 0 | -5 | 3 |
| MARK4 | 15 | 9 | -10 | -3 |
| MATK (HYL) | 2 | 14 | 13 | 13 |
| MELK | 54 | 26 | 48 | 28 |
| MERTK (cMER) | 86 | 54 | 11 | 4 |
| MET (cMet) | 35 | 14 | -8 | 5 |
| MET (cMet) Y1235D | 4 | -2 | -44 | 1 |
| MET M1250T | 42 | 19 | -15 | 1 |
| MINK1 | 73 | 60 | 9 | 20 |
| MST1R (RON) | 39 | 22 | -16 | -1 |
| NEK1 | 58 | 23 | 6 | -1 |
| NEK2 | 0 | -4 | 1 | -3 |
| NEK6 | -4 | 4 | -4 | 1 |
| NEK9 | 9 | 4 | 5 | 6 |
| NIM1K | 13 | 7 | 2 | 7 |
| NTRK2 (TRKB) | 68 | 35 | 42 | 13 |
| NTRK3 (TRKC) | 66 | 42 | 11 | 10 |
| NUAK1 (ARK5) | 66 | 48 | 11 | 6 |
| PAK1 | 22 | 10 | 2 | 16 |
| PAK2 (PAK65) | 7 | 14 | 4 | 14 |
| PAK3 | 15 | 10 | 15 | 26 |
| PAK4 | 20 | 13 | 15 | 11 |
| PAK6 | -10 | 5 | 3 | 11 |
| PAK7 (KIAA1264) | 11 | 2 | 13 | 15 |
| PDGFRA (PDGFR alpha) | 47 | 20 | 6 | 7 |
| PDGFRA D842V | 35 | 3 | -2 | -4 |
| PEAK1 | 16 | -1 | 0 | 2 |
| PHKG1 | 12 | 8 | -11 | 3 |
| PHKG2 | 8 | 6 | -3 | 6 |
| PI4K2A (PI4K2 alpha) | 25 | 7 | -1 | -7 |
| PI4K2B (PI4K2 beta) | 20 | 3 | 0 | 9 |
| PI4KA (PI4K alpha) | 23 | 13 | -1 | 1 |
| PI4KB (PI4K beta) | 100 | 94 | 43 | -2 |
| PIK3C2A (PI3K-C2 alpha) | 43 | 26 | 1 | -5 |
| PIK3C2B (PI3K-C2 beta) | 68 | 53 | 12 | 2 |
| PIK3C2G (PI3K-C2 gamma) | 94 | 92 | 87 | 56 |
| PIK3C3 (hVPS34) | 49 | 23 | 4 | 4 |
| PIK3CA E542K/PIK3R1 (p11 0 alpha E542K/p85 alpha) | 96 | 94 | 68 | 22 |
| PIK3CA E545K/PIK3R1 (p11 0 alpha E545K/p85 alpha) | 97 | 91 | 61 | 15 |
| PIK3CA/PIK3R1 (p11 0 alpha/p85 alpha) | 86 | 81 | 52 | 14 |
| PIK3CA/PIK3R3 (p11 0 alpha/p55 gamma) | 96 | 92 | 63 | 16 |
| PIK3CB/PIK3R1 (p110 beta/p85 alpha) | 67 | 30 | 67 | 21 |
| PIK3CB/PIK3R2 (p11 0 beta/p85 beta) | 66 | 24 | 64 | 15 |
| PIK3CD/PIK3R1 (p11 0 delta/p85 alpha) | 89 | 79 | 77 | 40 |
| PIK3CG (p11 0 gamma) | 88 | 81 | 70 | 20 |
| PIM1 | 24 | 11 | -9 | 8 |
| PIM2 | 13 | 6 | -3 | -1 |
| PIM3 | 42 | 22 | 2 | 11 |
| PIP4K2A | -3 | -8 | -9 | -10 |
| PIP5K1A | 43 | 25 | 5 | -6 |
| PIP5K1B | 21 | 4 | 7 | -13 |
| PIP5K1C | 69 | 54 | 26 | -1 |
| PKN1 (PRK1) | 16 | 9 | 4 | 15 |
| PLK1 | 3 | 5 | 6 | 6 |
| PLK2 | -2 | -1 | -2 | 3 |
| PLK3 | 23 | 6 | 12 | 5 |
| PRKACA (PKA) | 17 | 6 | -15 | 12 |
| PRKCA (PKC alpha) | 18 | 11 | -1 | 4 |
| PRKCB1 (PKC beta I) | -17 | 1 | -7 | 5 |
| PRKCB2 (PKC beta II) | 14 | 11 | -5 | 9 |
| PRKCD (PKC delta) | 17 | 6 | 0 | 14 |
| PRKCE (PKC epsilon) | 8 | 9 | -12 | 6 |
| PRKCG (PKC gamma) | 16 | 13 | -3 | 11 |
| PRKCH (PKC eta) | 7 | 1 | -9 | -3 |
| PRKCI (PKC iota) | -8 | 4 | 6 | 20 |
| PRKCN (PKD3) | 57 | 34 | 15 | 17 |
| PRKCQ (PKC theta) | 21 | 4 | -15 | 2 |
| PRKCZ (PKC zeta) | 10 | 18 | -4 | 16 |
| PRKD1 (PKC mu) | 49 | 28 | 9 | 5 |
| PRKD2 (PKD2) | 71 | 38 | 10 | 11 |
| PRKG1 | 59 | 27 | -11 | 5 |
| PRKG2 (PKG2) | 72 | 47 | 0 | 7 |
| PRKX | 16 | 22 | -2 | 8 |
| PTK2B (FAK2) | 9 | 2 | -7 | -2 |
| RET | 34 | 15 | 12 | 9 |
| RET A883F | 24 | 10 | 3 | 7 |
| RET S891A | 30 | -3 | -18 | -15 |
| RET V804E | 17 | 5 | -7 | 1 |
| RET V804L | 38 | 15 | 0 | 2 |
| RET Y791F | 32 | 17 | 7 | 5 |
| ROCK1 | 13 | 0 | -5 | -4 |
| ROCK2 | 18 | 14 | 4 | 14 |
| RPS6KA1 (RSK1) | 19 | 12 | 3 | 10 |
| RPS6KA2 (RSK3) | 11 | 6 | 6 | 5 |
| RPS6KA3 (RSK2) | 10 | 5 | 2 | 3 |
| RPS6KA4 (MSK2) | 24 | 12 | 4 | -3 |
| RPS6KA5 (MSK1) | 25 | 5 | -14 | 2 |
| RPS6KA6 (RSK4) | 17 | 5 | 4 | 2 |
| RPS6KB1 (p70S6K) | 15 | 19 | 7 | 12 |
| RPS6KB2 (p70S6Kb) | 1 | 7 | 15 | 3 |
| SBK1 | 3 | 3 | -26 | -7 |
| SGK (SGK1) | 15 | -4 | -12 | -2 |
| SGK2 | 14 | 6 | -11 | -3 |
| SGKL (SGK3) | 10 | 12 | 6 | 8 |
| SNF1LK2 | 12 | 5 | -2 | 4 |
| SPHK1 | -3 | -7 | 1 | -2 |
| SPHK2 | -8 | -16 | -4 | -7 |
| SRC | 16 | 0 | 3 | 6 |
| SRC N1 | 25 | 15 | 14 | 16 |
| SRPK1 | 15 | 12 | 10 | 8 |
| SRPK2 | 0 | -2 | 10 | 4 |
| STK22B (TSSK2) | 20 | 11 | 2 | 11 |
| STK22D (TSSK1) | 22 | 11 | 5 | 2 |
| STK25 (YSK1) | -3 | -9 | -11 | -3 |
| STK4 (MST1) | 11 | 4 | 3 | 4 |
| SYK | 37 | 16 | 7 | 5 |
| TBK1 | 17 | 8 | -8 | 11 |
| TEK (Tie2) | 5 | 19 | 12 | -2 |
| TEK (TIE2) Y897S | -10 | -14 | -11 | -18 |
| TXK | 29 | 12 | -13 | 4 |
| TYRO3 (RSE) | 29 | 11 | 2 | 7 |
| YES1 | 16 | 17 | 20 | 15 |
| ZAP70 | 16 | 12 | 11 | 15 |

### CLK2 and CLK4 are identified as the molecular targets shared by MVV01 and MW05

To confirm kinase inhibition by MW01 and MW05 in our cell-based assays and to identify their shared kinases involved in DNA-damage pathway-specific NF-κB inhibition, we tested the NF-κB pathway pharmacologically using commercially available PI3K and CDC-like kinase inhibitors. Putative target kinase IC50 data of our lead compounds were compared with IC50 data of commercially available inhibitors, and we selected PI-103, NU-7441, and MU-1210 as PI3K, DNA-PK, and CLK inhibitors, respectively, based on similar or lower IC50s for the shared target kinases of MW01 and MW05 (Leahy et al., 2004; Němec et al., 2019; Raynaud et al., 2007). In addition, PI-103 also inhibits DNA-PK and mTOR with low nanomolar IC50s (Raynaud et al., 2007), and therefore, this compound can be used to test if PI3K, DNA-PK, or mTOR are involved in NF-κB activation. Based on the low nanomolar PI3K IC50s of MW01 and PI-103, we observed a complete reduction of p-Akt after 1-hour pre-treatment by either compound, with MW05 producing a partial reduction in agreement with its comparatively higher PI3K IC50s (Fig 2C) (Raynaud et al., 2007). Despite a previously suggested link between PI3K/Akt/mTOR and NF-κB (Reddy et al., 2000), we did not observe any reduction of p-p65 following either DNA damage or TNFα stimulation and were unable to confirm these findings within our context. In addition, DNA-PK inhibitor NU-7441 did not reduce p-p65 following either stimulation, suggesting that it does not play a direct role in regulating DNA damage-induced NF-κB activity. We observed a reduction in p65 phosphorylation by MW01, MW05, and interestingly, CLK inhibitor MU-1210, but not its negative control MU140 (Moyano et al., 2020), only after DNA damage, suggesting experimentally for the first time that these kinases regulate irradiation-induced NF-κB activity (Fig 2C). To investigate the role of CLK2 and CLK4 in regulating DNA damage-induced NF-κB activity, siRNAs were used to knock down CLK2, monitoring any change in NF-κB activation. Recapitulating the effect of MW01 and MW05, CLK2 or CLK4 knockdown produced a significant irradiation-specific reduction in NFKBIA expression and p-p65 level, while leaving unstimulated and TNF-stimulated activation unchanged compared to the negative control siNC (2D, 2E, 2G, 7E). Furthermore, we determined potency and isoform specificity for all four CLKs for both MW01 and MW05, finding that MW01 is a CLK1, CLK2, and CLK4 inhibitor and MW05 is displays higher selectivity toward CLK4, while both compounds largely spare CLK3 (Fig. 2F). Importantly, with the CLK2 IC50 of 360 nM for MW01 and of 1,440 nM for MW05, we expect sufficient levels of CLK2 and CLK4 inhibition at 10 µM in cell-based assays. In addition, we performed similar knockdown experiments targeting CLK4 and confirmed a significant reduction of p-p65 and NFKBIA expression following DNA damage. (Fig 7E, F). Furthermore, knockdown of CLK2 or CLK4 produced a strong reduction in IKKy-S85 phosphorylation, mirroring the ablation of this DNA damage-specific, ATM dependent IKKy PTM by MW01 and MW05, and localizing CLKs upstream of IKK (Fig. 2E, Fig 7A). Taken together, these data suggest that CLK2 and 4 are the functional targets of MW01 and MW05.

### CLKs are the shared targets of active derivatives of MW01 and MW05

To validate the structural backbones of MW01 and MW05 and leverage additional structures in discerning on- and off-target activity, we screened more than 30 structural derivatives for similar NF-κB pathway inhibition compared to our lead compounds following irradiation or TNF stimulation (Table 1 and 2). Several structural derivatives of each lead compound were identified which reduced p-p65 and NFKBIA expression in an irradiation-specific manner. (Fig 3A, B, C, Fig. 8). We then selected four compounds for an in vitro kinase inhibition assay panel containing all kinases that were strongly inhibited by MW01 and MW05 (Table 7). As expected for structurally similar derivatives, kinase inhibition profiles of active derivatives were similar to the respective lead compounds, validating the common structural backbones of each as kinase inhibitors. However, inhibition of several kinases was lost some derivatives of MW01 and MW05, including PI3K isoforms, adding further evidence that the NF-κB inhibition by our compounds is not mediated by inhibition of these kinases. Crucially, all compounds inhibited CLK2 mostly with IC50 values of less than 2000nM and CLK with IC50 values less than 300 nM (Fig 3D, Table 7), in agreement with our kinase panel criteria (Fig 3D). The compound MW05-E2 inhibited CLK2 despite lacking activity in cell-based NF-κB activation assays, likely due to poor cellular uptake, an issue absent under in vitro kinase assay conditions (Table 7). Moderate CLK4 inhibition was noted for three of four inactive derivatives while one, MW05-E10, inhibited CLK4 comparably to the active compounds. However, none of the inactive compounds share an CLK2 and 4 inhibition profile comparable to the leads or their active derivatives. Considering the phenotypic similarity between two structurally distinct lead compounds, the agreement of the derivatives with our kinase IC50 criteria, and significant results following siRNA knockdown of CLK2 and CLK4, we proceeded to further characterize our lead compounds.

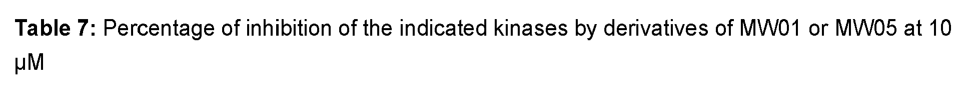

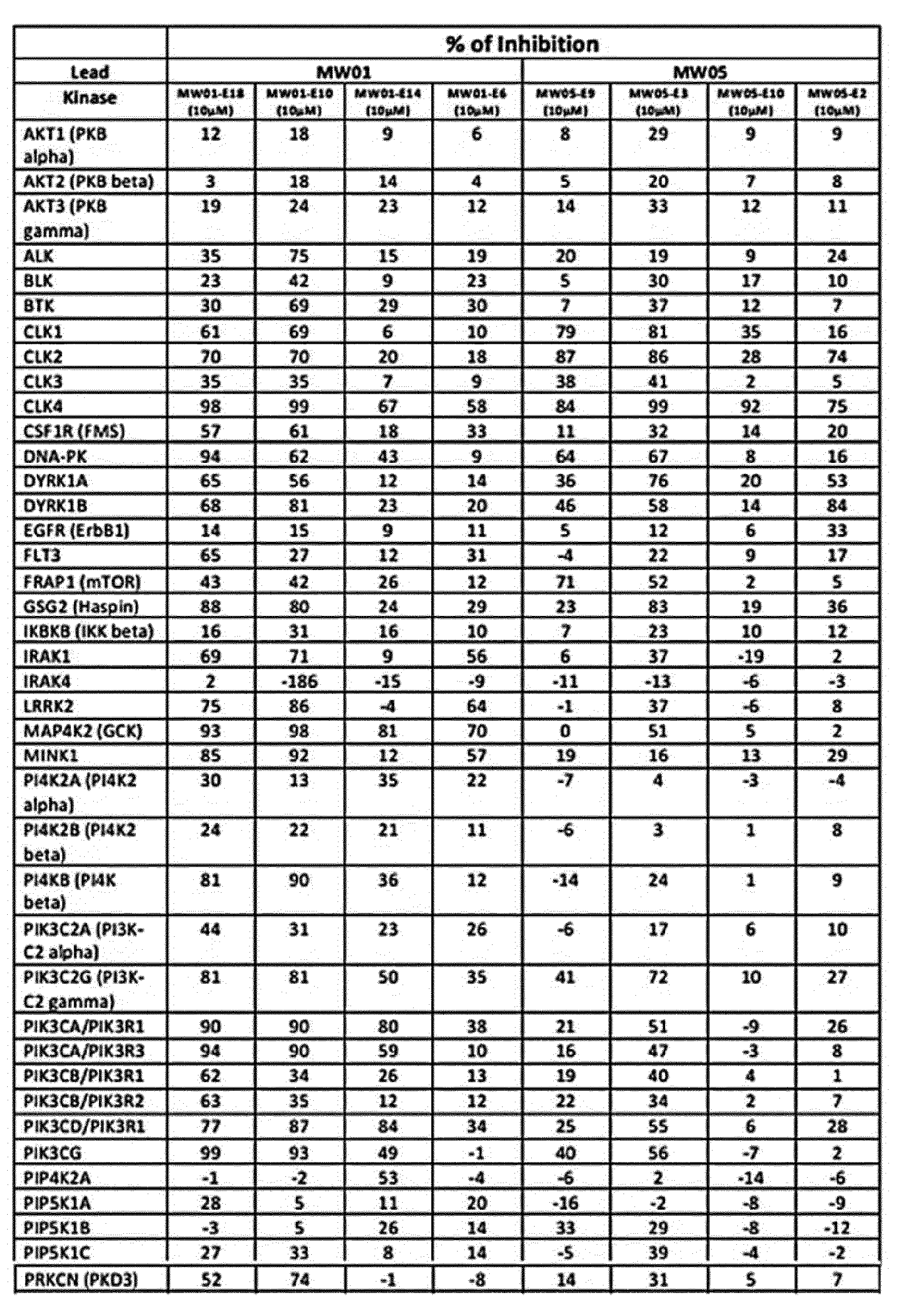

### MW01 and MW05 reduce cell viability and induce cell cycle arrest and potentiate cell killing effects of chemotherapeutic agents

We hypothesized that due to the important function of the DNA double strand-induced IKK-NF-κB pathway as in anti-apoptotic signalling (Stilmann et al., 2009), both MW01 and MW05 should sensitize cells to DNA damaging agents, and therefore support their potential use to potentiate genotoxic therapies. To characterize the effect of MW01 and MW05 on cell viability, we treated cells with both compounds alone or in combination with etoposide to induce DNA lesions (Fig. 4A). Both, MW01 and MW05 alone slightly reduced cell viability at 6, 24, and 48 hours compared to DMSO- treated controls (Fig 4A, B). Importantly, at 48 hours MW01 or MW05 treated cells did not display any obvious characteristics of cell death, suggesting that the compounds alone are not grossly toxic. MW01 and etoposide co-treatment significantly reduced cell viability after 6 hours, while MW05 and etoposide co-treatment yielded a significant reduction after 24 hours, compared to etoposide alone (Fig. 4B). Interestingly, 24 hours after co-treatment with either MW01 or MW05 and etoposide, attached cells were visibly distressed and the number of floating cells was drastically increased compared to etoposide alone (Fig 4A). This effect was greatly increased at 48 hours, with relatively fewer attached cells and more floating cells, indicating cell death. The same effect in cell viability reduction was confirmed for MW01 and MW05 in co-treatment with cisplatin, another widely used chemotherapeutic drug. To investigate the underlying molecular causes of this phenotype, we looked at markers of NF-κB and p53 activation, DNA damage, and apoptosis after single and co-treatment with MW01, MW05, and etoposide (Fig 4C). MW01 and MW05 abrogated etoposide-stimulated p65 phosphorylation, thereby validating our inhibition of IKK and NF-κB activity. At 24 hours, a weak activation of γH2AX in MW01 treatment alone was apparent compared to DMSO-treated controls. Moreover, γH2AX activation was persistent in MW01 and etoposide co-treatment as well as in MW05 and etoposide co-treatment compared to DMSO, indicating an accumulation of DNA damage. We did not observe an increase in cleaved caspase-3 in MW01 or MW05 treatment alone at 24 hours. Notably, this apoptosis marker was increased following MW01 and etoposide co-treatment as well as after MW05 and etoposide co-treatment. Interestingly, we also observed a stabilization in p53 following MW01 treatment alone and in co-treatment of both lead compounds with etoposide compared to DMSO. We suspect the disparity in γH2AX, p53, and cleaved caspase-3 levels between MW01 and MW05 in etoposide co-treatment is due to MW01 co-treated cells more quickly undergoing p53- mediated cell death compared to MW05. Thus, in the co-treatment at 24 hours, γH2AX levels were comparatively lower and cleaved caspase-3 higher for MW01 than MW05 because the apoptotic cells are no longer repairing DNA damage. Taken together with the cell viability and image data, these data indicate that MW01 and MW05 are promising lead compounds for cancer treatment, reducing the expression of pro-survival NF-κB target genes and increasing the expression of pro-apoptotic p53 target genes in co-administration with chemo- or radiotherapy.

### REFERENCES

Antolin, A.A., Ameratunga, M., Banerji, U., Clarke, P.A., Workman, P., and Al-Lazikani, B. (2020). The kinase polypharmacology landscape of clinical PARP inhibitors. Scientific Reports 10.
Antonia, R.J., Hagan, R.S., and Baldwin, A.S. (2021). Expanding the View of IKK: New Substrates and New Biology. Trends in Cell Biology 31, 166-178.
Baud, V., and Karin, M. (2009). Is NF-κB a good target for cancer therapy? Hopes and pitfalls. Nature Reviews Drug Discovery 8, 33-40.
Cohen, P., Cross, D., and Jänne, P.A. (2021). Kinase drug discovery 20 years after imatinib: progress and future directions. Nature Reviews Drug Discovery 20, 551-569.
De Rooij, M.F.M., Kuil, A., Geest, C.R., Eldering, E., Chang, B.Y., Buggy, J.J., Pals, S.T., and Spaargaren, M. (2012). The clinically active BTK inhibitor PCI-32765 targets B-cell receptor- and chemokinecontrolled adhesion and migration in chronic lymphocytic leukemia. Blood 119, 2590-2594.
Ellis, L.M., and Hicklin, D.J. (2009). Resistance to Targeted Therapies: Refining Anticancer Therapy in the Era of Molecular Oncology. Clinical Cancer Research 15, 7471-7478.
Gilmore, T.D., and Herscovitch, M. (2006). Inhibitors of NF-κB signaling: 785 and counting. Oncogene 25, 6887-6899.
Hanahan, D. & Weinberg, R. A. Hallmarks of cancer: the next generation. Cell 144, 646-674, (2011).
Hayden, M.S., and Ghosh, S. (2008). Shared Principles in NF-κB Signaling. Cell 132, 344-362.
Hinz, M., and Scheidereit, C. (2014). The IκB kinase complex in <scp>NF</scp> -κB regulation and beyond. EMBO reports 15, 46-61.
Hinz, M., Stilmann, M., Arslan, S.C., Khanna, K.K., Dittmar, G., and Scheidereit, C. (2010). A cytoplasmic ATM-TRAF6-clAP1 module links nuclear DNA damage signaling to ubiquitin-mediated NF-kappaB activation. Mol Cell 40, 63-74.
Huang, T.T., Wuerzberger-Davis, S.M., Wu, Z.-H., and Miyamoto, S. (2003). Sequential Modification of NEMO/IKKγ by SUMO-1 and Ubiquitin Mediates NF-κB Activation by Genotoxic Stress. Cell 115, 565-576.
Hunter, J.E., Willmore, E., Irving, J.A.E., Hostomsky, Z., Veuger, S.J., and Durkacz, B.W. (2012). NF-κB mediates radio-sensitization by the PARP-1 inhibitor, AG-014699. Oncogene 31, 251-264.
Iwai, K., Yaguchi, M., Nishimura, K., Yamamoto, Y., Tamura, T., Nakata, D., Dairiki, R., Kawakita, Y., Mizojiri, R., Ito, Y., et al. (2018). Anti-tumor efficacy of a novel CLK inhibitor via targeting RNA splicing and MYC-dependent vulnerability. EMBO Mol Med 10.
Karin, M., and Greten, F.R. (2005). NF-κB: linking inflammation and immunity to cancer development and progression. Nature Reviews Immunology 5, 749-759.
Kolesnichenko, M., Mikuda, N., Höpken, U.E., Kärgel, E., Uyar, B., Tufan, A.B., Milanovic, M., Sun, W., Krahn, I., Schleich, K., et al. (2021). Transcriptional repression of NFKBIA triggers constitutive IKK- and proteasome-independent p65/ReIA activation in senescence. Embo j 40, e104296.
Labrie, M., Brugge, J.S., Mills, G.B., and Zervantonakis, I.K. (2022). Therapy resistance: opportunities created by adaptive responses to targeted therapies in cancer. Nature Reviews Cancer 22, 323-339.
Leahy, J.J., Golding, B.T., Griffin, R.J., Hardcastle, I.R., Richardson, C., Rigoreau, L., and Smith, G.C. (2004). Identification of a highly potent and selective DNA-dependent protein kinase (DNA-PK) inhibitor (NU7441) by screening of chromenone libraries. Bioorg Med Chem Lett 14, 6083-6087.
Li, N., Banin, S., Ouyang, H., Li, G.C., Courtois, G., Shiloh, Y., Karin, M., and Rotman, G. (2001). ATM Is Required for IκB Kinase (IKK) Activation in Response to DNA Double Strand Breaks*. Journal of Biological Chemistry 276, 8898-8903.
Lin, J., Lin, G., Chen, B., Yuan, J., and Zhuang, Y. (2022). CLK2 Expression Is Associated with the Progression of Colorectal Cancer and Is a Prognostic Biomarker. Biomed Res Int 2022, 7250127.
Liu, B., Kong, X., Wang, R., and Xin, C. (2021). CLK2 promotes occurrence and development of nonsmall cell lung cancer. J buon 26, 58-64.
Mabb, A.M., Wuerzberger-Davis, S.M., and Miyamoto, S. (2006). PIASy mediates NEMO sumoylation and NF-κB activation in response to genotoxic stress. Nature Cell Biology 8, 986-993.
Martin Moyano, P., Nemec, V., and Paruch, K. (2020). Cdc-Like Kinases (CLKs): Biology, Chemical Probes, and Therapeutic Potential. Int J Mol Sci 21. 10.3390/ijms21207549.
Mikuda, N., Kolesnichenko, M., Beaudette, P., Popp, O., Uyar, B., Sun, W., Tufan, A.B., Perder, B., Akalin, A., Chen, W., et al. (2018). The IκB kinase complex is a regulator of <scp>mRNA</scp> stability. The EMBO Journal 37, e98658.
Nakanishi, C., and Toi, M. (2005). Nuclear factor-κB inhibitors as sensitizers to anticancer drugs. Nature Reviews Cancer 5, 297-309.
Němec, V., Hylsová, M., Maier, L., Flegel, J., Sievers, S., Ziegler, S., Schröder, M., Berger, B.T., Chaikuad, A., Valčíková, B., et al. (2019). Furo[3,2-b]pyridine: A Privileged Scaffold for Highly Selective Kinase Inhibitors and Effective Modulators of the Hedgehog Pathway. Angew Chem Int Ed Engl 58, 1062-1066.
Oeckinghaus, A., Hayden, M.S., and Ghosh, S. (2011). Crosstalk in NF-κB signaling pathways. Nature Immunology 12, 695-708.
Park, S.Y., Piao, Y., Thomas, C., Fuller, G.N., and de Groot, J.F. (2016). Cdc2-like kinase 2 is a key regulator of the cell cycle via FOXO3a/p27 in glioblastoma. Oncotarget 7, 26793-26805.
Piret, B., Schoonbroodt, S., and Piette, J. (1999). The ATM protein is required for sustained activation of NF-κB following DNA damage. Oncogene 18, 2261-2271.
Rayet, B., and Gélinas, C. (1999). Aberrant rel/nfkb genes and activity in human cancer. Oncogene 18, 6938-6947.
Raynaud, F.I., Eccles, S., Clarke, P.A., Hayes, A., Nutley, B., Alix, S., Henley, A., Di-Stefano, F., Ahmad, Z., Guillard, S., et al. (2007). Pharmacologic characterization of a potent inhibitor of class I phosphatidylinositide 3-kinases. Cancer Res 67, 5840-5850.
Reddy, S.A.G., Huang, J.H., and Liao, W.S.L. (2000). Phosphatidylinositol 3-Kinase as a Mediator of TNFlnduced NF-κB Activation. The Journal of Immunology 164, 1355-1363.
Riggs, J.R., Nagy, M., Elsner, J., Erdman, P., Cashion, D., Robinson, D., Harris, R., Huang, D., Tehrani, L., Deyanat-Yazdi, G., et al. (2017). The Discovery of a Dual TTK Protein Kinase/CDC2-Like Kinase (CLK2) Inhibitor for the Treatment of Triple Negative Breast Cancer Initiated from a Phenotypic Screen. J Med Chem 60, 8989-9002.
Salvador, F., and Gomis, R.R. (2018). CLK2 blockade modulates alternative splicing compromising MYC-driven breast tumors. EMBO Mol Med 10.
Scheidereit, C. (2006). IkappaB kinase complexes: gateways to NF-kappaB activation and transcription. Oncogene 25, 6685-6705.
Stilmann, M., Hinz, M., Arslan, S.C., Zimmer, A., Schreiber, V., and Scheidereit, C. (2009). A nuclear poly(ADP-ribose)-dependent signalosome confers DNA damage-induced IkappaB kinase activation. Mol Cell 36, 365-378.
Taniguchi, K., and Karin, M. (2018). NF-κB, inflammation, immunity and cancer: coming of age. Nature Reviews Immunology 18, 309-324.
Tergaonkar, V., Pando, M., Vafa, O., Wahl, G., and Verma, I. (2002). p53 stabilization is decreased upon NFκB activation. Cancer Cell 1, 493-503.
Tufan, A.B., Lazarow, K., Kolesnichenko, M., Sporbert, A., von Kries, J.P., and Scheidereit, C. (2022). TSG101 Associates with PARP1 and is Essential for PARylation and DNA Damage-induced NF-κB Activation. The EMBO Journal. 41: e110372 doi: 10.15252/embj.2021110372.
Uhlén, M., Fagerberg, L., Hallström, B.M., Lindskog, C., Oksvold, P., Mardinoglu, A., Sivertsson, Å., Kampf, C., Sjöstedt, E., Asplund, A., et al. (2015). Proteomics. Tissue-based map of the human proteome. Science 347, 1260419.
Wang, M.L., Rule, S., Martin, P., Goy, A., Auer, R., Kahl, B.S., Jurczak, W., Advani, R.H., Romaguera, J.E., Williams, M.E., et al. (2013). Targeting BTK with Ibrutinib in Relapsed or Refractory Mantle-Cell Lymphoma. New England Journal of Medicine 369, 507-516.
Wu, Z.-H., Shi, Y., Tibbetts, R.S., and Miyamoto, S. (2006). Molecular Linkage Between the Kinase ATM and NF-κB Signaling in Response to Genotoxic Stimuli. Science 311, 1141-1146.
Yoshida, T., Kim, J.H., Carver, K., Su, Y., Weremowicz, S., Mulvey, L., Yamamoto, S., Brennan, C., Mei, S., Long, H., et al. (2015). CLK2 Is an Oncogenic Kinase and Splicing Regulator in Breast Cancer. Cancer Res 75, 1516-1526.
Sen'ko, O.A., Dybenko, A.G., Garazd, M.M., and Kartsev, V.G. (2017). Synthesis of Benzoannelated Canthin-6-One Analogs. Chemistry of Natural Compounds 53, 523-528. 10.1007/s10600-017-2037-9. 44.
Lapa, G.B., Bekker, O.B., Mirchink, E.P., Danilenko, V.N., and Preobrazhenskaya, M.N. (2013). Regioselective acylation of congeners of 3-amino-1H-pyrazolo[3,4-b]quinolines, their activity on bacterial serine/threonine protein kinases and in vitro antibacterial (including antimycobacterial) activity. J Enzyme Inhib Med Chem 28, 1088-1093. 10.3109/14756366.2012.716056.

## Claims

1. Compound according to **Formula I** for use in the treatment of a cancer exhibiting genotoxic stress-induced IKK/NF-κB activation, 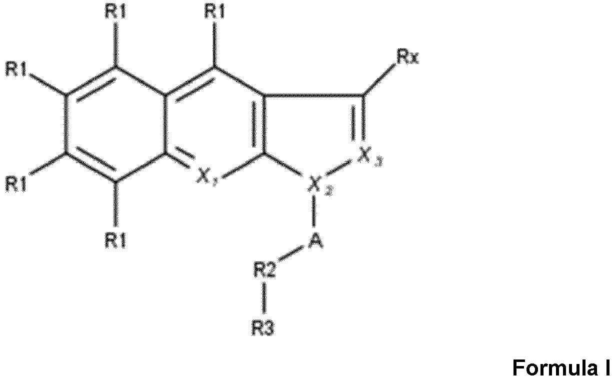 wherein
**R1** = can be the same or different, H, OH, halogen (preferably Br, Cl or F), alkyl (preferably C1 to C6 alkyl), alkenyl, alkynyl, alkoxy (preferably OCH₃, OCH₂CH₃), alkyl carbonyl, carboxyl, alkoxy carbonyl, amine (preferably primary, secondary or tertiary amine),
**Rx** = amine (preferably primary, secondary or tertiary amine), OH, H, alkyl (preferably C1 to C6 alkyl),
**X1, X2, X3** = can be the same or different, N or C,
**A=** alkyl (preferably C1 to C6 alkyl), alkenyl, alkynyl, alkoxy, carbonyl, carboxyl, cycloalkyl (preferably C3-C6 cycloalkyl), aryl (preferably phenyl), sulfonyl
**R2** = alkyl (preferably C1 to C6-alkyl), alkenyl, alkynyl, alkoxy, carbonyl, carboxyl, alkyl carbonyl, alkoxy carbonyl, amine (preferably primary, secondary or tertiary amine), aryl (preferably phenyl or naphtyl), C3 to C8 cycloalkyl, a 5 or 6-membered heterocyclyl or heteroaryl comprising one or more of N, O and/or S, and
**R3** = is absent, H, halogen (preferably Cl, Br, F), alkyl (preferably C1 to C6 alkyl), haloalkyl, OH, alkoxy (preferably OCH₃, OCH₂CH₃), alkoxy carbonyl (preferably - COOCH₃, - CH₂COOCH₃, -CH₂CH₂COOCH₃), cycloalkyl (preferably C3 to C6 cycloalkyl), aryl (preferably phenyl), alkyl-cycloalkyl, C1 to C6 alkyl-aryl, heterocyclyl, heteroaryl, carboxamide, CO-Cx alkyl carboxamide (preferably CON(CH₂CH₂)₂O); amine (preferably primary, secondary or tertiary amine), carbamate, nitro, -CN.

2. Compound according to the preceding claim, according to **Formula II** 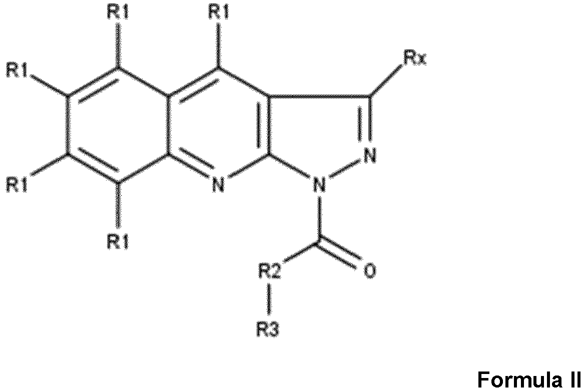 wherein
**R1** = can be the same or different, H, OH, halogen (preferably Br, Cl or F), alkyl (preferably C1 to C3 alkyl), alkenyl, alkynyl, alkoxy (preferably OCH₃, OCH₂CH₃), alkyl carbonyl, carboxyl, alkoxy carbonyl, amine (preferably primary, secondary or tertiary amine),
**Rx** = amine (preferably primary, secondary or tertiary amine), OH, H, alkyl (preferably C1 to C3 alkyl),
**R2** = alkyl (preferably C1 to C3-alkyl), alkenyl, alkynyl, alkoxy, carbonyl, carboxyl, alkyl carbonyl alkoxy carbonyl, amine (preferably primary, secondary or tertiary amine), aryl (preferably phenyl or naphtyl), C3 to C8-cycloalkyl, a 5- or 6-membered heterocyclyl or heteroaryl comprising one or more of N, O and/or S, and
**R3** = H, halogen (preferably Cl, Br, F), alkyl (preferably C1 to C3 alkyl), haloalkyl, OH, alkoxy (preferably OCH₃, OCH₂CH₃), alkoxy carbonyl (preferably - COOCH₃, -CH₂COOCH₃, - CH₂CH₂COOCH₃), cycloalkyl (preferably C3 to C6 cycloalkyl), aryl (preferably phenyl), alkyl-cycloalkyl, C1 to C6 alkyl-aryl, heterocyclyl, heteroaryl, carboxamide, CO-Cx alkyl carboxamide (preferably CON(CH₂CH₂)₂); amine (preferably primary, secondary or tertiary amine), carbamate, nitro, -CN.

3. Compound according to any one of the preceding claims, according to **Formula III** 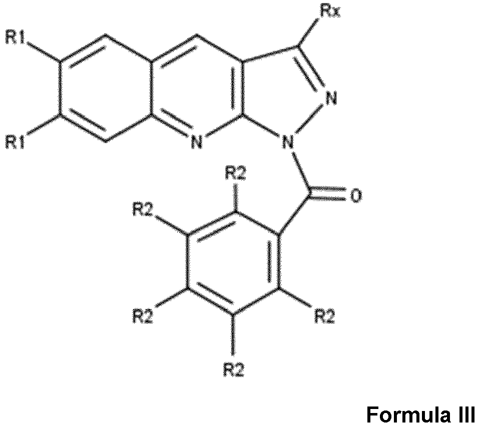 wherein
**R1** = can be the same or different, H, OH, halogen (preferably Br, Cl or F), alkyl (preferably C1 to C3 alkyl), alkenyl, alkynyl, alkoxy, alkyl carbonyl, carboxyl, alkoxy carbonyl, amine (preferably primary, secondary or tertiary amine),
**Rx** = amine (preferably primary, secondary or tertiary amine), OH, H, alkyl (preferably C1 to C3 alkyl), and
**R2** = can be the same or different, halogen (preferably Cl, Br, F), alkyl (preferably C1 to C6 alkyl), haloalkyl, OH, alkoxy (preferably OCH₃, OCH₂CH₃), alkoxy carbonyl (preferably - COOCH₃, -CH₂COOCH₃, -CH₂CH₂COOCH₃), cycloalkyl (preferably C3 to C6 cycloalkyl), aryl (preferably phenyl), alkyl-cycloalkyl, C1 to C5 alkyl-aryl, heterocyclyl, heteroaryl, carboxamide, CO-Cx alkyl carboxamide (preferably CON(CH₂CH₂)₂O); amine (preferably primary, secondary or tertiary amine), carbamate, nitro, -CN, O.

4. Compound according to any one of the preceding claims, according to **Formula III** 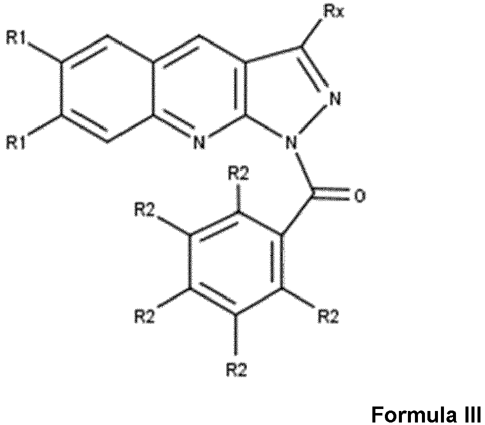 wherein
**R1** = can be the same or different, H, OH, halogen (preferably Br, Cl or F), alkoxy, amine (preferably primary, secondary or tertiary amine),
**Rx** = amine (preferably primary, secondary or tertiary amine), OH, H, alkyl (preferably C1 to C3 alkyl), and
**R2** = can be the same or different, H, alkoxy (preferably OCH₃, OCH₂CH₃), cycloalkyl (preferably C3 to C6 cycloalkyl), aryl (preferably phenyl), alkyl-cycloalkyl, heterocyclyl, heteroaryl, amine (preferably primary, secondary or tertiary amine), carboxamide,

5. Compound according to the preceding claim, according to **Formula IV** 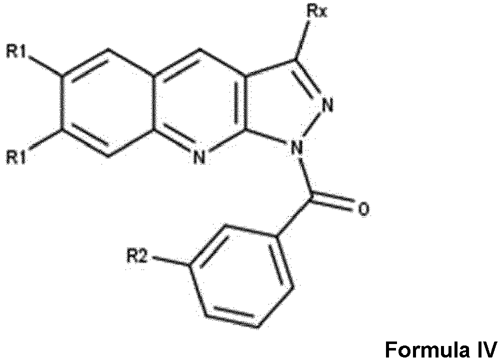 wherein
**R1** = can be the same or different, H, OH, halogen (preferably Br, Cl or F), alkyl (preferably C1 to C3 alkyl), alkenyl, alkynyl, alkoxy, alkyl carbonyl, carboxyl, alkoxy carbonyl, amine (preferably primary, secondary or tertiary amine),
**Rx** = amine (preferably primary, secondary or tertiary amine), H, OH, alkyl (preferably C1 to C3 alkyl), and
**R2** = halogen (preferably Cl, Br, F), alkyl (preferably C1 to C6 alkyl), haloalkyl, OH, alkoxy (preferably OCH₃, OCH₂CH₃), alkoxy carbonyl (preferably - COOCH₃, -CH₂COOCH₃,-CH₂CH₂COOCH₃), cycloalkyl (preferably C3 to C6 cycloalkyl), aryl (preferably phenyl), alkyl-cycloalkyl, C1 to C3 alkyl-aryl, heterocyclyl, heteroaryl, carboxamide, CO-Cx alkyl carboxamide (preferably CON(CH₂CH₂)₂O); amine (preferably primary, secondary or tertiary amine), carbamate, nitro, -CN, O.

6. Compound according to any one of the preceding claims, according to **Formula V** 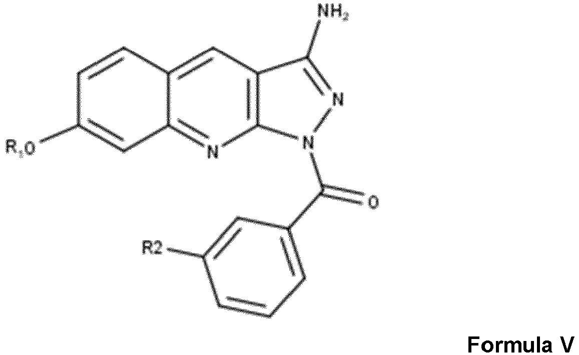 wherein
**R1=** H, alkyl (preferably C1 to C6 alkyl), alkenyl, alkynyl, alkoxy, carbonyl, carboxyl, alkoxy carbonyl, carboxamide, CO-Cx alkyl carboxamide (preferably CON(CH₂CH₂)₂O), and
**R2** = H, alkyl (preferably C1 to C6 alkyl), OH, alkoxy (preferably OCH₃, OCH₂CH₃), cycloalkyl (preferably C3 to C6 cycloalkyl), aryl (preferably phenyl), alkyl-cycloalkyl, heterocyclyl, heteroaryl, amine (preferably primary, secondary or tertiary amine), carboxamide.

7. Compound according to **Formula VI** 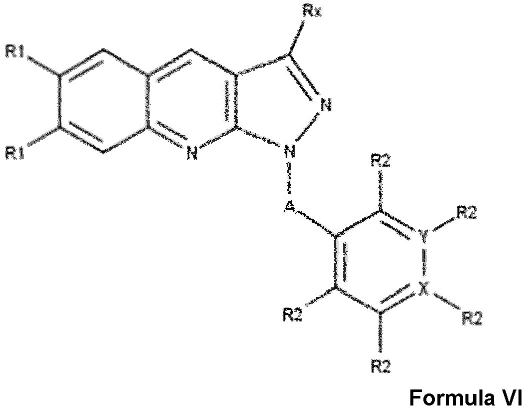 wherein
**R1** = can be the same or different, H, OH, halogen (preferably Br, Cl or F), alkyl (preferably C1 to C6 alkyl), alkoxy (preferably OCH₃, OCH₂CH₃), amine (preferably primary, secondary or tertiary amine),
**Rx** = amine (preferably primary, secondary or tertiary amine), OH, H, alkyl (preferably C1 to C3 alkyl),
**A=** alkyl (preferably branched C1 to C6 alkyl), alkenyl, alkynyl, alkoxy, alkyl carbonyl, carbonyl, carboxyl, cycloalkyl (preferably C3 to C6 cycloalkyl), aryl (preferably phenyl), sulfonyl, carboxamide,
**X** and **Y** are the same or different = C or N, and
**R2** = can be the same or different, H, alkoxy (preferably OCH₃, OCH₂CH₃), alkyl (preferably C1 to C6 alkyl), haloalkyl, OH, cycloalkyl (preferably C3 to C6 cycloalkyl), aryl (preferably phenyl), alkyl-cycloalkyl, heterocyclyl, heteroaryl, carboxamide, CO-Cx alkyl carboxamide (preferably C=ON(CH₂CH₂)₂^{o}), amine (preferably primary, secondary or tertiary amine), nitro, -CN, O,
wherein when **Rx** is a primary or secondary amine and **R1** is OCH₃
- **X** and/or **Y** are N and at least one of **R2** is alkoxy (preferably OCH₃, OCH₂CH₃), alkyl (preferably C1 to C6 alkyl), haloalkyl, OH, cycloalkyl (preferably C3 to C6 cycloalkyl), aryl (preferably phenyl), alkyl-cycloalkyl, heterocyclyl, heteroaryl, carboxamide, CO-Cx alkyl carboxamide (preferably C=ON(CH₂CH₂)₂), amine (preferably primary, secondary or tertiary amine), nitro, -CN, O, and/or
- **A** is alkyl (preferably branched C1 to C6 alkyl), alkenyl, alkynyl, alkoxy (preferably OCH₃, OCH₂CH₃), alkyl carbonyl, carboxyl, cycloalkyl (preferably C3 to C6 cycloalkyl), aryl (preferably phenyl), sulfonyl, carboxamide, and/or
- at least one of **R2** is alkyl (preferably C1 to C6 alkyl), haloalkyl, OH, cycloalkyl (preferably C3 to C6 cycloalkyl), aryl (preferably phenyl), alkyl-cycloalkyl, heteroaryl, carboxamide, CO-Cx alkyl carboxamide (preferably C=ON(CH₂CH₂)₂), nitro, -CN, O, and/or
- at least one of **R2** is a 5-membered heterocyclyl comprising one or more of N, S and/or O and at least one of **X** or **Y** is N.

8. Compound according to the preceding claim, according to **Formula VII** 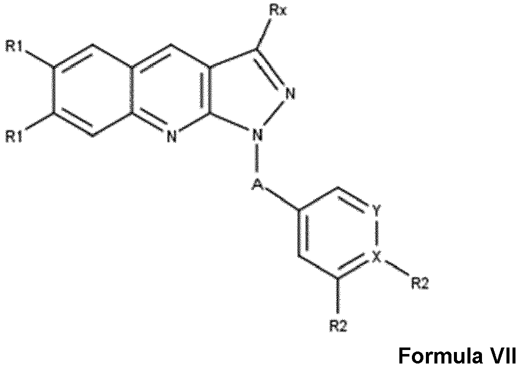 wherein
**R1** = can be the same or different, H, OH, halogen (preferably Br, Cl or F), alkoxy (preferably OCH₃, OCH₂CH₃), amine (preferably primary, secondary or tertiary amine),
**Rx** = amine (preferably primary, secondary or tertiary amine), OH, H, alkyl (preferably C1 to C3 alkyl),
**A=** alkyl (preferably branched C1 to C6 alkyl), alkenyl, alkynyl, alkoxy, alkyl carbonyl, carbonyl, carboxyl, cycloalkyl (preferably C3 to C6 cycloalkyl), aryl (preferably phenyl), sulfonyl, carboxamide, and
**R2** = can be the same or different, H, alkoxy (preferably OCH₃, OCH₂CH₃), alkyl (preferably C1 to C3 alkyl), cycloalkyl (preferably C3 to C6 cycloalkyl), aryl (preferably phenyl), alkyl-cycloalkyl, heterocyclyl (preferably C4 to C6 heterocyclyl), heteroaryl (preferably C4 to C6 heteroaryl), amine (preferably primary, secondary or tertiary amine),
wherein when **Rx** is a primary or secondary amine and **R1** is OCH₃
- **X** and/or **Y** are N and at least one of **R2** is alkoxy (preferably OCH₃, OCH₂CH₃), alkyl (preferably C1 to C3 alkyl), cycloalkyl (preferably C3 to C6 cycloalkyl), aryl (preferably phenyl), alkyl-cycloalkyl, (preferably C4 to C6 heterocyclyl), heteroaryl (preferably C4 to C6 heteroaryl), amine (preferably primary, secondary or tertiary amine), and/or
- - **A** is alkyl (preferably branched alkyl), alkenyl, alkynyl, alkoxy, alkyl carbonyl, carboxyl, cycloalkyl (preferably C3 to C6 cycloalkyl), aryl (preferably phenyl), sulfonyl, carboxamide, and/or
- at least one of **R2** is alkyl (preferably C1 to C3 alkyl), cycloalkyl (preferably C3 to C6 cycloalkyl), aryl (preferably phenyl), alkyl-cycloalkyl, heteroaryl, a 6-membered heterocyclyl, and/or
- at least one of **R2** is a 5-membered heterocyclyl comprising one or more of N, S and/or O and at least one of **X** or **Y** is N.

9. Compound according to any one of claims 7 and 8, according to **Formula VII** 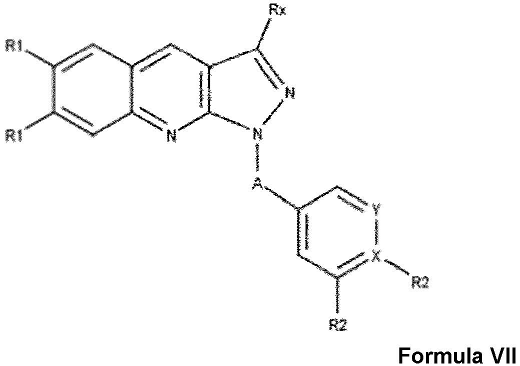 wherein
**R1** = can be the same or different, H, OH, halogen (preferably Br, Cl or F), alkoxy (preferably OCH₃, OCH₂CH₃), amine (preferably primary, secondary or tertiary amine),
**Rx** = amine (preferably primary, secondary or tertiary amine), OH, H, alkyl (preferably C1 to C3 alkyl),
**A=** alkyl (preferably branched alkyl), alkyl carbonyl, carbonyl C=OR, sulfonyl, carboxamide, and
**R2** = can be the same or different, H, alkoxy (preferably OCH₃, OCH₂CH₃), heterocyclyl (preferably C4 to C6 heterocyclyl), amine (preferably primary, secondary or tertiary amine),
wherein when **Rx** is a primary or secondary amine and **R1** is OCH₃
- **X** and/or **Y are** N and at least one of **R2** is alkoxy (preferably OCH₃, OCH₂CH₃), amine (preferably primary, secondary or tertiary amine), and/or
- - **A** is alkyl (preferably branched alkyl), alkyl carbonyl, sulfonyl, carboxamide and/or
- at least one of **R2** a 6-membered heterocyclyl comprising one or more of N, S and/or O, and/or
- at least one of **R2** is a 5-membered heterocyclyl comprising one or more of N, S and/or O and at least one of **X** or **Y** is N.

10. Compound according to any one of claims 7 to 9, according to **Formula VIII** 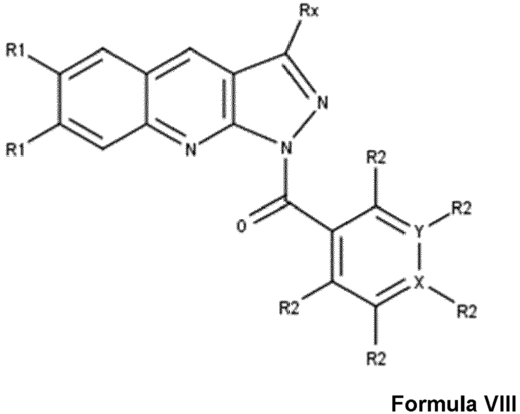 wherein
**R1** = can be the same or different, H, OH, halogen (preferably Br, Cl or F), alkyl (preferably C1 to C6 alkyl), alkoxy (preferably OCH₃, OCH₂CH₃), amine (preferably primary, secondary or tertiary amine),
**Rx** = amine (preferably primary, secondary or tertiary amine), OH, H, alkyl (preferably C1 to C3 alkyl),
**X** and **Y** are the same or different = C or N, and
**R2** = can be the same or different, H, alkoxy (preferably OCH₃, OCH₂CH₃), alkyl (preferably C1 to C6 alkyl), haloalkyl, OH, cycloalkyl (preferably C3 to C6 cycloalkyl), aryl (preferably phenyl), alkyl-cycloalkyl, heterocyclyl, heteroaryl, carboxamide, CO-Cx alkyl carboxamide (preferably C=ON(CH₂CH₂)₂^{o}), amine (preferably primary, secondary or tertiary amine), nitro, -CN, O,
wherein when **Rx** is a primary or secondary amine and **R1** is OCH₃
- **X** and/or **Y are** N and at least one of **R2** is alkoxy (preferably OCH₃, OCH₂CH₃), alkyl (preferably C1 to C6 alkyl), haloalkyl, OH, cycloalkyl (preferably C3 to C6 cycloalkyl), aryl (preferably phenyl), alkyl-cycloalkyl, heterocyclyl, heteroaryl, carboxamide, CO-Cx alkyl carboxamide (preferably C=ON(CH₂CH₂)₂^{o}), amine (preferably primary, secondary or tertiary amine), nitro, -CN, O, and/or
- at least one of **R2** is alkyl (preferably C1 to C6 alkyl), haloalkyl, OH, cycloalkyl (preferably C3 to C6 cycloalkyl), aryl (preferably phenyl), alkyl-cycloalkyl, heteroaryl, carboxamide, CO-Cx alkyl carboxamide (preferably C=ON(CH₂CH₂)₂), nitro, -CN, O, and/or
- at least one of **R2** is a 5-membered heterocyclyl comprising one or more of N, S and/or O and at least one of **X** or **Y** is N.

11. Compound according to any one of claims 7 to 10, according to Formula IX 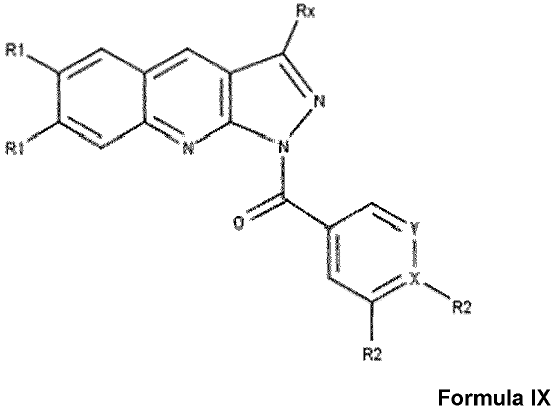 wherein
**R1** = can be the same or different, H, OH, halogen (preferably Br, Cl or F), alkoxy (preferably OCH₃, OCH₂CH₃), amine (preferably primary, secondary or tertiary amine),
**Rx** = amine (preferably primary, secondary or tertiary amine), OH, H, alkyl (preferably C1 to C3 alkyl), and
**R2** = can be the same or different, H, alkoxy (preferably OCH₃, OCH₂CH₃), alkyl (preferably C1 to C3 alkyl), cycloalkyl (preferably C3 to C6 cycloalkyl), aryl (preferably phenyl), alkyl-cycloalkyl, heterocyclyl (preferably C4 to C6 heterocyclyl), heteroaryl (preferably C4 to C6 heteroaryl), amine (preferably primary, secondary or tertiary amine),
wherein when **Rx** is a primary or secondary amine and **R1** is OCH₃
- **X** and/or **Y are** N and at least one of **R2** is alkoxy (preferably OCH₃, OCH₂CH₃), alkyl (preferably C1 to C3 alkyl), cycloalkyl (preferably C3 to C6 cycloalkyl), aryl (preferably phenyl), alkyl-cycloalkyl, (preferably C4 to C6 heterocyclyl), heteroaryl (preferably C4 to C6 heteroaryl), amine (preferably primary, secondary or tertiary amine), and/or
- at least one of **R2** is alkyl (preferably C1 to C3 alkyl), cycloalkyl (preferably C3 to C6 cycloalkyl), aryl (preferably phenyl), alkyl-cycloalkyl, heteroaryl, a 6-membered heterocyclyl, and/or
- at least one of **R2** is a 5-membered heterocyclyl comprising one or more of N, S and/or O and at least one of **X** or **Y** is N.

12. Compound according to any one of claims 7 to 11, according to **Formula IX** 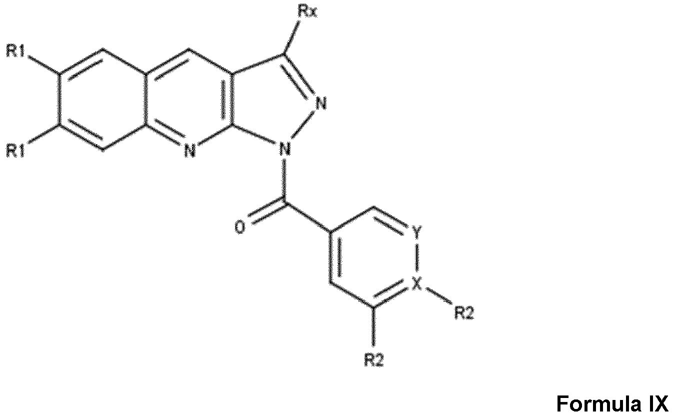 wherein
**R1** = can be the same or different, H, OH, halogen (preferably Br, Cl or F), alkoxy (preferably OCH₃, OCH₂CH₃), amine (preferably primary, secondary or tertiary amine),
**Rx** = amine (preferably primary, secondary or tertiary amine), OH, H, alkyl (preferably C1 to C3 alkyl), and
**R2** = can be the same or different, H, alkoxy (preferably OCH₃, OCH₂CH₃), heterocyclyl (preferably C4 to C6 heterocyclyl), amine (preferably primary, secondary or tertiary amine),
wherein when **Rx** is a primary or secondary amine and **R1** is OCH₃
- **X** and/or **Y are** N and at least one of **R2** is alkoxy (preferably OCH₃, OCH₂CH₃), amine (preferably primary, secondary or tertiary amine), and/or
- at least one of **R2** a 6-membered heterocyclyl comprising one or more of N, S and/or O, and/or
- at least one of **R2** is a 5-membered heterocyclyl comprising one or more of N, S and/or O and at least one of **X** or **Y** is N.

13. Compound for use in the treatment of a cancer exhibiting genotoxic stress-induced IKK/NF-κB activation according to any one of the preceding claims, wherein the compound is more effective in inhibiting NF-κB-signaling induced by genotoxic stress compared to inhibiting NF-κB-signaling induced by TNF-alpha and/or IL-1β.

14. Compound for use in the treatment of a cancer exhibiting genotoxic stress-induced IKK/NF-κB activation according to any one of the preceding claims, wherein the disease is associated with genomic instability due to defective DNA-repair mechanisms, and/or wherein said cancer is associated with NF-κB-mediated resistance to therapy-induced tumor cell apoptosis, and/or wherein the compound is administered in combination with one or more genotoxic stress-inducing (DNA damage-inducing) cancer therapies, such as genotoxic stress-inducing chemotherapy and/or irradiation therapy.

15. *In vitro* method for the inhibition of genotoxic stress-induced NF-kB signaling or inhibition of DNA repair mechanisms, preferably in a cell-based assay, comprising the use of a compound according to any one of the preceding claims.
